# EUROPEAN PATENT SPECIFICATION

(11) **EP 3 125 934 B1**
(45) Date of publication and mention of the grant of the patent: **23.10.2019**
(21) Application number: 15709319.6
(22) Date of filing: 05.03.2015
(51) Int. Cl.: G01N 33/574, A61K 47/68, C07K 16/28, C07K 14/725

(54) **CHIMERIC ANTIGEN RECEPTOR (CAR) WITH ANTIGEN BINDING DOMAINS TO THE T CELL RECEPTOR BETA CONSTANT REGION**
CHIMÄREN ANTIGEN REZEPTOR MIT ANTIGEN-BINDUNGSDOMÄNEN AN DIE T-ZELL-REZEPTOR BETA KONSTANTEN REGION
RECEPTEUR D'ANTIGENE CHIMERIQUE CONTENANT DES DOMAINES DE LIASION A LA RÉGION CONSTANTE BETA DU RÉCEPTEUR DES LYMPHOCYTES T

(30) Priority: 05.03.2014 GB 201403905; 25.09.2014 GB 201416908
(43) Date of publication of application: 08.02.2017
(60) Divisional application: 17172755.5
(73) Proprietor: UCL Business Plc., London W1T 4TP (GB)
(72) Inventor: PULÉ, Martin, London W1T 4TP (GB); MACIOCIA, Paul, London W1T 4TP (GB)
(74) Representative: D Young & Co LLP
(86) International application number: PCT/GB2015/050643
(87) International publication number: WO 2015/132598

(56) References cited:
- WO-A1-03/093318
- WO-A1-2013/074916
- S. Mathas ET AL: "Gene deregulation and spatial genome reorganization near breakpoints prior to formation of translocations in anaplastic large cell lymphoma", Proceedings of the National Academy of Sciences, vol. 106, no. 14, 7 April 2009 (2009-04-07), pages 5831-5836, XP55491246, US ISSN: 0027-8424, DOI: 10.1073/pnas.0900912106
- Cameron J Turtle ET AL: "Engineered T cells for anti-cancer therapy", CURRENT OPINION IN IMMUNOLOGY., vol. 24, no. 5, 18 July 2012 (2012-07-18), pages 633-639, XP055272888, GB ISSN: 0952-7915, DOI: 10.1016/j.coi.2012.06.004
- B. SAVOLDO ET AL: "Epstein Barr virus specific cytotoxic T lymphocytes expressing the anti-CD30 artificial chimeric T-cell receptor for immunotherapy of Hodgkin disease", BLOOD, vol. 110, no. 7, 1 October 2007 (2007-10-01), pages 2620-2630, XP55564731, ISSN: 0006-4971, DOI: 10.1182/blood-2006-11-059139

## Description

### FIELD OF THE DISCLOSURE

The present disclosure relates to cells and agents useful in the treatment of T-cell lymphoma or leukaemia.

### BACKGROUND TO THE DISCLOSURE

Lymphoid malignancies can largely be divided into those which are derived from either T-cells or B-cells. T-cell malignancies are a clinically and biologically heterogeneous group of disorders, together comprising 10-20% of non-Hodgkin's lymphomas and 20% of acute leukaemias. The most commonly identified histological subtypes are peripheral T-cell lymphoma, not otherwise specified (PTCL-NOS); angio-immunoblastic T-cell lymphoma (AITL) and anaplastic large cell lymphoma (ALCL). Of all acute Lymphoblastic Leukaemias (ALL), some 20% are of a T-cell phenotype.

These conditions typically behave aggressively, compared for instance with B-cell malignancies, with estimated 5-year survival of only 30%. In the case of T-cell lymphoma, they are associated with a high proportion of patients presenting with disseminated disease, unfavourable International Prognostic Indicator (IPI) score and prevalence of extra-nodal disease. Chemotherapy alone is not usually effective and less than 30% of patients are cured with current treatments.

Further, unlike in B-cell malignancies, where immunotherapies such as the anti-CD20 monoclonal antibody rituximab have dramatically improved outcomes, there is currently no equivalently effective, minimally toxic immunotherapeutic available for the treatment of T-cell malignancies. An important difficulty in the development of immunotherapy for T-cell disorders is the considerable overlap in marker expression of clonal and normal T-cells, with no single antigen clearly able to identify clonal (malignant) cells.

The same problem exists when targeting a pan-B-cell antigen to treat a B-cell malignancy. However, in this case, the concomitant depletion of the B-cell compartment results in relatively minor immunosuppression which is readily tolerated by most patients. Further, in therapies which result in particularly long-term depletion of the normal B-compartment, its loss can be largely abrogated by administration of pooled immunoglobulin. The situation is completely different when targeting T-cell malignancies. Here, concomitant depletion of the T-cell compartment leads to severe immunosuppression and severe toxicity. Further, there is no satisfactory way to mitigate loss of the T-cell compartment.

The toxicity is in part illustrated by the clinical effects of the therapeutic monoclonal antibody Alemtuzumab. This agent lyses cells which express CD52 and has some efficacy in T-cell malignancies. The utility of this agent is greatly limited by a profound cellular immunodeficiency, largely due to T-cell depletion, with markedly elevated risk of infection.

There is thus a need for a new method for targeted treatment of T-cell malignancies which is not associated with the above disadvantages.

Using anaplastic large cell lymphoma (ALCL), ,S. Mathas et al. in PNAS, vol. 106, no. 14, 7 April 2009, pages 5831-5836, shows a variation in the expression of several T cell associated genes, such as TRBC1. Savoldo, B. et al in Blood, 2007, pp. 2620-2630 discloses the construction of a CD30-CAR that is able to kill both autologous EBV+ cells through their native receptor and EBV-/CD30+ targets through their major MHC-unrestricted CAR..

### DESCRIPTION OF THE FIGURES

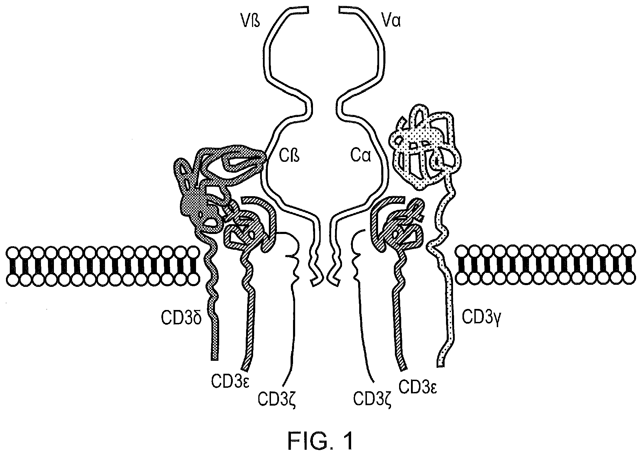

**Figure 1****: A diagram of the αβ T-cell Receptor/CD3 Complex.** The T-cell receptor is formed from 6 different protein chains which must assemble in the endoplasmic reticulum to be expressed on the cell surface. The four proteins of the CD3 complex (CD3ζ, CD3γ, CD3ε and CD3δ) sheath the T-cell Receptor (TCR). This TCR imbues the complex with specificity of a particular antigen and is composed of two chains: TCRα and TCRβ. Each TCR chain has a variable component distal to the membrane and a constant component proximal to the membrane. Nearly all T-cell lymphomas and many T-cell leukaemias express the TCR/CD3 complex.

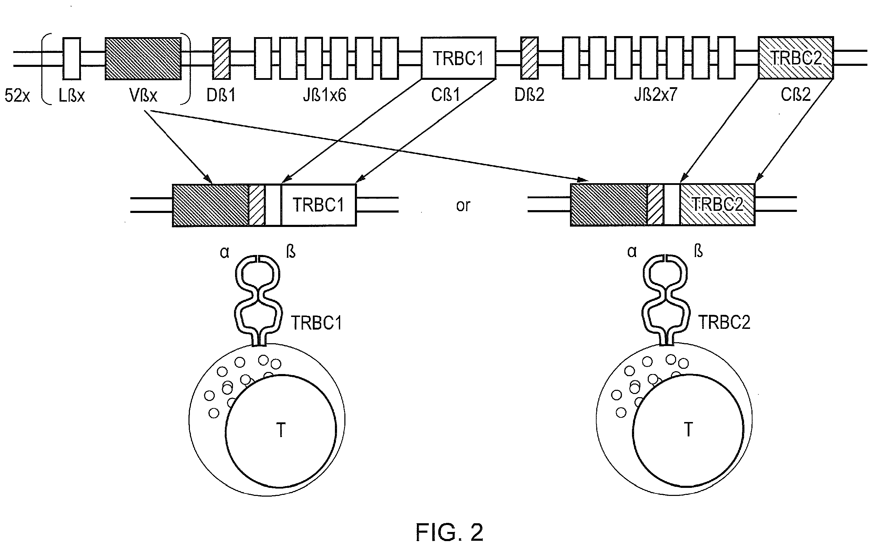

**Figure 2****: The segregation of T-cell Receptor β-constant region (TRBC)-1 and TRBC2 during T-cell receptor rearrangement.** Each TCR beta chain is formed from genomic recombination of a particular beta variable (V), diversity (D), joining (J) and constant (TRBC) regions. The human genome contains two very similar and functionally equivalent TRBC loci known as TRBC1 and TRBC2. During TCR gene re-arrangement, a J-region recombines with either TRBC1 or TRBC2. This rearrangement is permanent. T-cells express many copies of a single TCR on their surface, hence each T-cell will express a TCR whose β-chain constant region is coded for by either TRBC1 or TRBC2.

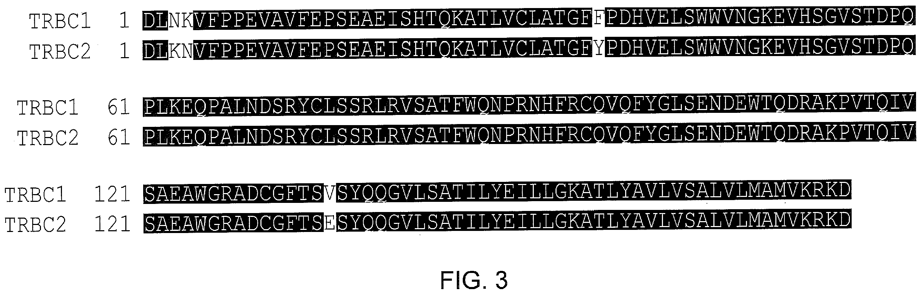

**Figure 3****: Alignment of human TRBC1 and TRBC2 at the amino acid level.** The TCRβ constant chain coded for by TRBC1 and TRBC2 differ by only 4 amino acid differences: K / N at position 3 of the TRBC; N / K at position 4 of the TRBC; F / Y at position 36 of the TRBC; V / E at position 135 of the TRBC;

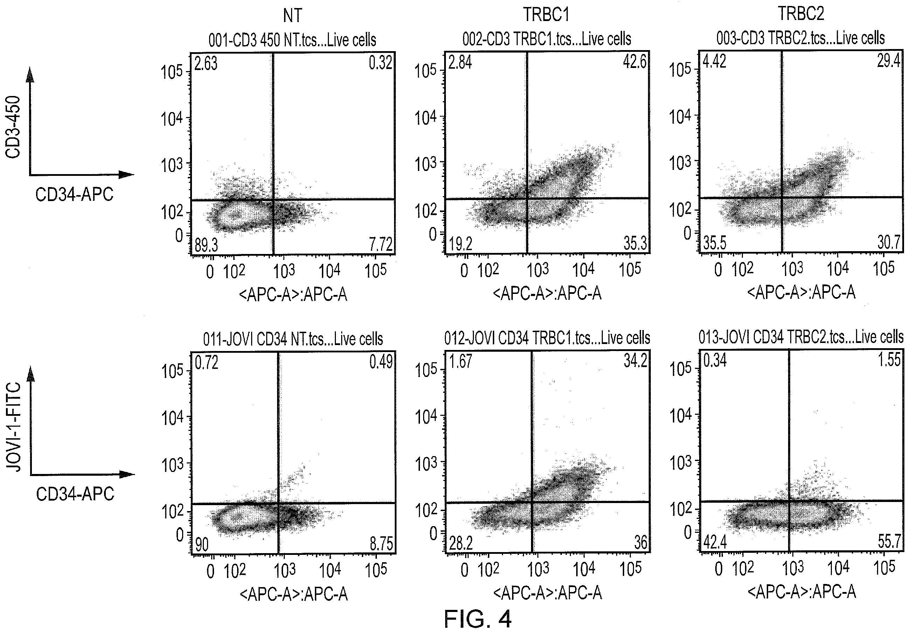

**Figure 4****: Definitive demonstration that the JOVI-1 antibody binds to TRBC1 but not TRBC2.** Genetic engineering of cells was used to definitively demonstrate that the JOVI-1 monoclonal antibody recognizes TRBC1 variant of the TCRβ constant chain. A tri-cistronic retroviral cassette was generated. This coded for both the TCRα and TCRβ chains of a human TCR which recognizes the minor histocompatibility antigen HA1, along with truncated human CD34 as a convenient marker gene. The HA1 TCR is natively TRBC2. A second retroviral cassette was generated which was identical to the first except the 4 residues in the TCR β constant region which differentiate TRBC1 from TRBC2 were changed to those coded by TRBC1. Jurkat T-cells which have both their TCRα and TCRβ chains knocked-out were transduced with either vector. These cells were stained with either a pan-TCR/CD3 antibody or the monoclonal JOVI-1 conjugated along with antibodies against CD34 and analysed in a flow cytometer. The upper row demonstrates staining with a pan-TCR/CD3 antibody v CD34 (marker of transduction), the lower row demonstrates staining with JOVI-1 vs CD34. Transduced cells demonstrate similar TCR/CD3 staining but only TRBC1 +ve cells stain with JOVI-1. Hence, JOVI-1 is specific to TRBC1 and further it is possible to use an antibody to distinguish TRBC1 and 2 TCRs.

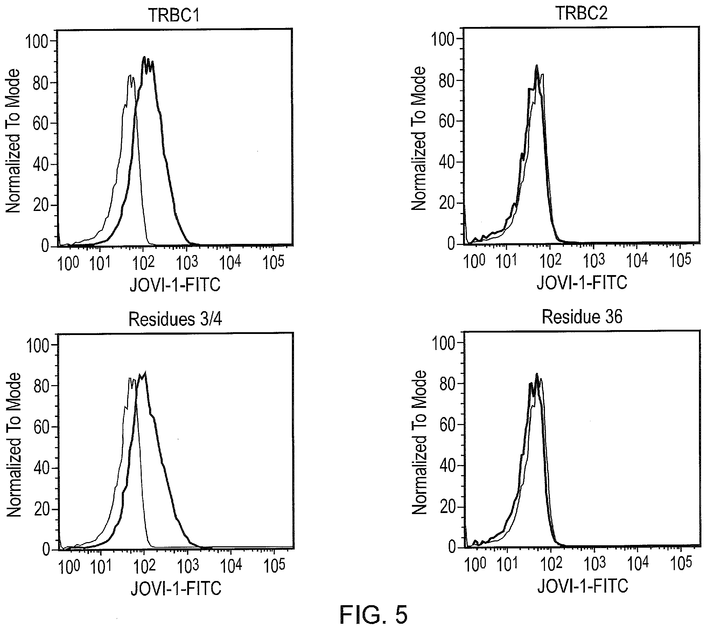

**FIGURE 5****: The JOVI-1 mAb differentiates TRBC1 from TRBC2 by recognizing residues 3 and 4 of the TRBC.** To precisely determine how JOVI-1 discriminates TRBC1 from TRBC2, the HA1 TCR TRBC2 construct detailed above was mutated to make two TRBC1/2 hybrids. An additional variant was generated so that only residues 3 and 4 of the TCRβ constant chain were changed from those of TRBC2 to those found in TRBC1. A further variant was made where only residue 36 was changed from that found in TRBC2 to TRBC1. TCR knock-out Jurkat T-cells were transduced with these new constructs. The original TRBC2 and TRBC1 transduced Jurkats described in Figure 4 were used as controls. The Jurkat T-cells were stained with JOVI-1 and analysed with a flow cytometer. Staining of JOVI-1 is overlaid over that of non-transduced TCR knock-out Jurkat T-cells. JOVI-1 stained Jurkats expressing the TRBC1 TCR but not the TRBC2 TCR. JOVI-1 stained TRBC1/2 hybrid where only TRBC residues 3 and 4 were those of TRBC1. JOVI-1 did not stain Jurkat T-cells where only TRBC residue 36 was that of TRBC1.

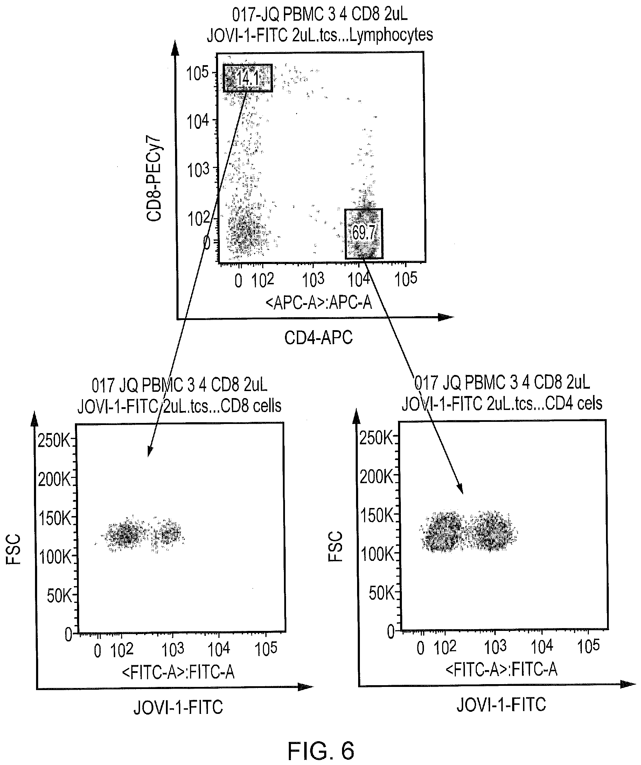

**FIGURE 6****: Example of normal donor T-cell expression of TRBC1.** Normal donor peripheral blood mononuclear cells were stained with antibodies against CD3, CD4, CD8 and JOVI-1 and analysed by flow cytometry. CD4+ and CD8+ T-cell populations are shown on the upper panel. Each of this population is gated and forward scatter vs JOVI-1 staining are shown on the Y and X-axes respectively. These data show that both CD4+ and CD8+ compartments contain cells which are TRBC1 +ve and -ve. This is representative data from one donor.

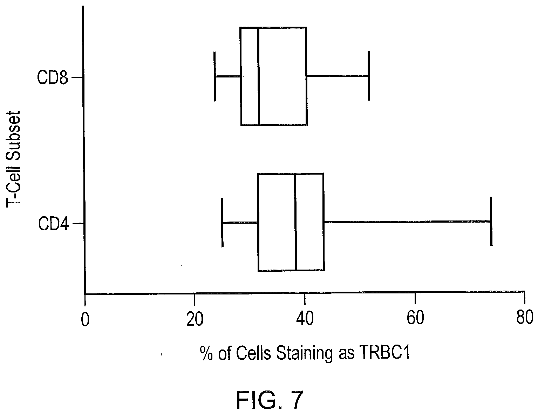

**FIGURE 7****: TRBC1+ T-cells in several normal donors.** Ten normal donors were bled and peripheral blood mononuclear cells were stained as described in figure 4 above. The aggregate data of the proportion of TRBC1 T-cells in both CD4 and CD8 compartments is shown in a bar graph along with median and range. All donors had TRBC1+ and TRBC1- compartments. Median % of TRBC1+ cells = 36%.

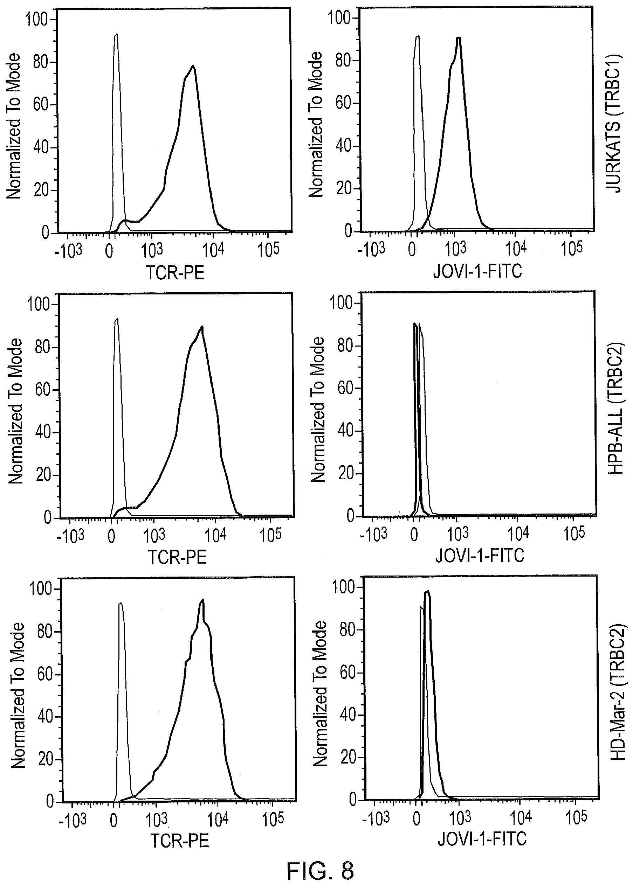

**FIGURE 8****: T-cell malignancy derived cell lines stained with JOVI-1.** Several cell lines have been derived from T-cell malignancies. Many of these cell lines still express the TCR. We selected Jurkats (A T-cell leukaemia cell line), HPB-ALL (another T-cell leukaemia cell line) and HD-Mar-2 (a T-cell lymphoma cell line) for study. By staining these cell lines with a pan-TCR/CD3 antibody, we were able to demonstrate that all three express TCR (left panels, staining overlaid over isotype control staining). Next, by staining with JOVI-1 we were able to determine that these T-cell lines are either TRBC1 negative or positive. Only Jurkats cells (TRBC1+) and not HPB-ALL or HD-Mar-2 (TRBC2+) cells stain with JOVI-1, supporting exclusive expression of either TRBC1 or 2.

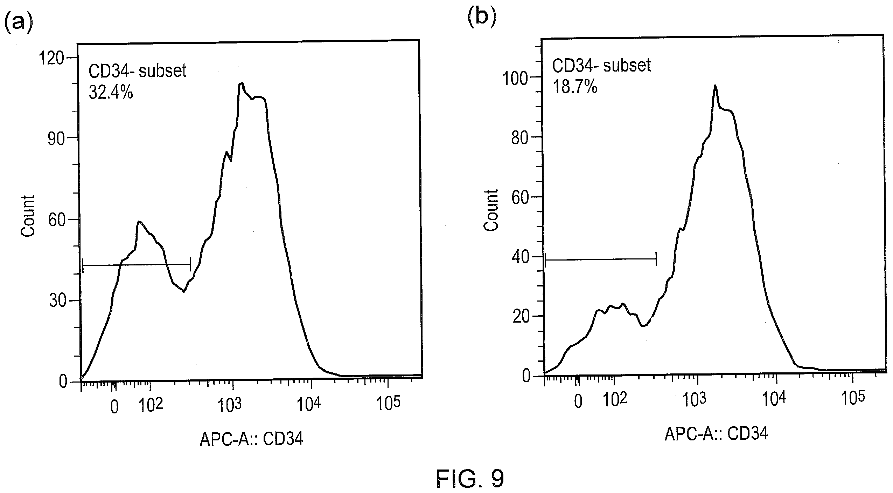

**FIGURE 9****: Selective Killing of TRBC1 T-cells with JOVI-1 mAb.** Wild-type Jurkat T-cells (CD34-, TRBC1+) were mixed with TCRαβ knock-out Jurkat T-cells transduced with TRBC2 co-expressed with the CD34 marker gene (CD34+TRBC2+). These cells were incubated with JOVI-1 alone or incubated with JOVI-1 and complement for 1 hour. Cells were washed and stained for CD34, Annexin V and 7-AAD. Cells were analysed by flow-cytometry. Shown below is CD34 expression in the live population as defined by Annexin-V negative and 71AAD dim population. (a) JOVI-1 alone; (b) JOVI-1 with complement. Selective killing of TRBC1 T-cells (CD34-) is observed.

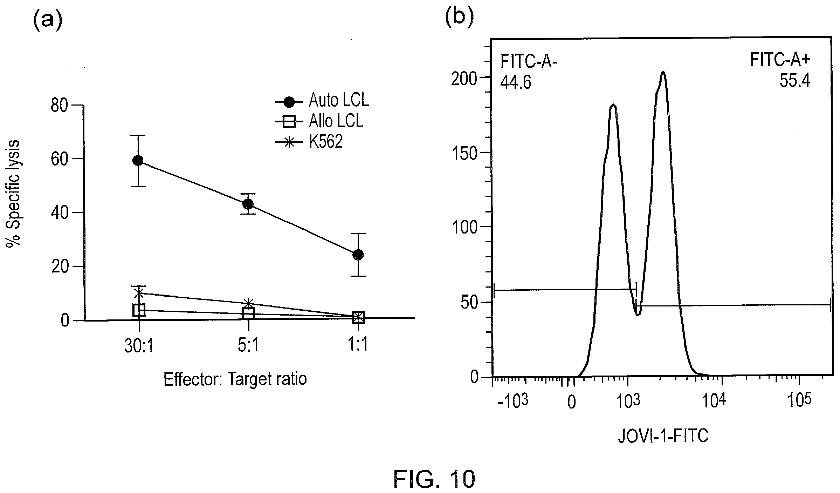

**FIGURE 10****: Polyclonal Epstein Barr Virus (EBV) specific T-cells can be split into two approximately equal TRBC1/2 populations.** Using well established methods, the inventors selectively expanded EBV specific T-cells from the peripheral blood of a normal donor. The subsequent line had a high degree of selectively against autologous EBV infected B-cells (auto LCLs), and no activity against allogeneic EBV infected T-cells (allo LCLs), and no non-specific NK activity (as measured by testing against K562 cells). Such a line is representative of the donor's EBV immunity. (b) When stained with JOVI-1, these T-cells typed approximately equally for TRBC1 and TRBC2.

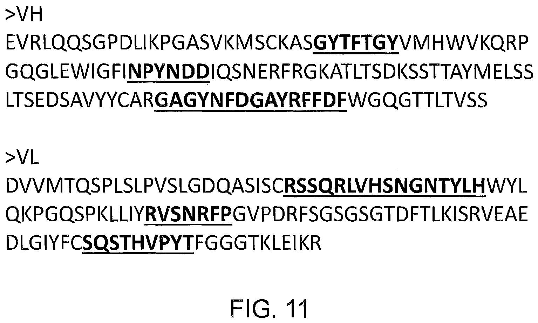

**FIGURE 11****: Annotated sequence of JOVI-1 VH and VL.** The hypervariable regions are underlined and in bold.

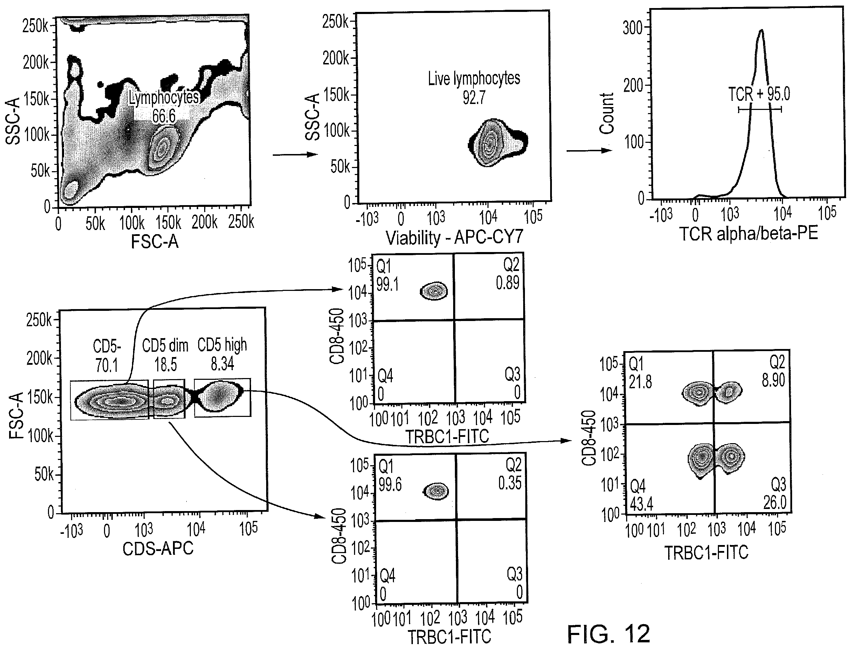

**FIGURE 12****: Demonstration that a peripheral T-cell lymphoma is TRBC restricted, but normal circulating T-cells are not.** Peripheral blood T-cells from a patient with circulating T-cell lymphoma cells were drawn. Peripheral mononuclear cells were isolated and stained with a panel of antibodies including CD5, TCR and JOVI-1. Normal and malignant T-cells could be differentiated on flow by CD5 expression intensity. CD5 bright (normal T-cells) had approximately equal TRBC1 and 2 populations. The CD5 intermediate and dim populations (the tumour) were all TRBC2 positive. If this patient had a TRBC2 directed therapy, the lymphoma would be eradicated and approximately half of their T-cells would be spared.

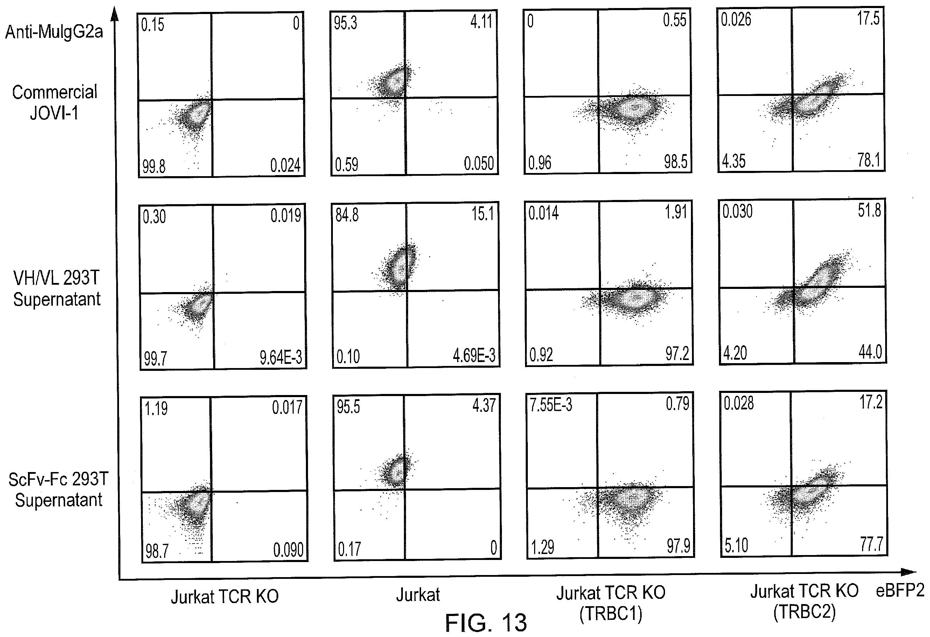

**FIGURE 13****: Demonstration that the VH and VL derived from JOVI-1 were correct and that they can fold as a single-chain variable fragment.** Original hybridoma supernatant, recombinant JOVI-1 antibody and scFv-Fc generated from transfected 293T cells were used to stain a number of cell lines: Jurkat TCR knockouts, wild-type Jurkats, Jurkat TCR knock-out transduced with a TRBC1 TCR in a vector co-expressing eBFP2; Jurkat TCR knock-out transduced with a TRBD2 TCR in a vector co-expressing eBFP2. Staining was analysed by flow cytometry. Both the recombinant antibody and the scFv bound cells expressing TRBC2.

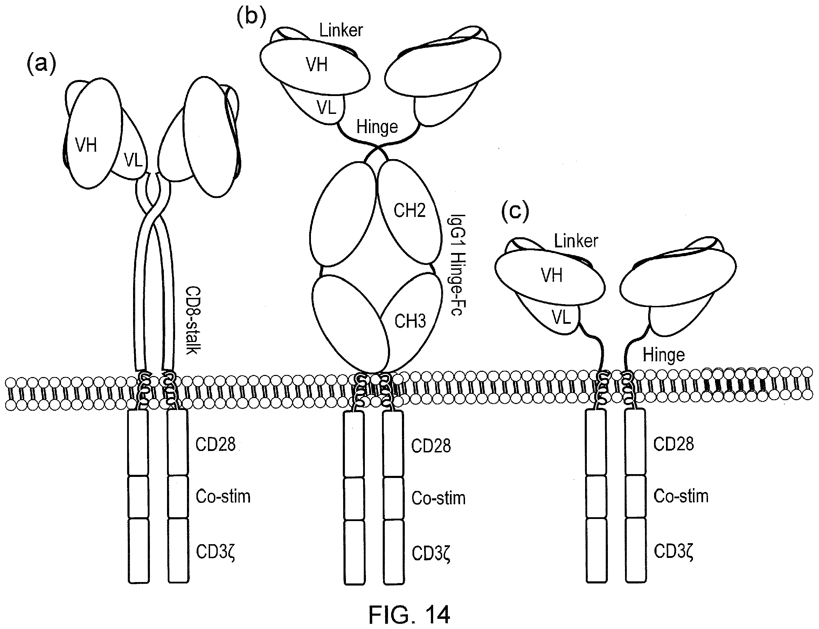

**FIGURE 14****: JOVI-1 based CARs in different formats.** CARs typically comprise of a binding domain, a spacer, a transmembrane domain and an intracellular signalling domain. In this study CARs were generated which comprise of the JOVI-1 scFv; a spacer derived from either the CD8 stalk, the hinge-CH2-CH3 domain of human IgG1 with mutations which remove FcR binding; or a spacer derived from human IgG1.

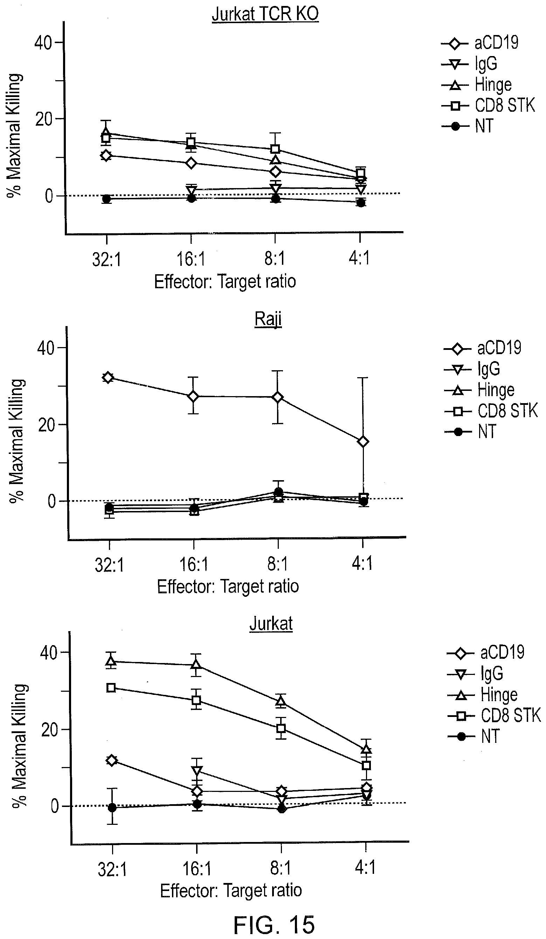

**FIGURE 15****: Function of JOVI-1 based CAR.** Normal donor peripheral blood T-cells were transduced with the different CARs described above. T-cells were also transduced with a CD19-specific CAR as a control. These T-cells were then challenged with target cells: Jurkats - TCR knock-out and Jurkats wild-type and Raji cells (a CD19+ B-cell lymphoma line). Chromium release data is shown of the effectors against different targets. JOVI-1 CAR T-cells kill Jurkats but not Raji cells or Jurkats with TCR knocked out.

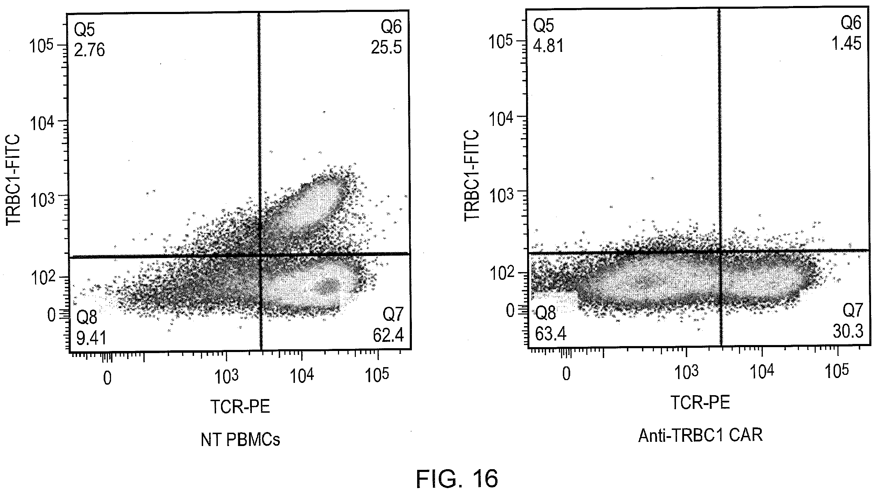

**FIGURE 16****: Self-Purging of JOVI-1 CAR T-cell cultures.** Since T-cells comprise of approximately equal numbers of T-cells which are either TRBC1 or TRBC2 positive, it is possible that after introduction of CARs a certain amount of "fratricide" or self-purging of the culture may occur. It was demonstrated that this was the case. In this example, CAR T-cells were stained after transduction and analysed by flow-cytometry. Comparing mock-transduced versus transduced, one can observe that the T-cell population loses TRBC1 positive T-cells.

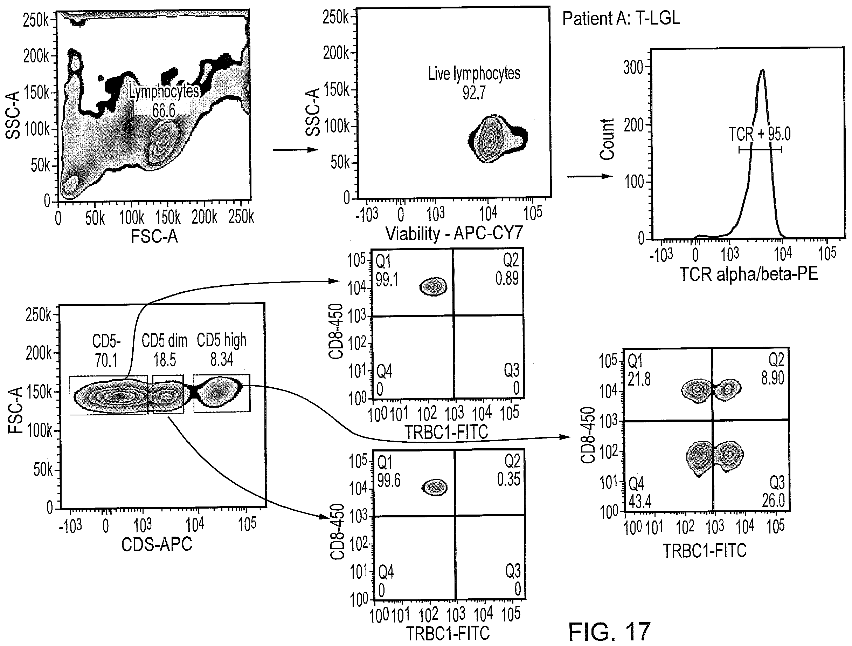

**Figure 17****: Investigating the clonality of T-cell large granular Leukaemia (T-LGL)** - Patient A

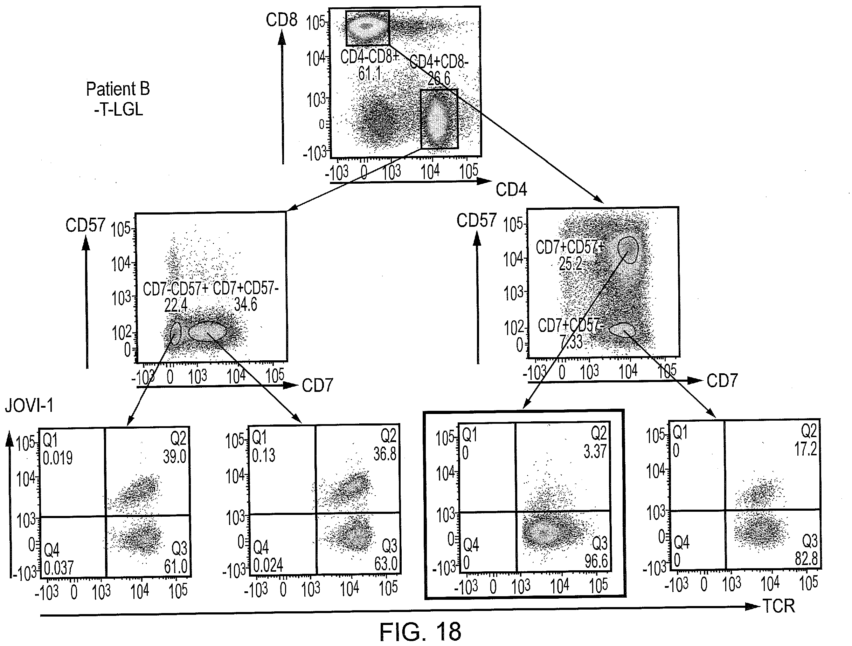

**Figure 18****: Investigating the clonality of T-cell large granular Leukaemia (T-LGL)** - Patient B

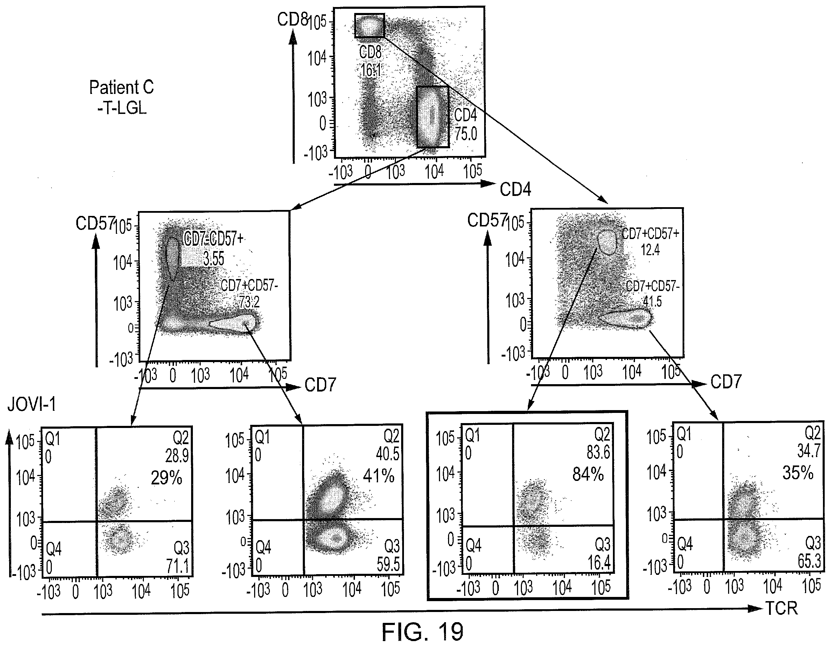

**Figure 19****: Investigating the clonality of T-cell large granular Leukaemia (T-LGL)** - Patient C

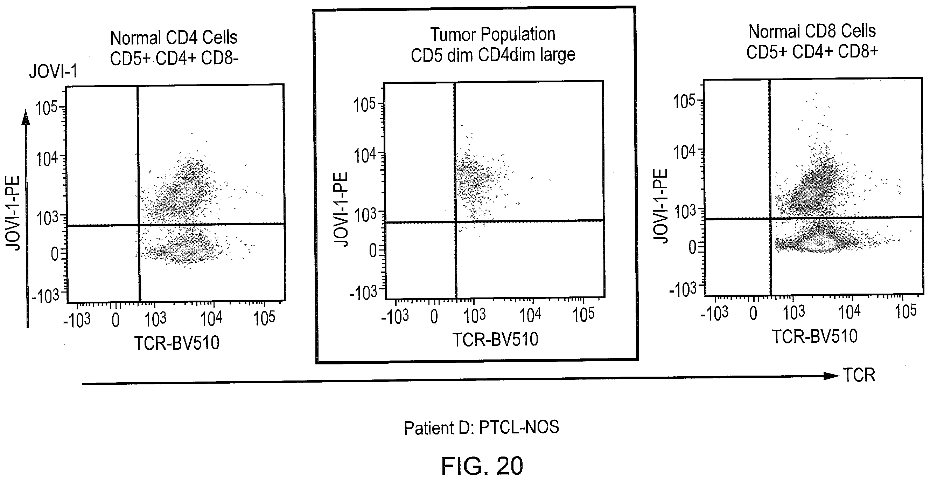

**Figure 20****: Investigating the clonality of polyclonal T-cell lymphoma (PCTL)** - Patient D

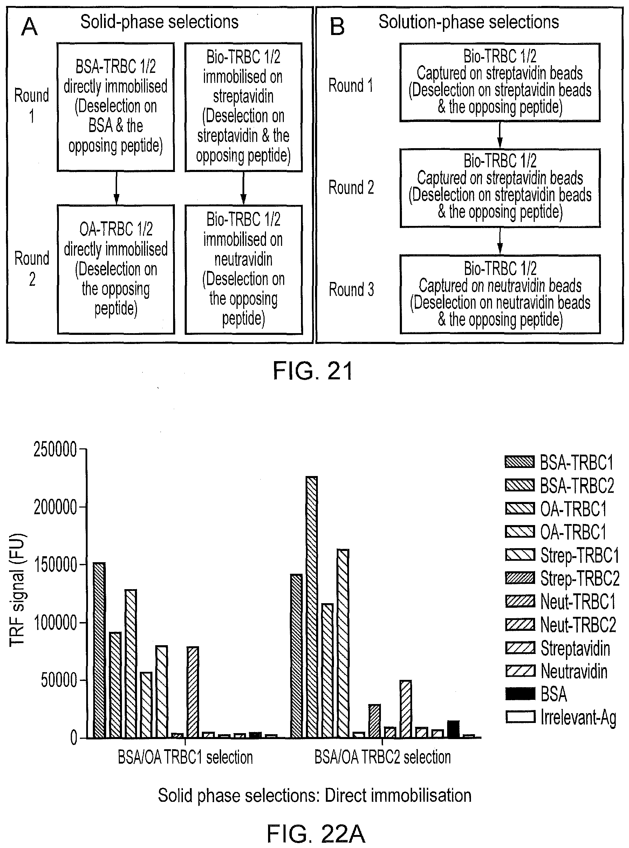

**Figure 21** **:TRBC peptide phage selection strategies.** A) Two rounds of solid-phase phage display selections on TRBC peptides directly or indirectly immobilised on a surface. B) Three rounds of solution-phase phage display selections on biotinylated TRBC peptides.

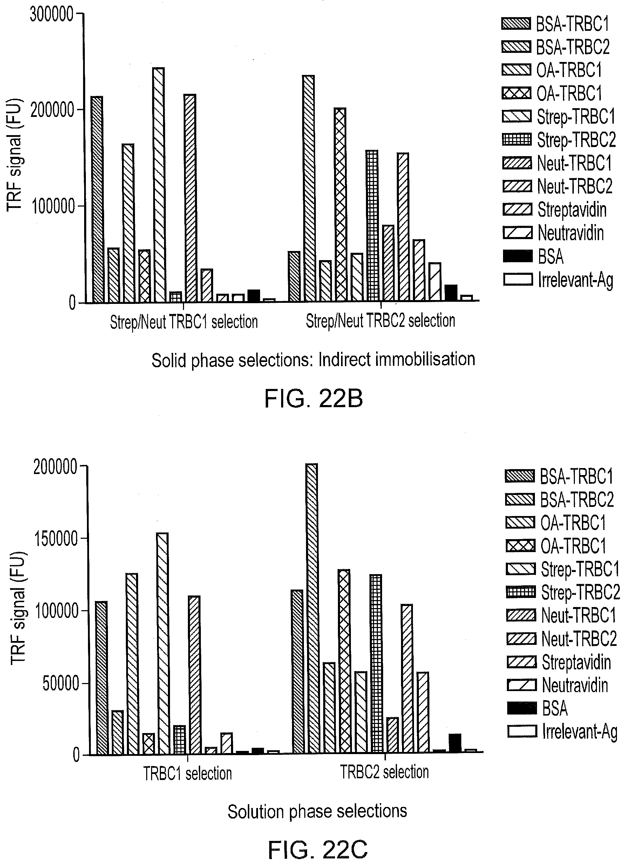

**Figure 22****: Analysis of polyclonal phage outputs from TRBC peptide phage display selections.** TRF binding assay using polyclonal phage from solid phase selections carried out on TRBC peptides directly immobilised as BSA/OA conjugates (A), solid phase selections on TRBC peptides immobilised on streptavidin/neutravidin (B) and from solution phase selections (C).

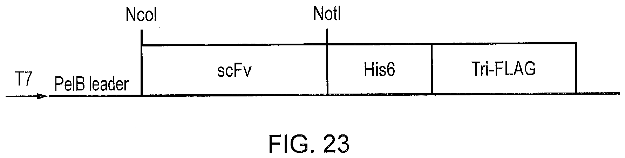

**Figure 23****: Schematic representation of pSANG10-3F vector.** Gene encoding single chain antibody (scFv) is cloned at NcoI/NotI site downstream of T7 promoter and pelB leader (for periplasmic translocation). The vector also contains a C-terminal hexa-histidine tag (His6) for purification and tri-FLAG tag detection.

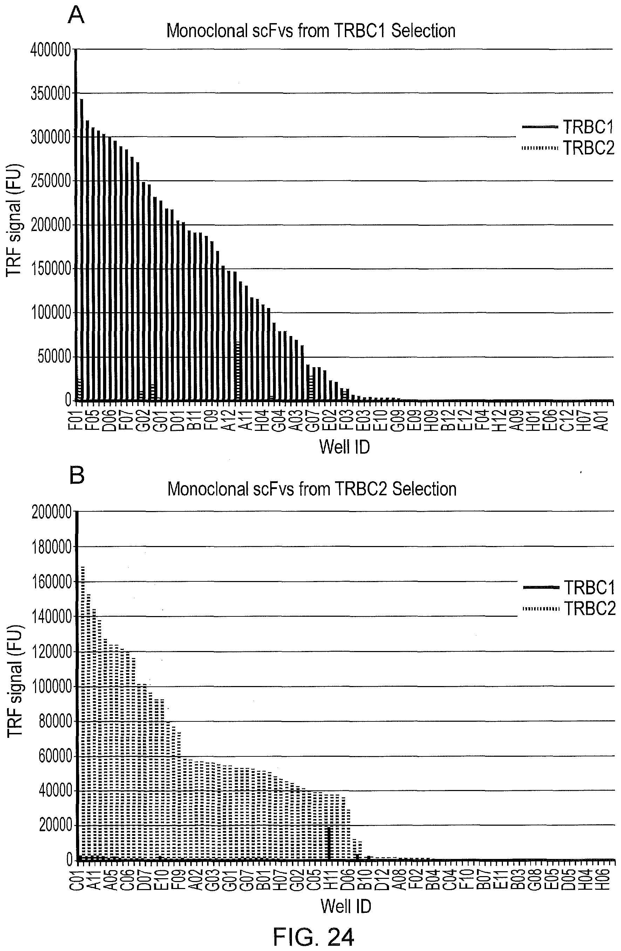

**Figure 24****: Primary screening for TRBC1 and TRBC2 specific binders.** Binding of 94 scFvs from TRBC1 (A) and TRBC2 (B) selections to biotinylated TRBC1 and TRBC2 (0.5 µg/ml) immobilised on neutravidin (10 µg/ml) coated Nunc Maxisorp™ 96 well plates. The scFv binding to the immobilised peptides was detected using an anti-FLAG antibody conjugated to europium.

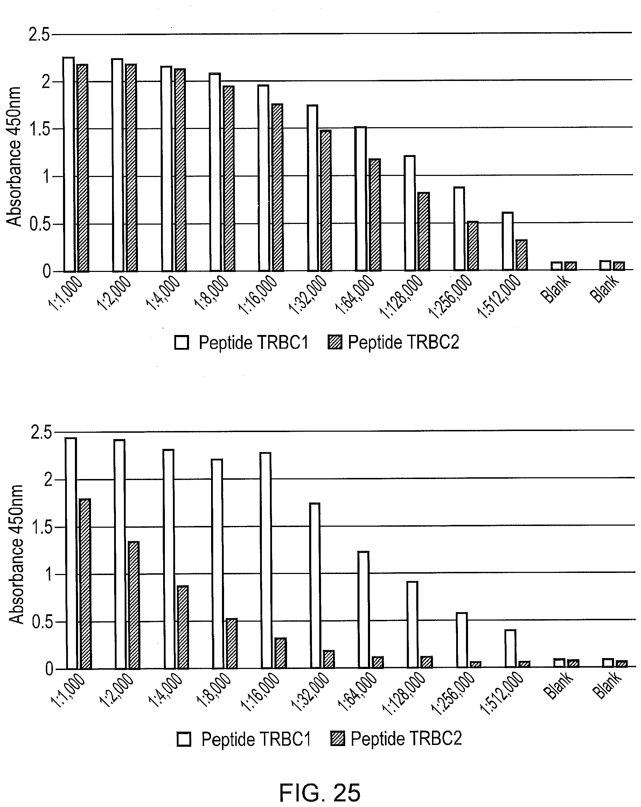

**Figure 25****: Binding of polyclonal antibody sera from rabbit #13174 immunized with TRBC1 against TRBC1 and TRBC2 peptides.** (A) After 3rd immunization (B) After 3rd immunization and purification of TRBC1 specific antibodies.

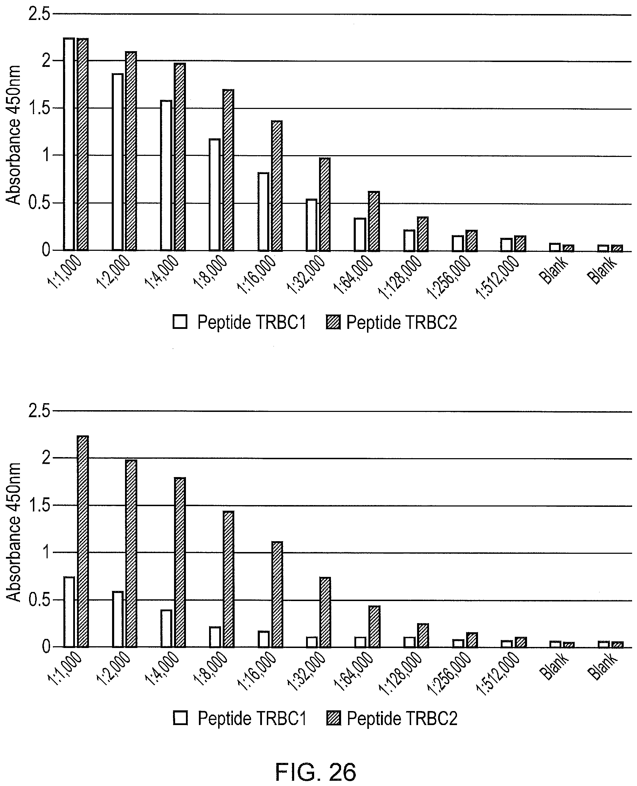

**Figure 26****: Binding of polyclonal antibody sera from rabbit #17363 immunized with TRBC2 peptide against TRBC1 and TRBC2 peptides.** (A) After 3rd immunization (B) After 3rd immunization and purification of TRBC2 specific antibodies.

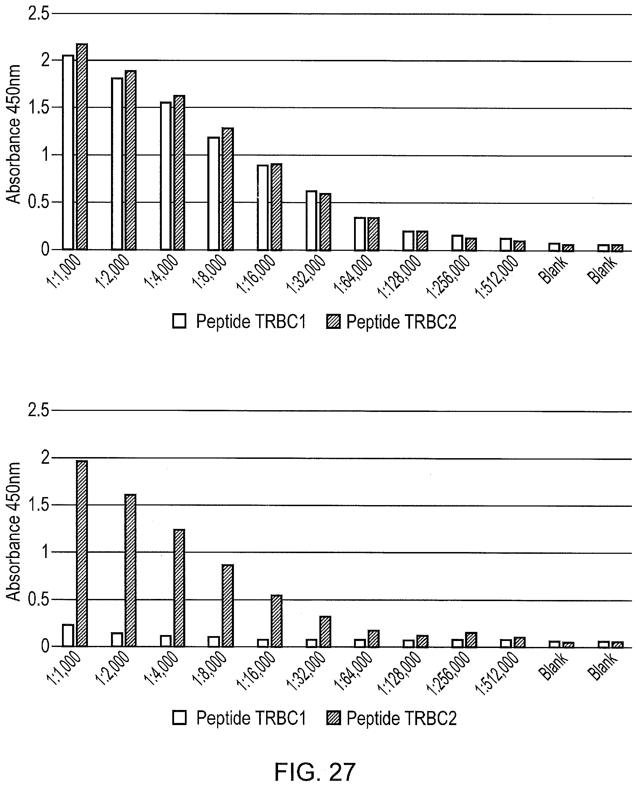

**Figure 27****: Binding of polyclonal antibody sera from rabbit #17364 immunized with TRBC2 peptide against TRBC1 and TRBC2 peptides.** (A) After 3rd immunization (B) After 3rd immunization and purification of TRBC2 specific antibodies.

### SUMMARY OF ASPECTS OF THE DISCLOSURE

The present inventors have devised a method whereby it is possible to deplete malignant T-cells in a subject, without affecting a significant proportion of healthy T cells. In particular, they have developed TRBC1 and TRBC2-specific chimeric antigen receptors (CARs) for use in the treatment of T-cell malignancies.

Thus in a first aspect the present disclosure provides a chimeric antigen receptor (CAR) which comprises an antigen-binding domain which selectively binds TCR beta constant region 1 (TRBC1) or TRBC2.

In a first aspect of the first aspect of the disclosure there is provided a CAR which selectively binds TRBC1. In this aspect the CAR may comprise an antigen-binding domain which has a variable heavy chain (VH) and a variable light chain (VL) which comprise the following complementarity determining regions (CDRs):
VH CDR1: SEQ ID No. 7;
VH CDR2: SEQ ID No. 8;
VH CDR3: SEQ ID No. 9;
VL CDR1: SEQ ID No. 10;
VL CDR2: SEQ ID No. 11; and
VL CDR3: SEQ ID No. 12.

The CAR may comprise an antigen-binding domain which comprises a variable heavy chain (VH) having the sequence shown as SEQ ID No. 1 and a variable light chain (VL) having the sequence shown as SEQ ID No. 2.

The CAR may comprise an antigen-binding domain which comprises an scFv having the amino acid sequence shown as SEQ ID No. 3.

The CAR may comprise an amino acid sequence selected from SEQ ID No. 33, 34 and 35.

The CAR may comprise a VH CDR3 and/or a VL CDR3 from those listed in Table 1. The CAR may comprise an antibody or functional fragment thereof which comprises:
(i) the heavy chain CDR3 and/or the light chain CDR3;
(ii) heavy chain CDR1, CDR2 and CDR3 and/or light chain CDR1, CDR2 and CDR3; or
(iii) the variable heavy chain (VH) and/or the variable light chain (VL);
from one of the scFvs shown as SEQ ID No. 13 to 22.

In a second aspect of the first aspect of the invention there is provided a CAR which selectively binds TRBC2.

In connection with this aspect the CAR may comprise a VH CDR3 and/or a VL CDR3 from those listed in Table 2.

The CAR may comprise an antibody or functional fragment thereof which comprises:
(i) the heavy chain CDR3 and/or the light chain CDR3;
(ii) heavy chain CDR1, CDR2 and CDR3 and/or light chain CDR1, CDR2 and CDR3; or
(iii) the variable heavy chain (VH) and/or the variable light chain (VL);
from one of the scFvs shown as SEQ ID No. 23 to 32.

In a second aspect, the present invention provides a nucleic acid sequence encoding a CAR according to the first aspect of the invention.

In a third aspect, there is provided a vector which comprises a nucleic acid sequence according to the second aspect of the invention.

In a fourth aspect, there is provided a cell which comprises a CAR according to the first aspect of the invention. The cell may be a cytolytic immune cell, such as a T-cell or natural killer (NK) cell.

In a fifth aspect there is provided a method for making a cell according to the fourth aspect of the invention which comprises the step of transducing or transfecting a cell with a nucleic acid sequence according to the second aspect of the invention or a vector according to the third aspect of the invention.

In a sixth aspect there is provided a cell according to the fourth aspect of the invention for use in a method for treating a T-cell lymphoma or leukaemia in a subject which comprises the step of
administrating the cell comprising the TCRB1 or TCRB2 selective CAR to the subject, to cause selective depletion of the malignant T-cells, together with normal T-cells expressing the same TRBC as the malignant T-cells, but not to cause depletion of normal T-cells expressing the TRBC not expressed by the malignant T-cells.

The method may also comprise the step of investigating the TCR beta constant region (TCRB) of a malignant T cell from the subject to determine whether it expresses TRBC1 or TRBC2.

There is also disclosed a method for treating a T-cell lymphoma or leukaemia in a subject which comprises the step of administering a TCRB1 or TCRB2 selective agent to the subject, wherein the agent causes selective depletion of the malignant T-cells, together with normal T-cells expressing the same TRBC as the malignant T-cells, but does not cause depletion of normal T-cells expressing the TRBC not expressed by the malignant T-cells.

In a first aspect of this aspect of the disclosure the agent is a TCRB1 selective agent. In a second aspect of this aspect of the disclosure the agent is a TRBC2 selective agent.

The method may also comprise the step of investigating the TCR beta constant region (TRBC) of a malignant T-cell to determine whether it expresses TRBC1 or TRBC2, prior to the administration step.

The agent may be a depleting monoclonal antibody or a fragment thereof. The agent may be a conjugated antibody, which may comprise a chemotherapeutic entity.

The agent may be a bispecific T-cell engager. The agent may be a chimeric antigen receptor (CAR) expressing T-cell. The CAR may comprise an amino acid sequence selected from the group consisting of SEQ ID No. 33, 34 and 35.

The agent may comprise the JOVI-1 antibody or a functional fragment thereof.

The agent may comprise an antibody or a functional fragment thereof having a variable heavy chain (VH) and a variable light chain (VL) which comprise the following complementarity determining regions (CDRs):
VH CDR1: SEQ ID No. 7;
VH CDR2: SEQ ID No. 8;
VH CDR3: SEQ ID No. 9;
VL CDR1: SEQ ID No. 10;
VL CDR2: SEQ ID No. 11; and
VL CDR3: SEQ ID No. 12.

The agent may comprise an antibody of functional fragment thereof which comprises a variable heavy chain (VH) having the amino acid sequence shown as SEQ ID No. 1 and a variable light chain (VL) having the amino acid sequence shown as SEQ ID No. 2.

The agent may comprise an ScFv having the amino acid sequence shown as SEQ ID No. 3.The agent may be provided as a pharmaceutical composition.

The T-cell lymphoma or leukaemia may be selected from peripheral T-cell lymphoma, not otherwise specified (PTCL-NOS); angio-immunoblastic T-cell lymphoma (AITL), anaplastic large cell lymphoma (ALCL), enteropathy-associated T-cell lymphoma (EATL), hepatosplenic T-cell lymphoma (HSTL), extranodal NK/T-cell lymphoma nasal type, cutaneous T-cell lymphoma,primary cutaneous ALCL, T cell prolymphocytic leukaemia and T-cell acute lymphoblastic leukaemia.

The present disclosure also provides an agent for use in treating a T-cell lymphoma or leukaemia according to such a method.

The present disclosure also provides a kit comprising an agent for use as defined above.

The present disclosure also provides the use of an agent in the manufacture of a medicament for treatment of a T-cell lymphoma or leukaemia according to the above method.

The present disclosure also provides a method for diagnosing a T-cell lymphoma or leukaemia in a subject which comprises the step of determining the percentage of total T-cells in a sample which are TRBC1 or TRBC2 positive.

A percentage of TRBC1 or TRBC2 positive T-cells which is greater than about 80% may indicate the presence of a T-cell lymphoma or leukaemia.

The sample may be a peripheral blood sample or a biopsy.

The agent which binds total T-cells may bind CD3.

### DETAILED DESCRIPTION

The present disclosure provides agents, such as chimeric antigen receptors (CARs) which selectively bind TRBC1 or TRBC2. Such agents are useful in methods for treating a T-cell lymphoma or leukaemia in a subject. T cell malignancies are clonal, so they either express TRBC1 or TRBC2. By administering a TCRB1 or TCRB2 selective agent to the subject, the agent causes selective depletion of the malignant T-cells, together with normal T-cells expressing the same TRBC as the malignant T-cells, but does not cause depletion of normal T-cells expressing the TRBC not expressed by the malignant T-cells.

### TCR β CONSTANT REGION (TRBC)

The T-cell receptor (TCR) is expressed on the surface of T lymphocytes and is responsible for recognizing antigens bound to major histocompatibility complex (MHC) molecules. When the TCR engages with antigenic peptide and MHC (peptide/MHC), the T lymphocyte is activated through a series of biochemical events mediated by associated enzymes, co-receptors, specialized adaptor molecules, and activated or released transcription factors.

The TCR is a disulfide-linked membrane-anchored heterodimer normally consisting of the highly variable alpha (α) and beta (β) chains expressed as part of a complex with the invariant CD3 chain molecules. T-cells expressing this receptor are referred to as α:β (or αβ) T-cells (-95% total T-cells). A minority of T-cells express an alternate receptor, formed by variable gamma (γ) and delta (δ) chains, and are referred to as γδ T-cells (-5% total T cells).

Each α and β chain is composed of two extracellular domains: Variable (V) region and a Constant (C) region, both of Immunoglobulin superfamily (IgSF) domain forming antiparallel β-sheets. The constant region is proximal to the cell membrane, followed by a transmembrane region and a short cytoplasmic tail, while the variable region binds to the peptide/MHC complex (see Figure 1). The constant region of the TCR consists of short connecting sequences in which a cysteine residue forms disulfide bonds, which forms a link between the two chains.

The variable domains of both the TCR α-chain and β-chain have three hypervariable or complementarity determining regions (CDRs). The variable region of the β-chain also has an additional area of hypervariability (HV4), however, this does not normally contact antigen and is therefore not considered a CDR.

The TCR also comprises up to five invariant chains γ,δ,ε (collectively termed CD3) and ζ. The CD3 and ζ subunits mediate TCR signalling through specific cytoplasmic domains which interact with second-messenger and adapter molecules following the recognition of the antigen by αβ or γδ. Cell-surface expression of the TCR complex is preceded by the pair-wise assembly of subunits in which both the transmembrane and extracellular domains of TCR α and β and CD3 γ and δ play a role

TCRs are therefore commonly composed of the CD3 complex and the TCR α and β chains, which are in turn composed of variable and constant regions (Figure 1).

The locus (Chr7:q34) which supplies the TCR β-constant region (TRBC) has duplicated in evolutionary history to produce two almost identical and functionally equivalent genes: *TRBC1* and *TRBC2* (Figure 2), which differ by only 4 amino acid in the mature protein produced by each (Figure 3). Each TCR will comprise, in a mutually exclusive fashion, either TRBC1 or TRBC2 and as such, each αβ T-cell will express either TRBC1 or TRBC2, in a mutually exclusive manner.

The present inventors have determined that, despite the similarity between the sequence of the TRBC1 and TRBC2, it is possible to discriminate between them. The inventors have also determined that amino acid sequences of TRBC1 and TRBC2 can be discriminated whilst *in situ* on the surface of a cell, for example a T-cell.

### MALIGNANT CELLS

The term 'malignant' is used herein according to its standard meaning to refer to a cell which is not self-limited in its growth, may be capable of invading into adjacent tissues and may be capable of spreading to distant tissue. As such the term 'malignant T cell' is used herein to refer to a clonally expanded T cell in the context of a lymphoma or leukaemia.

The present invention involves determining the TRBC of a malignant T-cell. This may be performed using methods known in the art. For example it may be determined by PCR, western blot, flow cytometry or fluorescent microscopy methods.

Once the TRBC expressed by a malignant T-cell has been determined, the appropriate TRBC1 or TRBC2 selective agent is administered to the subject. The 'appropriate TRBC selective agent' means that where the malignant T-cell is determined to express TRBC1, a TRBC1 selective agent is administered, whereas where the malignant T-cell is determined to express TRBC2, a TRBC2 selective agent is administered.

### SELECTIVE AGENT

The selective agent binds to either TRBC1 or TRBC2 in a mutually exclusive manner.

As stated above, each αβ T-cell expresses a TCR which comprises either TRBC1 or TRBC2. In a clonal T-cell disorder, such as a T-cell lymphoma or leukaemia, malignant T-cells derived from the same clone will all express either TRBC1 or TRBC2.

Thus the present disclosure comprises the step of administering a TRBC1 or TRBC2 selective agent to the subject, wherein the agent causes selective depletion of the malignant T-cells, together with normal T-cells which express the same TRBC as the malignant T-cells, but does not cause significant depletion of normal T-cells expressing the other TRBC from the malignant T-cells.

Because the TRBC selective agent does not cause significant depletion of normal T-cells expressing the other TRBC from the malignant T-cells it does not cause depletion of the entire T-cell compartment. Retention of a proportion of the subject's T-cell compartment (i.e. T-cells which do not express the same TRBC as the malignant T-cell) results in reduced toxicity and reduced cellular and humoral immunodeficiency, thereby reducing the risk of infection.

Administration of a TRBC1 selective agent according to the present disclosure may result in a 5, 10, 20, 50, 75, 90, 95 or 99% depletion, i.e. reduction in the number of T-cells expressing TRBC1.

Administration of a TRBC2 selective agent according to the present disclosure may result in a 5, 10, 20, 50, 75, 90, 95 or 99% depletion, i.e.reduction in the number of T-cells expressing TRBC2.

A TRBC1 selective agent may bind TRBC1 with an at least 2-fold, 4-fold, 5-fold, 7-fold or 10-fold greater affinity that TRBC2. Likewise, a TRBC2 selective agent may bind TRBC2 with an at least 2-fold, 4-fold, 5-fold, 7-fold or 10-fold greater affinity that TRBC1.

A TRBC1 selective agent causes depletion of a greater proportion of TRBC1-expressing T-cells in cell population than TRBC2-expressing cell. For example, the ratio of depletion of TRBC1-expressing T-cells to TRBC2-expressing cells may be at least 60%:40%, 70%:30%, 80%:20%, 90%:10% or 95%:5%. Likewise, a TRBC2 selective agent causes depletion of a greater proportion of TRBC1-expressing T-cells in cell population than TRBC2-expressing cell. For example, the ratio of depletion of TRBC2-expressing T-cells to TRBC1-expressing cells may be at least 60%:40%, 70%:30%, 80%:20%, 90%:10% or 95%:5%.

Using the method of the disclosure, malignant T-cells are deleted in a subject, without affecting a significant proportion of healthy T cells. By "a significant proportion" it is meant that a sufficient proportion of T cells expressing the TRBC different from the maltgnant T cells survive in order to maintain T-cell function in the subject. The agent may cause depletion of less than 20%, 15%, 10% or 5% of the T-cell population expressing the other TCRB.

The selective agent may be selective for either TRBC1 or TRBC2 because it discriminates residues as listed below:
(i) N from K at position 3 of the TRBC;
(ii) K from N at position 3 of the TRBC;
(iii) K from N at position 4 of the TRBC;
(iv) N from K at position 4 of the TRBC;
(v) F from Y at position 36 of the TRBC;
(vi) Y from F at position 36 of the TRBC;
(vii) V from E at position 135 of the TRBC; and/or
(viii) E from V at position 135 of the TRBC.

The selective agent may discriminate any combination of the differences above differences.

### ANTIBODY

The agent used in the method of the present disclosure may be a depleting monoclonal antibody (mAb) or a functional fragment thereof, or an antibody mimetic.

The term 'depleting antibody' is used in the conventional sense to relate to an antibody which binds to an antigen present on a target T-cell and mediates death of the target T-cell. The administration of a depleting antibody to a subject therefore results in a reduction/decrease in the number of cells within the subject which express the target antigen.

As used herein, "antibody" means a polypeptide having an antigen binding site which comprises at least one complementarity determining region CDR. The antibody may comprise 3 CDRs and have an antigen binding site which is equivalent to that of a domain antibody (dAb). The antibody may comprise 6 CDRs and have an antigen binding site which is equivalent to that of a classical antibody molecule. The remainder of the polypeptide may be any sequence which provides a suitable scaffold for the antigen binding site and displays it in an appropriate manner for it to bind the antigen. The antibody may be a whole immunoglobulin molecule or a part thereof such as a Fab, F(ab)'2, Fv, single chain Fv (ScFv) fragment or Nanobody. The antibody may be a bifunctional antibody. The antibody may be non-human, chimeric, humanised or fully human.

The antibody may therefore be any functional fragment which retains the antigen specificity of the full antibody.

### TRBC1-SELECTIVE ANTIBODIES

The agent of the present invention for use in the method of the present disclosure may comprise an antibody or a functional fragment thereof having a variable heavy chain (VH) and a variable light chain (VL) which comprises one or more of the following complementarity determining regions (CDRs):
VH CDR1: GYTFTGY (SEQ ID No. 7);
VH CDR2: NPYNDD (SEQ ID No. 8);
VH CDR3: GAGYNFDGAYRFFDF (SEQ ID No. 9);
VL CDR1: RSSQRLVHSNGNTYLH (SEQ ID No. 10);
VL CDR2: RVSNRFP (SEQ ID No. 11); and
VL CDR3: SQSTHVPYT (SEQ ID No. 12).

The one or more CDRs each independently may or may not comprise one or more amino acid mutations (eg substitutions) compared to the sequences given as SEQ ID No. 7 to 12, provided that the resultant antibody retains the ability to selectively bind to TRBC1.

Studies have shown that CDRs L3 and H3 are prevalently responsible for high energy interactions with the antigen, so the antibody or functional fragment thereof, may comprise VH CDR3 and/or VL CDR3 as outlined above.

Using phage display, several additional antibody binding domains have been identified which are highly selective for binding TRBC1 over TRBC2, as described in Example 12.

The agent may comprise an antibody or a functional fragment thereof having a variable heavy chain (VH) and/or a variable light chain (VL) which comprises one or more of the complementarity determining regions (CDR3s) shown in the Table 1.

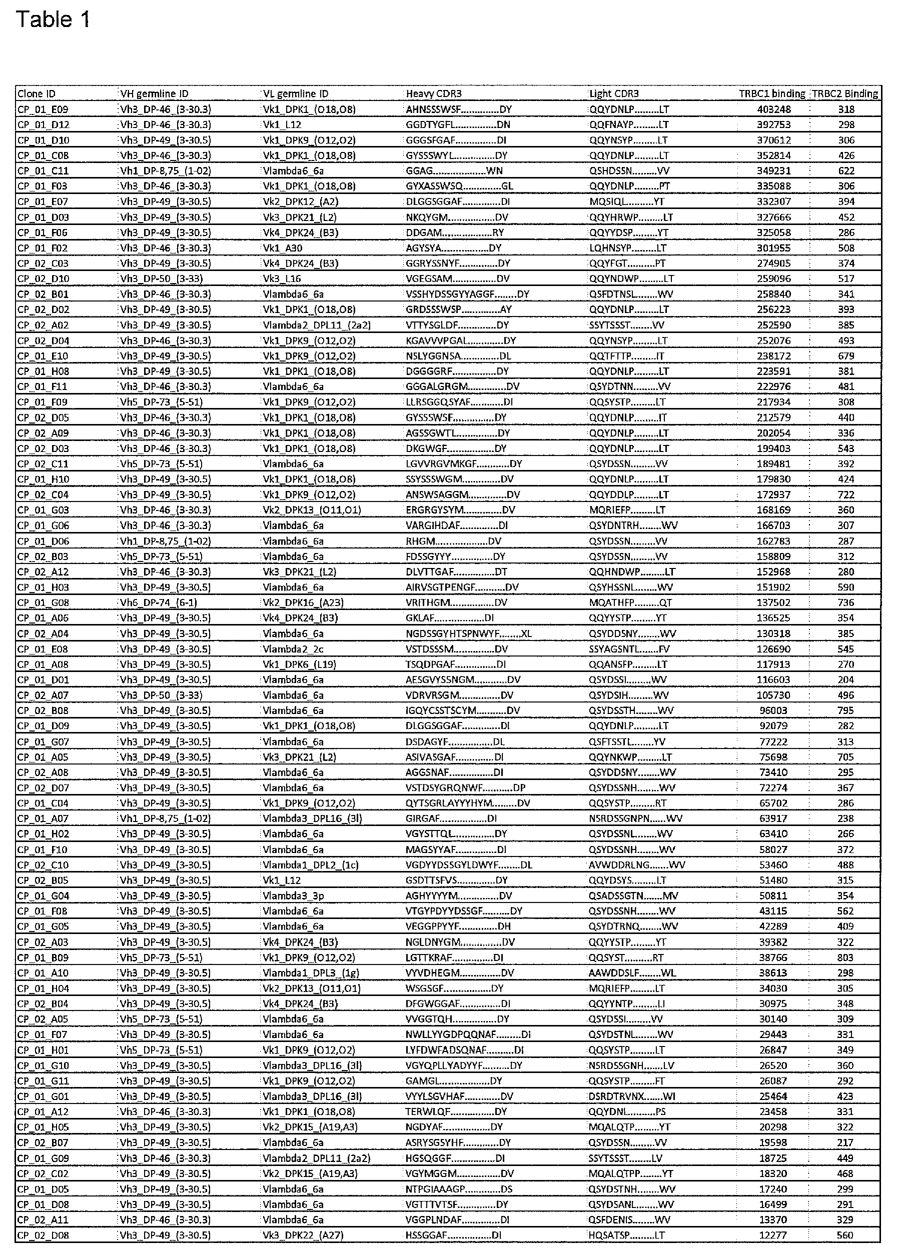

Where the agent is a domain antibody it may comprise 3 CDRs, i.e. either VH CDR1-CDR3 or VL CDR1-CDR3.

The agent may comprise an antibody of functional fragment thereof which comprises a variable heavy chain (VH) having the amino acid sequence shown as SEQ ID No. 1 and a variable light chain (VL) having the amino acid sequence shown as SEQ ID No. 2.
SEQ_lD_1 Jovi-1 VH
SEQ_ID_2 Jovi-1 VL

The agent may comprise an ScFv having the amino acid sequence shown as SEQ ID No. 3.
SEQ_ID_3 Jovi-1 scFv

Alternatively, the agent may comprise an antibody or functional fragment thereof which comprises:
(i) the heavy chain CDR3 and/or the light chain CDR3;
(ii) heavy chain CDR1, CDR2 and CDR3 and/or light chain CDR1, CDR2 and CDR3; or
(iii) the variable heavy chain (VH) and/or the variable light chain (VL);
from one of the scFvs shown as SEQ ID No. 13-22.

In the sequences shown as SEQ ID No. 13-22, the VH and VL portions of the sequence are shown in bold and the CDR1 and CDR2 sequences for the heavy and light chains are underlined. The CDR3 sequences for VH and VL are given in Table 1.
SEQ ID No. 13_(CP_01_E09)
SEQ ID No. 14 (CP_01_D12)
SEQ ID No. 15 (CP_01_D10)
SEQ ID No. 16 (CP_01_C08)
SEQ ID No. 17 (CP_01_C11)
SEQ ID No. 18 (CP_01_F03)
SEQ ID No. 19 (CP_01_E07)
SEQ ID No. 20 (CP_01_D03)
SEQ ID No. 21 (CP_01_F06)
SEQ ID No. 22 (CP_01_F02)

### TRBC2-SPECIFIC

Using phage display, several antibody binding domains have been identified which are highly selective for binding TRBC2 over TRBC1, as described in Example 12.

The agent may comprise an antibody or a functional fragment thereof having a variable heavy chain (VH) and/or a variable light chain (VL) which comprises one or more of the complementarity determining regions (CDR3s) shown in the Table 2.

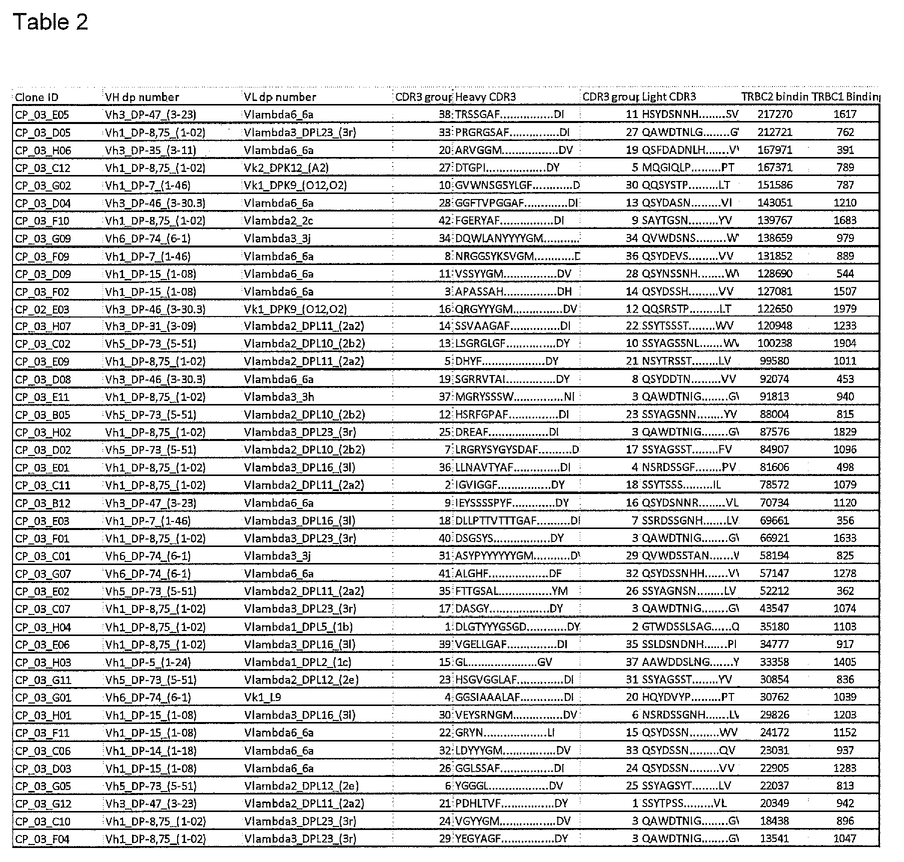

The agent may comprise an antibody or functional fragment thereof which comprises:
(i) the heavy chain CDR3 and/or the light chain CDR3;
(ii) Heavy chain CDR1, CDR2 and CDR3 and/or light chain CDR1, CDR2 and CDR3; or
(iii) the variable heavy chain (VH) and/or the variable light chain (VL);
from one of the scFvs shown as SEQ ID No. 23-32.

In the sequences shown as SEQ ID No. 23-32, the VH and VL portions of the sequence are shown in bold and the CDR1 and CDR2 sequences for the heavy and light chains are underlined. The CDR3 sequences for VH and VL are given in Table 2.
SEQ ID No. 23 (CP_03_E05)
SEQ ID No. 24 (CP_03_D05)
SEQ ID No. 25 (CP_03_H06)
SEQ ID No. 26 (CP_03_C12)
SEQ ID No. 27 (CP_03_G02)
SEQ ID No. 28 (CP_03_D04)
SEQ ID No. 29 (CP_03_F10)
SEQ ID No. 30 (CP_03_G09)
SEQ ID No. 31 (CP_03_F09)
SEQ ID No. 32 (CP_03_D09)

Variants of the above amino acid sequences may also be used in the present disclosure provided that the resulting antibody binds TRBC1 or TRBC2 and does not significantly cross-react. Typically such variants have a high degree of sequence identity with one of the sequences specified above.

Methods of alignment of sequences for comparison are well known in the art.

The NCBI Basic Local Alignment Search Tool (BLAST) is available from several sources, including the National Center for Biotechnology Information (NCBI, Bethesda, Md.) and on the internet, for use in connection with the sequence analysis programs blastp, blastn, blastx, tblastn and tblastx. A description of how to determine sequence identity using this program is available on the NCBI website on the internet. Variants of the VL or VH domain or scFv typically have at least about 75%, for example at least about 80%, 90%, 95%, 96%, 97%, 98% or 99% sequence identity with the sequences given as SEQ ID Nos 1-3, 13-32..

Typically variants may contain one or more conservative amino acid substitutions compared to the original amino acid or nucleic acid sequence. Conservative substitutions are those substitutions that do not substantially affect or decrease the affinity of an antibody to bind TRBC1 or TRBC2. For example, a human antibody that specifically binds TRBC1 or TRBC2 may include up to 1, up to 2, up to 5, up to 10, or up to 15 conservative substitutions in either or both of the VH or VL compared to any of the sequences given as SEQ ID No. 1-3 or 13-32 and retain specific binding to TRBC1 or TRBC2.

Functionally similar amino acids which may be exchanged by way of conservative substitution are well known to one of ordinary skill in the art. The following six groups are examples of amino acids that are considered to be conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); and 6) Phenylalanine (F), Tyrosine (Y), Tryptophan (W).

### PREPARATION OF ANTIBODIES

Preparation of antibodies may be performed using standard laboratory techniques. Antibodies may be obtained from animal serum, or, in the case of monoclonal antibodies or fragments thereof, produced in cell culture. Recombinant DNA technology may be used to produce the antibodies according to established procedure, in bacterial or mammalian cell culture.

Methods for the production of monoclonal antibodies are well known in the art. Briefly, monoclonal antibodies are typically made by fusing myeloma cells with the spleen cells from a mouse or rabbit that has been immunized with the desired antigen. Herein, the desired antigen is TRBC1 or TRBC2 peptide, or a TCRβ chain comprising either TRBC1 or TRBC2.

Alternatively, antibodies and related molecules, particularly scFvs, may be made outside the immune system by combining libraries of VH and VL chains in a recombinant manner. Such libraries may be constructed and screened using phage-display technology as described in Example 12.

### IDENTIFICATION OF TRBC1/TRBC2 SELECTIVE ANTIBODIES

Antibodies which are selective for either TRBC1 or TRBC2 may be identified using methods which are standard in the art. Methods for determining the binding specificity of an antibody include, but are not limited to, ELISA, western blot, immunohistochemistry, flow cytometry, Förster resonance energy transfer (FRET), phage display libraries, yeast two-hybrid screens, co-immunoprecipitation, bimolecular fluorescence complementation and tandem affinity purification.

To identify an antibody which is selective for either TRBC1 or TRBC2 the binding of the antibody to each of TRBC1 and TRBC2 is assessed. Typically, this is assessed by determining the binding of the antibody to each TRBC separately. An antibody which is selective binds to either TRBC1 or TRBC2 without significant binding to the other TRBC.

### ANTIBODY MIMETICS

The agent may alternatively be a molecule which is not derived from or based on an immunoglobulin. A number of "antibody mimetic" designed repeat proteins (DRPs) have been developed to exploit the binding abilities of non-antibody polypeptides.

Repeat proteins such as ankyrin or leucine-rich repeat proteins are ubiquitous binding molecules which occur, unlike antibodies, intra- and extracellularly. Their unique modular architecture features repeating structural units (repeats), which stack together to form elongated repeat domains displaying variable and modular target-binding surfaces. Based on this modularity, combinatorial libraries of polypeptides with highly diversified binding specificities can be generated. DARPins (Designed Ankyrin Repeat Proteins) are one example of an antibody mimetic based on this technology.

For Anticalins, the binding specificity is derived from lipocalins, a family of proteins which perform a range of functions in vivo associated with physiological transport and storage of chemically sensitive or insoluble compounds. Lipocalins have a robust intrinsic structure comprising a highly conserved β-barrel which supports four loops at one terminus of the protein. These loops for the entrance to a binding pocket and conformational differences in this part of the molecule account for the variation in binding specificity between different lipocalins.

Avimers are evolved from a large family of human extracellular receptor domainsby in vitro exon shuffling and phage display, generating multi-domain proteins with binding and inhibitory properties.

Versabodies are small proteins of 3-5kDa with >15% cysteines which form a high disulfide density scaffold, replacing the hydrophobic core present in most proteins. The replacement of a large number of hydrophobic amino acids, comprising the hydrophobic core, with a small number of disulphides results in a protein that is smaller, more hydrophilic, more resistant to proteases and heat and has a lower density of T-cell epitopes. All four of these properties result in a protein having considerably reduced immunogenicity. They may also be manufactured in E. coli, and are highly soluble and stable.

### CONJUGATES

The antibody or mimetic may be a conjugate of the antibody or mimetic and another agent or antibody, for example the conjugate may be a detectable entity or a chemotherapeutic entity.

The detectable entity may be a fluorescent moiety, for example a fluorescent peptide. A "fluorescent peptide" refers to a polypeptide which, following excitation, emits light at a detectable wavelength. Examples of fluorescent proteins include, but are not limited to, fluorescein isothiocyanate (FITC), phycoerythrin (PE), allophycocyanin (APC), green fluorescent protein (GFP), enhanced GFP, red fluorescent protein (RFP), blue fluorescent protein (BFP) and mCherry.

A selective TRBC1 or TRBC2 agent conjugated to a detectable entity may be used to determine the TRBC of a malignant T cell.

A chemotherapeutic entity as used herein refers to an entity which is destructive to a cell, that is the entity reduces the viability of the cell. The chemotherapeutic entity may be a cytotoxic drug. A chemotherapeutic agent contemplated includes, without limitation, alkylating agents, nitrosoureas, ethylenimines/methylmelamine, alkyl sulfonates, antimetabolites, pyrimidine analogs, epipodophylotoxins, enzymes such as L-asparaginase; biological response modifiers such as IFNα, IL-2, G-CSF and GM-CSF; platinium coordination complexes such as cisplatin and carboplatin, anthracenediones, substituted urea such as hydroxyurea, methylhydrazine derivatives including N-methylhydrazine (MIH) and procarbazine, adrenocortical suppressants such as mitotane (o,p'-DDD) and aminoglutethimide; hormones and antagonists including adrenocorticosteroid antagonists such as prednisone and equivalents, dexamethasone and aminoglutethimide; progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; and non-steroidal antiandrogens such as flutamide.

A TRBC selective agent conjugated to a chemotherapeutic entity enables the targeted delivery of the chemotherapeutic entity to cells which express either TRBC1 or TRBC2.

### BI-SPECIFIC T-CELL ENGAGERS

A wide variety of molecules have been developed which are based on the basic concept of having two antibody-like binding domains.

Bispecific T-cell engaging molecules are a class of bispecific antibody-type molecules that have been developed, primarily for the use as anti-cancer drugs. They direct a host's immune system, more specifically the T cells' cytotoxic activity, against a target cell, such as a cancer cell. In these molecules, one binding domain binds to a T cell via the CD3 receptor, and the other to a target cells such as a tumor cell (via a tumor specific molecule). Since the bispecific molecule binds both the target cell and the T cell, it brings the target cell into proximity with the T cell, so that the T cell can exert its effect, for example, a cytotoxic effect on a cancer cell. The formation of the T cell:bispecific Ab:cancer cell complex induces signaling in the T cell leading to, for example, the release of cytotoxic mediators. Ideally, the agent only induces the desired signaling in the presence of the target cell, leading to selective killing.

Bispecific T-cell engaging molecules have been developed in a number of different formats, but one of the most common is a fusion consisting of two single-chain variable fragments (scFvs) of different antibodies. These are sometimes known as BiTEs (Bi-specific T-cell Engagers).

The agent used in the method of the present disclosure may be a bi-specific molecule which selectively recognises TRBC1 or TRBC2 and is capable of activating a T cell. For example the agent may be a BiTE. The agent used in the method may comprise:
(i) a first domain which binds either TRBC1 or TRBC2; and
(ii) a second domain capable of activating a T cell.

The bi-specific molecule may comprise a signal peptide to aid in its production. The signal peptide may cause the bi-specific molecule to be secreted by a host cell, such that the bi-specific molecule can be harvested from the host cell supernatant.

The signal peptide may be at the amino terminus of the molecule. The bi-specific molecule may have the general formula: Signal peptide - first domain - second domain.

The bi-specific molecule may comprise a spacer sequence to connect the first domain with the second domain and spatially separate the two domains.

The spacer sequence may, for example, comprise an IgG1 hinge or a CD8 stalk. The linker may alternatively comprise an alternative linker sequence which has similar length and/or domain spacing properties as an IgG1 hinge or a CD8 stalk.

The bi-specific molecule may comprise JOVI-1, or a functional fragment thereof, as defined above.

### CHIMERIC ANTIGEN RECEPTOR (CAR)

Chimeric antigen receptors (CARs), also known as chimeric T-cell receptors, artificial T-cell receptors and chimeric immunoreceptors, are engineered receptors, which graft an arbitrary specificity onto an immune effector cell. In a classical CAR, the specificity of a monoclonal antibody is grafted on to a T-cell. CAR-encoding nucleic acids may be transferred to T-cells using, for example, retroviral vectors. In this way, a large number of cancer-specific T-cells can be generated for adoptive cell transfer. Phase I clinical studies of this approach show efficacy.

The target-antigen binding domain of a CAR is commonly fused via a spacer and transmembrane domain to an endodomain, which comprises or associates with an intrcellular T-cell signalling domain. When the CAR binds the target-antigen, this results in the transmission of an activating signal to the T-cell it is expressed on.

The agent used in the method of the present disclosure may be a CAR which selectively recognises TRBC1 or TRBC2. The agent may be a T-cell which expresses a CAR which selectively recognises TRBC1 or TRBC2.

The CAR may also comprise a transmembrane domain which spans the membrane. It may comprise a hydrophobic alpha helix. The transmembrane domain may be derived from CD28, which gives good receptor stability.

The endodomain is the portion of the CAR involved in signal-transmission. The endodomain either comprises or associates with an intracellular T-cell signalling domain. After antigen recognition, receptors cluster and a signal is transmitted to the cell. The most commonly used T-cell signalling component is that of CD3-zeta which contains 3 ITAMs. This transmits an activation signal to the T-cell after antigen is bound. CD3-zeta may not provide a fully competent activation signal and additional co-stimulatory signaling may be needed. For example, chimeric CD28 and OX40 can be used with CD3-Zeta to transmit a proliferative / survival signal, or all three can be used together.

The endodomain of the CAR may comprise the CD28 endodomain and OX40 and CD3-Zeta endodomain.

The CAR may comprise a signal peptide so that when the CAR is expressed inside a cell, such as a T-cell, the nascent protein is directed to the endoplasmic reticulum and subsequently to the cell surface, where it is expressed.

The CAR may comprise a spacer sequence to connect the TRBC-binding domain with the transmembrane domain and spatially separate the TRBC-binding domain from the endodomain. A flexible spacer allows to the TRBC-binding domain to orient in different directions to enable TRBC binding.

The spacer sequence may, for example, comprise an IgG1 Fc region, an IgG1 hinge or a CD8 stalk, or a combination thereof. The linker may alternatively comprise an alternative linker sequence which has similar length and/or domain spacing properties as an IgG1 Fc region, an IgG1 hinge or a CD8 stalk.

It was found that CARs comprising a spacer based on an IgG1 hinge or a CD8 stalk showed the best performance against Jurkat cells (Figure 15). The spacer may an therefore comprise an IgG1 hinge or a CD8 stalk or a spacer which has a similar length and/or domain spacing properties as an IgG1 hinge or a CD8 stalk.

A human IgG1 spacer may be altered to remove Fc binding motifs.

The CAR may comprise the JOVI-1 antibody, or a functional fragment thereof, as defined above.

The CAR may comprise an amino acid sequence selected from the group consisting of SEQ ID No. 33, 34 and 35.
>SEQ_ID_33 JOVI-1 CAR with CD8 stalk spacer
>SEQ_ID_34 JOVI-1 CAR with H-CH2-CH3pvaa spacer
>SEQ_ID_35 JOVI-1 CAR with IgG1 hinge spacer

In the CAR sequences given above, one or more of the 6 CDRs each independently may or may not comprise one or more amino acid mutations (eg substitutions) compared to the sequences given as SEQ ID No. 7 to 12, provided that the resultant CAR retains the ability to bind to TRBC1.

Variants of the above amino acid sequences may also be used in the present disclosure provided that the resulting CAR binds TRBC1 or TRBC2 and does not significantly cross-react. Typically such variants have a high degree of sequence identity with one of the sequences given as SEQ ID No. 33, 34 or 35.

Variants of the CAR typically have at least about 75%, for example at least about 80%, 90%, 95%, 96%, 97%, 98% or 99% sequence identity with one of the sequences given as SEQ ID Nos 33, 34 and 35.

### NUCLEIC ACID

The present disclosure further provides a nucleic acid encoding an agent such as a BiTE or CAR of the first aspect of the disclosure.

The nucleic acid sequence may encode a CAR comprising one of the amino acid sequences shown as SEQ ID No. 33, 34 and 35..

As used herein, the terms "polynucleotide", "nucleotide", and "nucleic acid" are intended to be synonymous with each other.

It will be understood by a skilled person that numerous different polynucleotides and nucleic acids can encode the same polypeptide as a result of the degeneracy of the genetic code. In addition, it is to be understood that skilled persons may, using routine techniques, make nucleotide substitutions that do not affect the polypeptide sequence encoded by the polynucleotides described here to reflect the codon usage of any particular host organism in which the polypeptides are to be expressed.

Nucleic acids according to the invention may comprise DNA or RNA. They may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the use as described herein, it is to be understood that the polynucleotides may be modified by any method available in the art. Such modifications may be carried out in order to enhance the in vivo activity or life span of polynucleotides of interest.

The terms "variant", "homologue" or "derivative" in relation to a nucleotide sequence include any substitution of, variation of, modification of, replacement of, deletion of or addition of one (or more) nucleic acid from or to the sequence.

### VECTOR

The present disclosure also provides a vector, or kit of vectors, which comprises one or more nucleic acid sequence(s) of the disclosure. Such a vector may be used to introduce the nucleic acid sequence(s) into a host cell so that it expresses a CAR according to the first aspect of the disclosure

The vector may, for example, be a plasmid or a viral vector, such as a retroviral vector or a lentiviral vector, or a transposon based vector or synthetic mRNA.

The vector may be capable of transfecting or transducing a T cell or a NK cell.

### CELL

The present disclosure also relates to a cell, such as an immune cell, comprising a CAR according to the first aspect of the disclosure.

The cell may comprise a nucleic acid or a vector of the present disclosure

The cell may be a T-cell or a natural killer (NK) cell.

T cell may be T cells or T lymphocytes which are a type of lymphocyte that play a central role in cell-mediated immunity. They can be distinguished from other lymphocytes, such as B cells and natural killer cells (NK cells), by the presence of a T-cell receptor (TCR) on the cell surface. There are various types of T cell, as summarised below.

Helper T helper cells (TH cells) assist other white blood cells in immunologic processes, including maturation of B cells into plasma cells and memory B cells, and activation of cytotoxic T cells and macrophages. TH cells express CD4 on their surface. TH cells become activated when they are presented with peptide antigens by MHC class II molecules on the surface of antigen presenting cells (APCs). These cells can differentiate into one of several subtypes, including TH1, TH2, TH3, TH17, Th9, or TFH, which secrete different cytokines to facilitate different types of immune responses.

Cytolytic T cells (TC cells, or CTLs) destroy virally infected cells and tumor cells, and are also implicated in transplant rejection. CTLs express the CD8 at their surface. These cells recognize their targets by binding to antigen associated with MHC class I, which is present on the surface of all nucleated cells. Through IL-10, adenosine and other molecules secreted by regulatory T cells, the CD8+ cells can be inactivated to an anergic state, which prevent autoimmune diseases such as experimental autoimmune encephalomyelitis.

Memory T cells are a subset of antigen-specific T cells that persist long-term after an infection has resolved. They quickly expand to large numbers of effector T cells upon re-exposure to their cognate antigen, thus providing the immune system with "memory" against past infections. Memory T cells comprise three subtypes: central memory T cells (TCM cells) and two types of effector memory T cells (TEM cells and TEMRA cells). Memory cells may be either CD4+ or CD8+. Memory T cells typically express the cell surface protein CD45RO.

Regulatory T cells (Treg cells), formerly known as suppressor T cells, are crucial for the maintenance of immunological tolerance. Their major role is to shut down T cell-mediated immunity toward the end of an immune reaction and to suppress auto-reactive T cells that escaped the process of negative selection in the thymus.

Two major classes of CD4+ Treg cells have been described - naturally occurring Treg cells and adaptive Treg cells.

Naturally occurring Treg cells (also known as CD4+CD25+FoxP3+ Treg cells) arise in the thymus and have been linked to interactions between developing T cells with both myeloid (CD11c+) and plasmacytoid (CD123+) dendritic cells that have been activated with TSLP. Naturally occurring Treg cells can be distinguished from other T cells by the presence of an intracellular molecule called FoxP3. Mutations of the FOXP3 gene can prevent regulatory T cell development, causing the fatal autoimmune disease IPEX.

Adaptive Treg cells (also known as Tr1 cells or Th3 cells) may originate during a normal immune response.

The cell may be a Natural Killer cell (or NK cell). NK cells form part of the innate immune system. NK cells provide rapid responses to innate signals from virally infected cells in an MHC independent manner
NK cells (belonging to the group of innate lymphoid cells) are defined as large granular lymphocytes (LGL) and constitute the third kind of cells differentiated from the common lymphoid progenitor generating B and T lymphocytes. NK cells are known to differentiate and mature in the bone marrow, lymph node, spleen, tonsils and thymus where they then enter into the circulation.

The CAR cells of the disclosure may be any of the cell types mentioned above.

T or NK cells expressing the a CAR according to the first aspect of the invention may either be created *ex vivo* either from a patient's own peripheral blood (1st party), or in the setting of a haematopoietic stem cell transplant from donor peripheral blood (2nd party), or peripheral blood from an unconnected donor (3rd party).

Alternatively, T or NK cells expressing a CAR according to the first aspect of the invention may be derived from *ex vivo* differentiation of inducible progenitor to T or NK cells. Alternatively, an immortalized T-cell line which retains its lytic function and could act as a therapeutic may be used.

In all these aspects CAR cells are generated by introducing DNA or RNA coding for the CAR by one of many means including transduction with a viral vector, transfection with DNA or RNA.

The CAR cell of the invention may be an ex vivo T or NK cell from a subject. The T or NK cell may be from a peripheral blood mononuclear cell (PBMC) sample. T or NK cells may be activated and/or expanded prior to being transduced with nucleic acid encoding a CAR according to the first aspect of the invention, for example by treatment with an anti-CD3 monoclonal antibody.

The T or NK cell of the invention may be made by:
(i) isolation of a T or NK cell-containing sample from a subject or other sources listed above; and
(ii) transduction or transfection of the T or NK cells with a nucleic acid sequence(s) encoding a CAR of the invention.

The T or NK cells may then by purified, for example, selected on the basis of expression of the antigen-binding domain of the antigen-binding polypeptide.

The present disclosure also provides a kit which comprises a T or NK cell comprising a CAR according to the first aspect of the invention.

### PHARMACEUTICAL COMPOSITION

The present invention also relates to a pharmaceutical composition containing a plurality of cells expressing a CAR of the first aspect of the invention. The pharmaceutical composition may additionally comprise a pharmaceutically acceptable carrier, diluent or excipient. The pharmaceutical composition may optionally comprise one or more further pharmaceutically active polypeptides and/or compounds. Such a formulation may, for example, be in a form suitable for intravenous infusion.

### T-CELL LYMPHOMA AND/OR LEUKAEMIA

The present invention relates to agents and cells for use in methods for treating a T-cell lymphoma and/or leukaemia.

A method for treating a T-cell lymphoma and/or leukaemia relates to the therapeutic use of an agent. Herein the agent may be administered to a subject having an existing disease of T-cell lymphoma and/or leukaemia in order to lessen, reduce or improve at least one symptom associated with the disease and/or to slow down, reduce or block the progression of the disease.

The method of the present disclosure may be used for the treatment of any lymphoma and/or leukaemia associated with the clonal expansion of a cell expressing a T-cell receptor (TCR) comprising a β constant region. As such the present document discloses a method for treating a disease which involves malignant T cells which express a TCR comprising a TRBC.

The method of the present disclosure may be used to treat a T-cell lymphoma in which the malignant T-cell expresses a TCR comprising a TRBC. 'Lymphoma' is used herein according to its standard meaning to refer to a cancer which typically develops in the lymph nodes, but may also affect the spleen, bone marrow, blood and other organs. Lymphoma typically presents as a solid tumour of lymphoid cells. The primary symptom associated with lymphoma is lymphadenopathy, although secondary (B) symptoms can include fever, night sweats, weight loss, loss of appetite, fatigue, respiratory distress and itching.

The method of the present disclosure may be used to treat a T-cell leukaemia in which the malignant T-cell expresses a TCR comprising a TRBC. 'Leukaemia' is used herein according to its standard meaning to refer to a cancer of the blood or bone marrow.

The following is an illustrative, non-exhaustive list of diseases which may be treated by the method of the present disclosure.

### PERIPHERAL T-CELL LYMPHOMA

Peripheral T-cell lymphomas are relatively uncommon lymphomas and account fewer than 10% of all non-Hodgkin lymphomas (NHL). However, they are associated with an aggressive clinical course and the causes and precise cellular origins of most T-cell lymphomas are still not well defined.

Lymphoma usually first presents as swelling in the neck, underarm or groin. Additional swelling may occur where other lymph nodes are located such as in the spleen. In general, enlarged lymph nodes can encroach on the space of blood vessels, nerves, or the stomach, leading to swollen arms and legs, to tingling and numbness, or to feelings of being full, respectively. Lymphoma symptoms also include nonspecific symptoms such as fever, chills, unexplained weight loss, night sweats, lethargy, and itching.

The WHO classification utilizes morphologic and immunophenotypic features in conjunction with clinical aspects and in some instances genetics to delineate a prognostically and therapeutically meaningful categorization for peripheral T-cell lymphomas (Swerdlow et al.; WHO classification of tumours of haematopoietic and lymphoid tissues. 4th ed.; Lyon: IARC Press; 2008). The anatomic localization of neoplastic T-cells parallels in part their proposed normal cellular counterparts and functions and as such T-cell lymphomas are associated with lymph nodes and peripheral blood. This approach allows for better understanding of some of the manifestations of the T-cell lymphomas, including their cellular distribution, some aspects of morphology and even associated clinical findings.

The most common of the T-cell lymphomas is peripheral T-cell lymphoma, not otherwise specified (PTCL-NOS) comprising 25% overall, followed by angioimmunoblastic T-cell lymphoma (AITL) (18.5%)

### PERIPHERAL T-CELL LYMPHOMA, NOT OTHERWISE SPECIFIED (PTCL-NOS)

PTCL-NOS comprises over 25% of all peripheral T-cell lymphomas and NK/T-cell lymphomas and is the most common subtype. It is determined by a diagnosis of exclusion, not corresponding to any of the specific mature T-cell lymphoma entities listed in the current WHO 2008. As such it is analogous to diffuse large B-cell lymphoma, not otherwise specified (DLBCL-NOS).

Most patients are adults with a median age of 60 and a male to female ratio 2:1. The majority of cases are nodal in origin, however, extranodal presentations occur in approximately 13% of patients and most commonly involve skin and gastrointestinal tract.

The cytologic spectrum is very broad, ranging from polymorphous to monomorphous. Three morphologically defined variants have been described, including lymphoepithelioid (Lennert) variant, T-zone variant and follicular variant. The lymphoepithelioid variant of PTCL contains abundant background epithelioid histiocytes and is commonly positive for CD8. It has been associated with a better prognosis. The follicular variant of PTCL-NOS is emerging as a potentially distinct clinicopathologic entity.

The majority of PTCL-NOS have a mature T-cell phenotype and most cases are CD4-positive. 75% of cases show variable loss of at least one pan T-cell marker (CD3, CD2, CD5 or CD7), with CD7 and CD5 being most often downregulated. CD30 and rarely CD15 can be expressed, with CD15 being an adverse prognostic feature. CD56 expression, although uncommon, also has negative prognostic impact. Additional adverse pathologic prognostic factors include a proliferation rate greater than 25% based on KI-67 expression, and presence of more than 70% transformed cells. Immunophenotypic analysis of these lymphomas has offered little insight into their biology.

### ANGIOIMMUNOBLASTIC T-CELL LYMPHOMA (AITL)

AITL is a systemic disease characterized by a polymorphous infiltrate involving lymph nodes, prominent high endothelial venules (HEV) and peri-vascular expansion of follicular dendritic cell (FDC) meshworks. AITL is considered as a *de-novo* T-cell lymphoma derived from αβ T-cells of follicular helper type (TFH), normally found in the germinal centres.

AITL is the second most common entity among peripheral T-cell lymphoma and NK/T-cell lymphomas, comprising about 18.5% of cases. It occurs in middle aged to elderly adults, with a median age of 65 years old, and an approximately equal incidence in males and females. Clinically, patients usually have advanced stage disease, with generalized lymphadenopathy, hepatosplenomegaly and prominent constitutional symptoms. Skin rash with associated pruritus is commonly present. There is often polyclonal hypergammaglobulinemia, associated with autoimmune phenomena.

Three different morphologic patterns are described in AITL. The early lesion of AITL (Pattern I) usually shows preserved architecture with characteristic hyperplastic follicles. The neoplastic proliferation is localized to the periphery of the follicles. In Pattern II the nodal architecture is partially effaced with retention of few regressed follicles. The subcapsular sinuses are preserved and even dilated. The paracortex contains arborizing HEV and there is a proliferation of FDC beyond the B-cell follicle. The neoplastic cells are small to medium in size, with minimal cytologic atypia. They often have clear to pale cytoplasm, and may show distincT-cell membranes. A polymorphous inflammatory background is usually evident.

Although AITL is a T-cell malignancy, there is a characteristic expansion of B-cells and plasma cells, which likely reflects the function of the neoplastic cells as TFH cells. Both EBV-positive and EBV-negative B-cells are present. Occasionally, the atypical B-cells may resemble Hodgkin/Reed-Sternberg-like cells morphologically and immunophenotypically, sometimes leading to a diagnostic confusion with that entity. The B-cell proliferation in AITL may be extensive and some patients develop secondary EBV-positive diffuse large B-cell lymphomas (DLBCL) or - more rarely - EBV-negative B-cell tumors, often with plasmacytic differentiation.

The neoplastic CD4-positive T-cells of AITL show strong expression of CD10 and CD279 (PD-1) and are positive for CXCL13. CXCL13 leads to an increased B-cell recruitment to lymph nodes via adherence to the HEV, B-cell activation, plasmacytic differentiation and expansion of the FDC meshworks, all contributing to the morphologic and clinical features of AITL. Intense PD-1-expression in the perifollicular tumor cells is particularly helpful in distinguishing AITL Pattern I from reactive follicular and paracortical hyperplasia.

The follicular variant of PTCL-NOS is another entity with a TFH phenotype. In contradistinction to AITL, it does not have prominent HEV or extra-follicular expansion of FDC meshworks. The neoplastic cells may form intrafollicular aggregates, mimicking B-cell follicular lymphoma, but also can have interfollicular growth pattern or involve expanded mantle zones. Clinically, the follicular variant of PTCL-NOS is distinct from AITL as patients more often present with early stage disease with partial lymph node involvement and may lack the constitutional symptoms associated with AITL.

### ANAPLASTIC LARGE CELL LYMPHOMA (ALCL)

ALCL may be subdivided as ALCL-'anaplastic lymphoma kinase' (ALK)+ or ALCL-ALK-.

ALCL-ALK+ is one of the best-defined entities within the peripheral T-cell lymphomas, with characteristic "hallmark cells" bearing horseshoe-shaped nuclei and expressing ALK and CD30. It accounts for about 7% of all peripheral T-cell and NK-cell lymphomas and is most common in the first three decades of life. Patients often present with lymphadenopathy, but the involvement of extranodal sites (skin, bone, soft tissues, lung, liver) and B symptoms is common.

ALCL, ALK+ shows a wide morphologic spectrum, with 5 different patterns described, but all variants contain some hallmark cells. Hallmark cells have eccentric horseshoe- or kidney-shaped nuclei, and a prominent perinuclear eosinophilic Golgi region. The tumour cells grow in a cohesive pattern with predilection for sinus involvement. Smaller tumour cells predominate in the small cell variant, and in the lymphohistiocytic variant abundant histiocytes mask the presence of tumour cells, many of which are small.

By definition, all cases show ALK and CD30 positivity, with expression usually weaker in the smaller tumour cells. There is often loss of pan-T-cell markers, with 75% of cases lacking surface expression of CD3.

ALK expression is a result of a characteristic recurrent genetic alteration consisting of a rearrangement of *ALK* gene on chromosome 2p23 to one of the many partner genes, resulting in an expression of chimeric protein. The most common partner gene, occurring in 75% of cases, is Nucleophosmin (*NPM1*) on chromosome 5q35, resulting in t(2;5)(p23;q35). The cellular distribution of ALK in different translocation variants may vary depending on the partner gene.

ALCL-ALK- is included as a provisional category in the 2008 WHO classification. It is defined as a CD30 positive T-cell lymphoma that is morphologically indistinguishable from ALCL-ALK+ with a cohesive growth pattern and presence of hallmark cells, but lacking ALK protein expression.

Patients are usually adults between the ages of 40 and 65, in contrast to ALCL-ALK+, which is more common in children and young adults. ALCL-ALK- can involve both lymph nodes and extranodal tissues, although the latter is seen less commonly than in ALCL-ALK+. Most cases of ALCL-ALK- demonstrate effacement of lymph node architecture by sheets of cohesive neoplastic cells with typical "hallmark" features. In contrast to the ALCL-ALK+, the small cell morphologic variant is not recognized.

Unlike its ALK+ counterpart, ALCL-ALK- shows a greater preservation of surface T-cell marker expression, while the expression of cytotoxic markers and epithelial membrane antigen (EMA) is less likely. Gene expression signatures and recurrent chromosomal imbalances are different in ALCL-ALK- and ALCL-ALK+, confirming that they are distinct entities at a molecular and genetic level.

ALCL-ALK- is clinically distinct from both ALCL-ALK+ and PTCL-NOS, with significant differences in prognosis among these three different entities. The 5 year overall survival of ALCL-ALK- is reported as 49% which is not as good as that of ALCL-ALK+ (at 70%), but at the same time it is significantly better than that of PTCL-NOS (32%).

### ENTEROPATHY-ASSOCIATED T-CELL LYMPHOMA (EATL)

EATL is an aggressive neoplasm which thought to be derived from the intraepithelial T-cells of the intestine. Two morphologically, immunohistochemically and genetically distinct types of EATL are recognized in the 2008 WHO classification: Type I (representing the majority of EATL) and Type II (comprising 10-20% of cases).

Type I EATL is usually associated with overt or clinically silent gluten-sensitive enteropathy, and is more often seen in patients of Northern European extraction due to high prevalence of celiac disease in this population.

Most commonly, the lesions of EATL are found in the jejunum or ileum (90% of cases), with rare presentations in duodenum, colon, stomach, or areas outside of the gastrointestinal tract. The intestinal lesions are usually multifocal with mucosal ulceration. Clinical course of EATL is aggressive with most patients dying of disease or complications of disease within 1 year.

The cytological spectrum of EATL type I is broad, and some cases may contain anaplastic cells. There is a polymorphous inflammatory background, which may obscure the neoplastic component in some cases. The intestinal mucosa in regions adjacent to the tumour often shows features of celiac disease with blunting of the villi and increased numbers of intraepithelial lymphocytes (IEL), which may represent lesional precursor cells.

By immunohistochemistry, the neoplastic cells are often CD3+CD4-CD8-CD7+CD5-CD56-βF1+, and contain cytotoxic granule-associated proteins (TIA-1, granzyme B, perforin). CD30 is partially expressed in almost all cases. CD103, which is a mucosal homing receptor, can be expressed in EATL.

Type II EATL, also referred to as monomorphic CD56+ intestinal T-cell lymphoma, is defined as an intestinal tumour composed of small- to medium-sized monomorphic T-cells that express both CD8 and CD56. There is often a lateral spread of tumour within the mucosa, and absence of an inflammatory background. The majority of cases express the γδ TCR, however there are cases associated with the αβ TCR.

Type II EATL has a more world-wide distribution than Type I EATL and is often seen in Asians or Hispanic populations, in whom celiac disease is rare. In individuals of European descent EATL, II represents about 20% of intestinal T-cell lymphomas, with a history of celiac disease in at least a subset of cases. The clinical course is aggressive.

### HEPATOSPLENIC T-CELL LYMPHOMA (HSTL)

HSTL is an aggressive systemic neoplasm generally derived from γδ cytotoxic T-cells of the innate immune system, however, it may also be derived from αβ T-cells in rare cases. It is one of the rarest T-cell lymphomas, and typically affects adolescents and young adults (median age, 35 years) with a strong male predominance.

### EXTRANODAL NK/T-CELL LYMPHOMA NASAL TYPE

Extranodal NK/T-cell lymphoma, nasal type, is an aggressive disease, often with destructive midline lesions and necrosis. Most cases are of NK-cell derivation, but some cases are derived from cytotoxic T-cells. It is universally associated with Epstein-Barr Virus (EBV).

### CUTANEOUS T-CELL LYMPHOMA

The method of the present disclosure may also be used to treat cutaneous T-cell lymphoma.

Cutaneous T-cell lymphoma (CTCL) is characterised by migration of malignant T-cells to the skin, which causes various lesions to appear. These lesions change shape as the disease progresses, typically beginning as what appears to be a rash and eventually forming plaques and tumours before metastasizing to other parts of the body.

Cutaneous T-cell lymphomas include those mentioned in the following illustrative, non-exhaustive list; mycosis fungoides, pagetoid reticulosis, Sezary syndrome, granulomatous slack skin, lymphomatoid papulosis, pityriasis lichenoides chronica, CD30+ cutaneous T-cell lymphoma, secondary cutaneous CD30+ large cell lymphoma, non-mycosis fungoides CD30- cutaneous large T-cell lymphoma, pleomorphic T-cell lymphoma, Lennert lymphoma, subcutaneous T-cell lymphoma and angiocentric lymphoma.

The signs and symptoms of CTCL vary depending on the specific disease, of which the two most common types are mycosis fungoides and Sezary syndrome. Classic mycosis fungoides is divided into three stages:
Patch (atrophic or nonatrophic): Nonspecific dermatitis, patches on lower trunk and buttocks; minimal/absent pruritus;
Plaque: Intensely pruritic plaques, lymphadenopathy; and
Tumor: Prone to ulceration

Sézary syndrome is defined by erythroderma and leukemia. Signs and symptoms include edematous skin, lymphadenopathy, palmar and/or plantar hyperkeratosis, alopecia, nail dystrophy, ectropion and hepatosplenomegaly.

Of all primary cutaneous lymphomas, 65% are of the T-cell type. The most common immunophenotype is CD4 positive. There is no common pathophysiology for these diseases, as the term cutaneous T-cell lymphoma encompasses a wide variety of disorders.

The primary etiologic mechanisms for the development of cutaneous T-cell lymphoma (ie, mycosis fungoides) have not been elucidated. Mycosis fungoides may be preceded by a T-cell-mediated chronic inflammatory skin disease, which may occasionally progress to a fatal lymphoma.

### PRIMARY CUTANEOUS ALCL (C-ALCL)

C-ALCL is often indistinguishable from ALC-ALK- by morphology. It is defined as a cutaneous tumour of large cells with anaplastic, pleomorphic or immunoblastic morphology with more than 75% of cells expressing CD30. Together with lymphomatoid papulosis (LyP), C-ALCL belongs to the spectrum of primary cutaneous CD30-positive T-cell lymphoproliferative disorders, which as a group comprise the second most common group of cutaneous T-cell lymphoproliferations after mycosis fungoides.

The immunohistochemical staining profile is quite similar to ALCL-ALK-, with a greater proportion of cases staining positive for cytotoxic markers. At least 75% of the tumour cells should be positive for CD30. CD15 may also be expressed, and when lymph node involvement occurs, the differential with classical Hodgkin lymphoma can be difficult. Rare cases of ALCL-ALK+ may present with localized cutaneous lesions, and may resemble C-ALCL.

### T-CELL ACUTE LYMPHOBLASTIC LEUKAEMIA

T-cell acute lymphoblastic leukaemia (T-ALL) accounts for about 15% and 25% of ALL in paediatric and adult cohorts respectively. Patients usually have high white blood cell counts and may present with organomegaly, particularly mediastinal enlargement and CNS involvement.

The method of the present disclosure may be used to treat T-ALL which is associated with a malignant T cell which expresses a TCR comprising a TRBC.

### T-CELL PROLYMPHOCYTIC LEUKAEMIA

T-cell-prolymphocytic leukemia (T-PLL) is a mature T-cell leukaemia with aggressive behaviour and predilection for blood, bone marrow, lymph nodes, liver, spleen, and skin involvement. T-PLL primarily affects adults over the age of 30. Other names include T-cell chronic lymphocytic leukaemia, "knobby" type of T-cell leukaemia, and T-prolymphocytic leukaemia/T-cell lymphocytic leukaemia.

In the peripheral blood, T-PLL consists of medium-sized lymphocytes with single nucleoli and basophilic cytoplasm with occasional blebs or projections. The nuclei are usually round to oval in shape, with occasional patients having cells with a more irregular nuclear outline that is similar to the cerebriform nuclear shape seen in Sezary syndrome. A small cell variant comprises 20% of all T-PLL cases, and the Sezary cell-like (cerebriform) variant is seen in 5% of cases.

T-PLL has the immunophenotype of a mature (post-thymic) T-lymphocyte, and the neoplastic cells are typically positive for pan-T antigens CD2, CD3, and CD7 and negative for TdT and CD1a. The immunophenotype CD4+/CD8- is present in 60% of cases, the CD4+/CD8+ immunophenotype is present in 25%, and the CD4-/CD8+ immunophenotype is present in 15% of cases

### PHARMACEUTICAL COMPOSITION

The method of the present disclosure may comprise the step of administering the agent in the form of a pharmaceutical composition.

The agent may be administered with a pharmaceutically acceptable carrier, diluent, excipient or adjuvant. The choice of pharmaceutical carrier, excipient or diluent can be selected with regard to the intended route of administration and standard pharmaceutical practice. The pharmaceutical compositions may comprise as (or in addition to) the carrier, excipient or diluent, any suitable binder(s), lubricant(s), suspending agent(s), coating agent(s), solubilising agent(s), and other carrier agents.

### ADMINISTRATION

The administration of the agent can be accomplished using any of a variety of routes that make the active ingredient bioavailable. For example, the agent can be administered by oral and parenteral routes, intraperitoneally, intravenously, subcutaneously, transcutaneously, intramuscularly, via local delivery for example by catheter or stent.

Typically, a physician will determine the actual dosage which will be most suitable for an individual subject and it will vary with the age, weight and response of the particular patient. The dosage is such that it is sufficient to reduce or deplete the number of clonal T-cells expressing either TRBC1 or TRBC2.

### USE

The present invention also provides an agent for use in treating a T-cell lymphoma according to the method of the disclosure. The agent may be any agent as defined above.

The present invention also relates to the use of an agent as defined above in the manufacture of a medicament for the treatment of a T-cell lymphoma according to the disclosed method.

### KIT

The present invention further provides a kit comprising an agent as defined above for use in the treatment of a T-cell lymphoma according to the method of the disclosure.

The kit may also comprise a reagent(s) suitable for determining the TRBC of a malignant T-cell. For example the kit may comprise PCR primers or an antibody (antibodies) which are specific for either TRBC1 or TRBC2.

### METHOD FOR DETERMINING T-CELL LYMPHOMA AND/OR LEUKAEMIA

The present invention further relates to a method for determining the presence of a T-cell lymphoma or leukaemia in a subject which comprises the step of determining the proportion of T-cells in a sample from a subject which are either TRBC1 or TRBC2 positive.

T-cell lymphomas involve the clonal expansion of individual malignant T-cells. As such the presence of a T-cell lymphoma in a subject may be identified by determining the proportion of either TRBC1 or TRBC2 T-cells in a sample derived from a patient.

The sample may be a peripheral blood sample, a lymph sample or a sample taken directly from a tumour e.g. a biopsy sample.

The proportion of total T-cells which are TRBC1 or TRBC2 positive which indicates the presence of a T-cell lymphoma or leukaemia may be, for example 80, 85, 90, 95, 98 or 99% of a total population of cells.

The method may involve determining infiltration by a distinct population of T-cells in a biopsy or a sample. Herein, the presence of a T-cell lymphoma or leukaemia is indicated where 80, 85, 90, 95, 98 or 99% of a total population of T cells in the sample are either TRBC1 or TRBC2.

The total T-cells in a sample may identified by determining the number of cells in the sample which express CD3, CD4, CD8 and/or CD45. A combination of these markers may also be used.

The proportion of total T cells in a sample which express either TRBC1 or TRBC2 may be determined using methods which are known in the art, for example flow cytometry, immunohistochemistry or fluorescent microscopy.

The invention will now be further described by way of Examples, which are meant to serve to assist one of ordinary skill in the art in carrying out the invention and are not intended in any way to limit the scope of the invention.

### EXAMPLES

### Example 1 - Discrimination of TRBC1 and TRBC2-expressing cells

The JOVI-1 antibody has been previously disclosed by Viney et al. (Hybridoma; 1992; 11(6); 701- 713) and is available commercially (Abcam, ab5465). The present inventors determined that JOVI-1 is able to discriminate cells based on specific expression of TRBC1 or TRBC2.

The inventors generated two plasmid vectors supplying the complete variable and constant regions of the TCR, differing only in expression of either TRBC1 or TRBC2. These plasmids were used to generate retroviral supernatant by transient transfection of 293T-cells. This supernatant was used to stably transduce Jurkats TCR-knockout T-cells (a T-ALL cell line with a mutation at the TCR beta chain locus precluding expression of this chain, and thereby the entire surface TCR/CD3 complex). This resulted in the production of cell lines which were identical other than expression of either TRBC1 or TRBC2. Staining of these cell lines revealed full expression of the surface TCR/CD3 complex, and that only cells expressing TRBC1 stained with the JOVI-1 antibody (Figure 4).

### Example 2 - Normal donor CD4+ and CD8+ T-cells contain separate TRBC1-positive and TRBC1-negative populations

The inventors tested the JOVI-1 antibody on primary human T-cells of normal donors. These analyses revealed that all donors had a proportion of both CD4+ and CD8+ T cells which expressed TRBC1 and a proportion of each which did not. Approximately 20-50% of normal CD4+ and CD8+ T-cells are TRBC1 +ve (Figures 6 and 7).

### Example 3 - Clonal T-cell lines expressing TCR are TRBC1 positive or negative

Cell lines are derived from an original clonal tumour population in a patient. Staining of T-cell lines expressing TCR reveals that T-cells express either TRBC1 or TRBC2, confirming this as a marker of clonality. Of three T-cell lines tested, Jurkats cells (known to be TRBC1+) and not HPB-ALL or HD-Mar-2 (known to be TRBC2+) cells stain with JOVI-1, supporting exclusive expression of either TRBC1 or 2 (Figure 8).

### Example 4 - Primary clonal T-cell in patients with T-prolymphocyctic leukaemia are TRBC1 positive or negative

Clonal T-cells extracted from peripheral blood of patients with T-prolymphocyctic leukaemia (T-PLL) are either uniformly TRBC1 positive or TRBC1 negative.

### Example 5-The effect of mutation of the residues that are unique to TCBC1

Plasmid vectors, coding for TCRs which are identical, except for hybrid TRBC1/2 mutations in the TCR β chain constant region, were generated. Analysis showed that JOVI-1 recognizes differences in residues at positions 3 and 4 of TCR β constant chain indicating that these residues are accessible to antibody recognition and are likely the best targets to generate agents discriminating TRBC1 from TRBC2 or TRBC2 from TRBC1 (Figure 5).

### Example 6 - Specific Lysis of TRBC1 but not TRBC2 TCR expressing T-cells.

Wild-type Jurkat T-cells (CD34-, TRBC1+) were mixed with TCRαβ knock-out Jurkat T-cells transduced with TRBC2 co-expressed with the CD34 marker gene (CD34+TRBC2+). These cells were incubated with JOVI-1 alone or incubated with JOVI-1 and complement for 1 hour. Cells were washed and stained for CD34, Annexin V and 7-AAD. Cells were analysed by flow-cytometry.

CD34 expression in the live population as defined by Annexin-V negative and 71AAD dim population is shown in Figure 9. Selective killing of TRBC1 T-cells (CD34-) was observed (Figure 9).

Wild-type Jurkat T-cells are naturally TRBC1+ and do not express the truncated CD34 marker gene. As described above the inventors derived a TRBC2+ Jurkat line by transducing TCRαβ knock-out Jurkat T-cells with a retroviral vector which codes for a TRBC2 TCR as well as the truncated CD34 marker gene. These T-cells were then mixed together. Next, the inventors incubated the T cells with either JOVI-1 alone or with JOVI-1 and complement for 1 hour. Conveniently, the inventors could discriminate TRBC1 and 2 populations by staining for the CD34 marker gene and thus avoided failing to detect TRBC1 TCRs due to TCR internalization after prolonged exposure to anti-TCR mAb. Cells were washed and stained for CD34, Annexin V and 7-AAD. The cells were analysed by flow-cytometry. By gating on live cells (i.e. cells which were Annexin V negative and 7-AAD dim), the inventors could determine that TRBC1 T-cells were selectively killed by JOVI-1 in the presence of complement (Figure 9).

### Example 7 - Polyclonal Epstein Barr Virus (EBV) specific T-cells can be split into two approximately equal TRBC1/2 populations.

Peripheral blood T-cells were drawn from a normal blood donor. Mononuclear cells were isolated and most of the cells were cryopreserved. A small number of cells were infected with a laboratory strain of EBV (B95-8). Over some weeks, an immortalized EBV infected cell line, known as a lymphoblastoid cell line (LCL) emerged. Such a cell line is known to present a large collection of different EBV antigens. The previously cryopreserved mononuclear cells were thawed and repeatedly stimulated with this LCL line weekly for 4 weeks in the presence of IL2. This process selectively expands EBV specific T-cells from the peripheral blood mononuclear population. It is also known that such a process results in a polyclonal line where >90% of the T-cells are EBV specific and represents the donor's EBV immune system. The specificity of this line is checked by showing a high degree of killing of autologous LCLs but not allogeneic LCLs or K562 cells (Figure 10a). This cell line was then stained with JOVI-1 and shown to contain an approximately equal mixture of TRBC1 and TRBC2 T-cells (Figure 10b).

Thus, if a therapeutic agent was administered which depleted either the TRBC1 or TRBC2 compartment, an adequate EBV immunity would remain. Since EBV immunity is regarded as a model system for an immune response it is reasonable to postulate that immunity to other pathogens would be equally conserved.

### Example 8 - JOV1 staining of a circulating peripheral T-cell lymphoma.

The hypothesis was that T-cell lymphomas, being clonal, would express either TRB1 or TRBC2 T-cell receptors, while normal T-cells being polyclonal would comprise of a population of T-cells which are a mixture of those that have TRBC1 or TRBC2. To demonstrate this, a blood sample of a T-cell lymphoma from a patient whose lymphoma was circulating in peripheral blood was obtained. Peripheral blood mononuclear cells were isolated and stained with a panel of antibodies which included CD5 and JOVI1. The total T-cell population (which contains both lymphoma and normal T-cells) was first identified. This population was comprised of T-cells with normal (bright) CD5 expression and T-cells with intermediate/dim CD5 expression. The former represent normal T-cells, while the latter represent the lymphoma. JOVI-1 binding was investigated next and the results are shown in Figure 12.
The CD5 intermediate and dim populations (the tumour) were all TRBC2 positive.

### Example 9 - Elucidation of the VH/VL sequences of JOVI-1

Using 5' RACE with primers which anneal to the constant regions of mouse IgG CH1 and the constant region of mouse kappa, we isolated a single functional VH sequence and a single functional VL sequence from the hybridoma JOVI-1. Sequences of the VH and VL are SEQ ID 1 and 2 respectively (see above). An annotated sequence of VH and VL is shown in Figure 11.

These VH and VL sequences were cloned back in frame with mouse IgG heavy chain and kappa light chain respectively. In addition, the VH and VL were fused to form a single-chain variable fragment (scFv), this was fused to the hinge-CH2-CH3 region of mouse IgG2a to create a scFv-Fv. The amino acid sequence of the scFv is given in the Detailed Description as SEQ ID No. 3. Recombinant antibody and recombinant scFv-Fc were generated by transfection into 293T cells. Along with JOVI-1 from the hybridoma, the following cells were stained: Jurkats with TCR knocked out; wild-type Jurkats; Jurkat TCR knock out transduced with TRBC1 co-expressed with eBFP2 and Jurkat TCR knock-out transduced with TRBC2 co-expressed with eBFP2. Both recombinant antibody and scFv-Fc derived from JOVI bound TRBC2 confirming that we had identified the correct VH/VL, and that JOVI-1 VH/VL can fold as a scFv. This binding data is shown in Figure 13.

### Example 10 - Function of JOVI-1 based CAR

The JOVI-1 scFv was cloned into CAR formats. To elucidate which spacer length would result in the optimal JOVI-1 based CAR, 3^{rd} generation CARs were generated with either a human Fc spacer, a human CD8 stalk spacer or with a spacer derived from an IgG1 hinge (Figure 4). Primary human T-cells from normal donors were transduced with these CARs and killing of Jurkats and Jurkats with TCR knocked-out were compared. CARs with JOVI-1 scFvs which had either an IgG1 hinge spacer or a CD8 stalk spacer killed Jurkats, but not Jurkats with TCR knockout (Figure 5), demonstrating the expected specificity. Since the normal donor T-cells transduced with the CARs should have a mixture of TRB1/2 T-cells, it was expected that the cultures would "self-purge". Indeed, this was observed. JOVI-1 staining of the CAR T-cell cultures which become 100% TRBC2 negative in shown in Figure 6.

### MATERIALS & METHODS

### DEMONSTRATION OF SPECIFICITY OF JOVI-1

A tri-cistronic retroviral cassette was generated which coded for a well characterized human TCR as well as a convenient marker gene. The coding sequences for the TCRα and β chains were generated using de-novo gene synthesis from overlapping oligonucleotides. These chains were connected in frame to a foot-and-mouth disease 2A peptide to allow co-expression. The truncated CD34 marker gene was cloned from cDNA by PCR and co-expressed with the TCR chains using an internal ribosome entry sequence (IRES). This cassette was introduced into a retroviral vector. Variants of this construct were generated by splice by over-lap PCR with primers which introduced the desired mutations. The veracity of the constructs was confirmed by Sanger sequencing. The Jurkat 76 line is a well characterized derivate of the Jurkat T-cell line which has both TCR α and β chains knocked out. This Jurkat line was transduced with the above retroviral vectors using standard techniques.

### STAINING AND ANALYSIS OF JURKATS, PERIPHERAL BLOOD T-CELLS AND CELL LINES

Jurkats were obtained from ECACC and engineered as detailed above. Other T-cell lines were also obtained from ECACC. Peripheral blood was drawn by venopunture from normal donors. Blood was ficolled to isolate mononuclear cells. Cells were stained with JOVI-1 as well as commercially available monoclonal antibodes which recognize all TCRs and CD3. In the case of engineered T-cells, cells were stained with antibodies which recognize CD34. In case of peripheral blood mononuclear cells, cells were stained with antibodies which recognize CD4 and CD8. The antibodies were purchased conjugated with suitable fluorophores so that independent fluorescent signals could be obtained while analyzing the cells with a flow-cytometer.

### DEMONSTRATION OF SPECIFIC LYSIS OF TRBC1 T-CELLS

Wild-type Jurkat T-cells (TRBC1 - TCR), and Jurkat T-cells TCR KO with TRBC2 TCR introduced were mixed together at a ratio of 1:1. This mixture of Jurkats was then incubated with JOVI-1 monoclonal antibody at 1ug/ml in the absence or presence of complement. Four hours later, cells were stained with Annexin-V and 7AAD and CD34. Conveniently, the marker gene CD34 can distinguish between wild-type (TRBC1) and transgenic (TRBC2) Jurkats. Cell populations were analysed by flow-cytometry. Live cells were selectively studied by gating on flow cytometric events which are negative for Annexin-V and dim for 7AAD. In this way, the survival of transgenic (TRBC2) vs wild-type (TRBC1) T-cells was studied.

### Example 11 - Investigating the clonality of T-cell lymphoproliferative disorders

Four patients: three with T-cell large granular lymphocyte lymphoproliferative disorder (T-LGL); and one with peripheral T-cell lymphoma (PCTL) were tested to confirm that malignant cells were uniformly either TRBC1 positive or negative.

Whole blood or bone marrow was collected from patients with T-lymphoproliferative orders. Peripheral blood mononuclear cells (PBMCs) were obtained by Ficoll gradient centrifugation. Freshly obtained PBMCs were pelleted and stained for 20 minutes with appropriate pre-conjugated antibodies. The cells were then washed and resuspended in phosphate buffered saline for immediate flow cytometric analysis on BD LSR Fortessa II. Live lymphocytes were identified by FSc/SSc properties and failure to uptake a dead cell discriminating dye. T-cells were identified by staining with anti-TCR alpha/beta antibody. Tumour and normal T-cell populations were identified using appropriate cell surface stains for each sample, based upon immunophenotype previously identified by clinical laboratory analysis.

The results are shown in Figures 18 to 21.
In patient A (T-LGL, Figure 18), normal T-cells were CD7bright and contained mixed CD4/CD8 cells, and a mixed population of TRBC1 or TRBC1- cells. In contrast, malignant cells were CD7- or CD7dim, were uniformly CD8+CD4-, and were uniformly TRBC1-.
In patient B (T-LGL, Figure 19), malignant cells were identified by CD4-, CD8+, CD7+ CD57+ and were clonally TRBC1- (highlighted panel). Normal CD4+CD8- and CD4-CD8+ T-cells contained TRBC1 + and TRBC1- populations.
In patient C (T-LGL, Figure 20), normal CD4+ and CD8+ T- cells populations were 30-40% TRBC1+. Malignant cells were identified by CD4-, CD8+, CD7+ CD57+ and were clonally TRBC1+ (highlighted panel, note 84% of cells are TRBC1 - remaining 16% likely to be contaminating 'normal' T-cells). Normal CD4+CD8- and CD4-CD8+ T-cells contained TRBC1+ and TRBC1- populations.
In patient D (PTCL-NOS, Figure 21), malignant cells in the bone marrow, identified on the basis of FSChigh CD5dim CD4dim, were uniformly TRBC1+, whereas CD4+CD8- and CD4-CD8+ T-cells contained both TRBC1+ and TRBC1- populations.

### Example 12 - Generation of monoclonal human antibodies which distinguish between the two isoforms of the T-cell receptor β chain constant domain using phage display

In order to generate antibodies which distinguished between TRBC2 and TRBC1 peptide fragments covering the region of difference between the two TRBC isoforms were synthesised. Of the four amino acid differences between TRBC2 and TRBC1, two are found at the beginning of the constant domains. Peptides (see below) representing these regions were synthesised and used for antibody generation.
**TRBC2** *VL*EDL**KN**VFPPEVAV (SEQ ID No. 36)
**TRBC1** *VL*EDL**NK**VFPPEVAV (SEQ ID No. 37)

These peptides were prepared in biotinylated, non-biotinylated and cysteine modified forms (by addition of a C terminal cysteine). The cysteine modified forms of TRBC1 and TRBC2 were subsequently conjugated to modified bovine serum albumin (Imm-Link BSA, Innova 462-001) or ovalbumin (Imm-Link Ovalbumin, Innova 461-001) according to manufacturers recommended conditions.

### Results

### Antibody phage display selections

A human phage display library was constructed and phage selections carried out as described in as described in (Schofield et al., 2007 Genome Biol 8, R254). In order to identify TRBC1 and TRBC2 specific antibodies from the antibody library, multiple rounds of phage display selections were carried out. Two phage selection strategies were used in parallel to maximise the chance of generating a large panel of specific binders. These strategies are known as solid phase and solution phase selections (Figure 21). In solid phase selections, phage antibodies are allowed to bind to the target antigen immobilised on a solid surface (Schofield et al., 2007, as above). In solution phase selections, phage antibodies binds to the biotinylated antigen in solution and the phage antibody-antigen complex is then captured by streptavidin or neutravidin coated paramagnetic beads. Within the solid phase selection strategy, two different immobilisation or antigen presentation approaches were employed. Using the first approach, TRBC peptides conjugated to bovine serum albumin (BSA) or ovalbumin (OA) were immobilised on Maxisorp™ immunotubes via direct adsorption. Using the second approach, biotinylated TRBC peptides were immobilised indirectly on Maxisorp™ immunotubes tubes that were pre-coated with streptavidin or neutravidin.

In order to select antibodies that are specific to the desired peptide, all selections were carried out in presence of an excess of the opposing peptide. For example, all TRBC1 selections were carried out in the presence of a 10-fold molar excess of non-biotinylated TRBC2. This method is known as 'deselection' and it was expected to deplete antibodies that recognise shared epitopes on both TRBC peptides as these preferentially bind to the excess TRBC2 in solution. In order to avoid enriching for antibody clones that bind to the carrier protein (BSA or OA) or the immobilisation partner (streptavidin or neutravidin from Thermo fisher scientific) two strategies were employed in combination.

The first strategy was to switch the conjugation or immobilisation partner between rounds of selection. For directly immobilised peptides the first round of selections were carried out on BSA-peptide and for round-2 OA-peptide conjugate was used. Similarly, biotinylated TRBC peptides were immobilised on streptavidin for the round-1 and neutravidin was used for immobilisation in round-2.

The second strategy was to deplete the phage library of any binders to the conjugation/immobilisation partner by performing a 'deselection' in round-1. For directly immobilised peptides, 'deselection was performed by carrying out the phage-peptide binding step in the presence of 10-fold molar excess of free BSA in solution. In the case of biotinylated peptides immobilised on streptavidin, the phage library was pre-incubated with streptavidin coated paramagnetic beads. The beads were removed prior to the addition of the phage to antigen tubes thereby limiting the entry streptavidin binders into the selection. The different selection conditions used are summarised in figure 21. See Table 3 for detailed information on selection conditions.

Polyclonal phage prepared from round-2 selection output was tested in ELISA using various presentations of the peptides or the support proteins. This included TRBC peptides directly immobilised as either BSA or OA conjugates or biotinylated peptides indirectly immobilised on streptavidin or neutravidin. Control proteins included were streptavidin, neutravidin, BSA and an irrelevant antigen. Phage binding was detected using a mouse anti-M13 antibody (GE healthcare) followed by an anti-mouse Fc antibody labelled with Europium (Perkin Elmers) using time resolved fluorescence (Figure 22). This result demonstrated the preferential binding of polyclonal phage populations to the respective TRBC peptide (as compared to the opposing TRBC peptide). For example, polyclonal phage prepared from TRBC1 selections showed significantly higher binding signal to TRBC1 than TRBC2 and vice versa. There was limited or no binding to the immobilisation or conjugation partners and the irrelevant antigen.

### Single chain antibody (scFv) sub-cloning and monoclonal screening

The scFv populations from round-2 and round-3 selection outputs were sub-cloned into the pSANG10-3F expression vector and transformed into E.coli BL21 (DE3) cells.. 1128 individual transformants (564 clones/TRBC peptide) were picked into 12 x 96 well culture plates (94 clones/plate) and antibody expression was induced using autoinduction media.. Recombinant monoclonal antibodies secreted into culture supernatant after overnight induction were tested for binding to biotinylated TRBC1 and TRBC2 immobilised on neutravidin coated Nunc Maxisorp™ 96 well plates. Out of the 564 clones screened from the TRBC1 selections, 255 clones were found to be specific for TRBC1 (>10000 TRF units for TRBC1 and <1000 TRF units for TRBC2). 138 TRBC2 specific binders (>10000 TRF units for TRBC2 and <2000 TRF units for TRBC1) were identified from the 564 clones screened from the TRBC2 selections. Figure 24 shows a representative binding profile from a single 96 well plate arising from selection on either TRBC1 (fig 24A) or TRBC2 (figure 24B). The details of specific binders generated using different selection conditions is summarised in Table 5.

142 and 138 specific binders were picked from the TRBC1 and TRBC2 selections respectively for sequence analysis and further characterisation. Sequences of cherry-picked clones were generated by Sanger sequencing using BigDye® terminator v3.1 cycle sequencing kit (Life technologies). DNA sequences were analysed to determine protein sequence and the CDRs of the VH and VL domains were identified. Analysis of the VH and VL CDR3 regions identified 74 unique TRBC1 and 42 unique TRBC2 clones (where unique is defined as any combination of VH CDR3 and VL CDR3 sequence). The TRBC1-specific clones and their VH CDR3 and VL CDR3 sequences are summarised in Table 1 above. The TRBC2-specific clones and their VH CDR3 and VL CDR3 sequences are summarised in Table 2 above.

**Table 3A. Details of solid phase TRBC selections**

| **Solid phase selections** | | | | | |
|---|---|---|---|---|---|
| **Selection antigen** | **No. of rounds** | **Antigen concentration** | **Immobilisation** | **Round 1 deselection antigens** | **Round 2 deselection antigens** |
| BSA TRBC1 (Round 1), OA TRBC1 (Round 2) | 2 | 10 µg/ml | Direct | BSA (100 µg/ml), TRBC2 (30 µM) | TRBC2 (30 µM) |
| BSA TRBC2 (Round 1), OA TRBC2 (Round 2) | 2 | 10 µg/ml | Direct | BSA (100 µg/ml), TRBC1 (30 µM) | TRBC1 (30 µM) |
| Bio-TRBC1 (Round 1&2) | 2 | 3 µg/ml | Streptavidin beads (Round 1), Neutravidin (Round 2) | Streptavidin beads, TRBC2 (30µg/ml) | Neutravidin beads, TRBC2 (30µg/ml) |
| Bio-TRBC2 (Round 1&2) | 2 | 3 µg/ml | Streptavidin beads (Round 1), Neutravidin (Round 2) | Streptavidin beads, TRBC1 (30µg/ml) | Neutravidin beads, TRBC1 (30µg/ml) |

**Table 3B. Details of solution phase TRBC selections**

| **Solution phase selections** | | | | | |
|---|---|---|---|---|---|
| **Selection antigen** | **No. of rounds** | **Antigen concentration** | **Round 1 deselection antigens** | **Round 2 deselection antigens** | **Round 3 deselection antigens** |
| Bio-TRBC1 (Round 1,2&3) | 3 | 500 nM | Streptavidin beads, TRBC2 (5 µM) | Streptavidin beads, TRBC2 (5 µM) | Neutravidin beads, TRBC2 (5 µM) |
| Bio-TRBC2 (Round 1,2&3) | 3 | 500 nM | Streptavidin beads, TRBC1 (5 µM) | Streptavidin beads, TRBC1 (5 µM) | Neutravidin beads, TRBC1 (5 µM) |

**Table 4: Selection output numbers**

| **Selection type** | **No. of rounds** | **Antigen** | **No. of plaque forming units (Round 1)** | **No. of plaque forming units (Round 2)** | **No. of plaque forming units (Round 3)** |
|---|---|---|---|---|---|
| Solid phase | 2 | BSA/OA TRBC1 | 1.0 x 10⁴ | 6.0 x 10⁵ | N.D |
| Solid phase | 2 | BSA/OA TRBC2 | 1.5 x 10³ | 2.2 x 10⁶ | N.D |
| Solid phase | 2 | Bio-TRBC1 | 5.0 x 10³ | 2.0 x 10⁵ | N.D |
| Solid phase | 2 | Bio-TRBC2 | 3.0 x 10³ | 1.0 x 10⁴ | N.D |
| Solution phase | 3 | Bio-TRBC1 | 1.5 x 10⁶ | >10⁸ | >10⁸ |
| Solution phase | 3 | Bio-TRBC2 | 2.7 x 10⁵ | >10⁸ | >10⁸ |

**Table 5: Details of monoclonal screening**

| **Selection number** | **Selection type** | **Selection antigen** | **Selection output** | **No. of clones screened** | **No. of specific binders** |
|---|---|---|---|---|---|
| 262 | Solid-phase, indirect immobilisation | TRBC1 | Round-2 | 186 | 93 |
| 263 | Solid-phase, indirect immobilisation | TRBC2 | Round-2 | 186 | 68 |
| 264 | Solution-phase | TRBC1 | Round-2 | 186 | 83 |
| 265 | Solution-phase | TRBC2 | Round-2 | 186 | 29 |
| 266 | Solution-phase | TRBC1 | Round-3 | 94 | 47 |
| 267 | Solution-phase | TRBC2 | Round-3 | 94 | 33 |
| 268 | Solid-phase, direct immobilisation (BSA/OA) | TRBC1 | Round-2 | 94 | 32 |
| 269 | Solid-phase, direct immobilisation (BSA/OA) | TRBC2 | Round-2 | 94 | 9 |

### Example 13 - TRBC polyclonal antibody production via peptide immunisation of rabbits

In order to generate antibodies which distinguish between TRBC2 and TRBC1, 2 peptides that cover the principle area of differentiation between the two TRBC isoforms were synthesized and used for the immunization of rabbits. The following peptide sequences were used:
TRBC1: VLEDLNKVFPPEVAVC (SEQ ID No. 38)
TRBC2: VLEDLKNVFPPEVAVC (SEQ ID No. 39).

15 mg of TRBC1 and TRBC2 peptides were synthesized. Keyhole Lympet Hemocyanin was conjugated to TRBC1 and TRBC2 peptides via C terminal cysteines present on the peptides. For each peptide 2 New England rabbits were immunized a total of three times with KLH conjugated TRBC1 or TRBC2 peptide. After the third immunization rabbits were sacrificed and bled, and the serum collected for purification. The crude serum obtained from the rabbits were passed through a crosslinked beaded agarose resin column coupled with the peptide used for immunization to collect antibodies specific for the common segments and the TRBC isoform specific epitope of the peptide. The initially purified supernatant was then purified further through a column with the alternative peptide immobilized, to remove the antibodies specific to common segments of the peptide.

### ELISA Setup

Coating Antigen(s):
A : peptide TRBC1
B : peptide TRBC2
Coating Concentration: 4ug/ml, 100 µl/well
Coating Buffer: Phosphate Buffered Saline, pH7.4
Secondary Antibody: Anti-RABBIT IgG (H&L) (GOAT) Antibody Peroxidase Conjugated

The results are shown in Figures 25 and 26. It is possible to make polyclonal serum comprising TRBC1 or TRBC2-specific antibodies by this method.

## Claims

1. A chimeric antigen receptor (CAR) which comprises an antigen-binding domain which selectively binds TCR beta constant region 1 (TRBC1) or TRBC2.

2. A CAR according to claim 1, which selectively binds TRBC1.

3. A CAR according to claim 2, wherein the antigen-binding domain has a variable heavy chain (VH) and a variable light chain (VL) which comprise the following complementarity determining regions (CDRs):
VH CDR1: SEQ ID No. 7;
VH CDR2: SEQ ID No. 8;
VH CDR3: SEQ ID No. 9;
VL CDR1: SEQ ID No. 10;
VL CDR2: SEQ ID No. 11; and
VL CDR3: SEQ ID No. 12.

4. A CAR according to claim 2, wherein the antigen-binding domain comprises a variable heavy chain (VH) having the sequence shown as SEQ ID No. 1 and a variable light chain (VL) having the sequence shown as SEQ ID No. 2.

5. A CAR according to claim 2, which comprises an amino acid sequence selected from SEQ ID No. 4, 5 and 6.

6. A CAR according to claim 1 which selectively binds TRBC2.

7. A nucleic acid sequence encoding a CAR according to any preceding claim.

8. A vector which comprises a nucleic acid sequence according to claim 7.

9. A cell which comprises a CAR according to any of claims 1 to 6.

10. A T cell according to claim 9.

11. A method for making a cell according to claim 9 or 10 which comprises the step of transducing or transfecting a cell ex vivo with a nucleic acid sequence according to claim 7 or a vector according to claim 8.

12. A cell according to claim 9 or 10 for use in a method for treating a T-cell lymphoma or leukaemia in a subject which comprises the step of
administrating the cell comprising the TCRB1 or TCRB2 selective CAR to the subject, to cause selective depletion of the malignant T-cells, together with normal T-cells expressing the same TRBC as the malignant T-cells, but not to cause depletion of normal T-cells expressing the TRBC not expressed by the malignant T-cells.

13. A cell for use according to claim 12, wherein the method also comprises the step of investigating the TCR beta constant region (TCRB) of a malignant T cell from the subject to determine whether it expresses TRBC1 or TRBC2.

14. A cell for use according to claim 12 or 13 wherein the T-cell lymphoma or leukaemia is selected from peripheral T-cell lymphoma, not otherwise specified (PTCL-NOS); angio-immunoblastic T-cell lymphoma (AITL), anaplastic large cell lymphoma (ALCL), enteropathy-associated T-cell lymphoma (EATL), hepatosplenic T-cell lymphoma (HSTL), extranodal NK/T-cell lymphoma nasal type, cutaneous T-cell lymphoma, primary cutaneous ALCL, T cell prolymphocytic leukaemia and T-cell acute lymphoblastic leukaemia.

15. A method for diagnosing a T-cell lymphoma or leukaemia in a subject which comprises the step of determining the percentage of total T-cells in a sample isolated from the subject which are TRBC1 or TRBC2 positive.

16. A method for selecting a suitable therapy to treat a subject suffering from T-cell lymphoma or leukaemia which comprises:
i) determining whether a malignant T cell from the subject expresses TRBC1 or TRBC2; and
ii) selecting a cell for use according to claim 12 based on the TRBC1 or TRBC2 expression of said malignant T cell.

17. A method for selecting a subject suffering from T-cell lymphoma or leukaemia to receive a therapy based on a cell for use according to claim 12, which comprises:
i) determining whether a malignant T cell from the subject expresses TRBC1 or TRBC2; and
ii) selecting said subject to receive a therapy based on a cell for use according to claim 12 based on the TRBC1 or TRBC2 expression of said malignant T cell.

## Patentansprüche

1. Chimärer Antigen-Rezeptor (CAR), der eine antigenbindende Domäne umfasst, die TCR-beta-konstante-Region 1 (TRBC1) oder TRBC2 selektiv bindet.

2. CAR nach Anspruch 1, der TRBC1 selektiv bindet.

3. CAR nach Anspruch 2, wobei die antigenbindende Domäne eine variable schwere Kette (VH) und eine variable leichte Kette (VL) aufweist, die die folgenden Komplementarität bestimmenden Regionen (CDR) umfassen:
VH-CDR1: SEQ ID Nr. 7;
VH-CDR2: SEQ ID Nr. 8;
VH-CDR3: SEQ ID Nr. 9;
VL-CDR1: SEQ ID Nr. 10;
VL-CDR2: SEQ ID Nr. 11; und
VL-CDR3: SEQ ID Nr. 12.

4. CAR nach Anspruch 2, wobei die antigen¬bindende Domäne eine variable schwere Kette (VH) mit der Sequenz gemäß SEQ ID Nr. 1 und eine variable leichte Kette (VL) mit der Sequenz gemäß SEQ ID Nr. 2 umfasst.

5. CAR nach Anspruch 2, der eine aus SEQ ID Nr. 4, 5 und 6 ausgewählte Aminosäuresequenz umfasst.

6. CAR nach Anspruch 1, der TRBC2 selektiv bindet.

7. Nukleinsäuresequenz, codierend einen CAR nach einem vorhergehenden Anspruch.

8. Vektor, der eine Nukleinsäuresequenz nach Anspruch 7 umfasst.

9. Zelle, die einen CAR nach einem der Ansprüche 1 bis 6 umfasst.

10. T-Zelle nach Anspruch 9.

11. Verfahren zur Erzeugung einer Zelle nach Anspruch 9 oder 10, das den Schritt der Transduktion oder Transfektion einer Zelle ex vivo mit einer Nukleinsäuresequenz nach Anspruch 7 oder einem Vektor nach Anspruch 8 umfasst.

12. Zelle nach Anspruch 9 oder 10 zur Verwendung bei einem Verfahren zur Behandlung eines T-Zell-Lymphoms bzw. einer T-Zell-Leukämie bei einem Individuum, das den Schritt des
Verabreichens der den TCRB1- oder TCRB2-selektiven CAR umfassenden Zelle an das Individuum, so dass eine selektive Abreicherung der bösartigen T-Zellen zusammen mit normalen T-Zellen, die die gleiche TRBC wie die bösartigen T-Zellen exprimieren, aber keine Abreicherung normaler T-Zellen, die die nicht von den bösartigen T-Zellen exprimierte TRBC exprimieren, herbeigeführt wird, umfasst.

13. Zelle zur Verwendung nach Anspruch 12, wobei das Verfahren auch den Schritt des Untersuchens der TCR-beta-konstante-Region (TCRB) einer bösartigen T-Zelle vom Individuum zur Bestimmung, ob sie TRBC1 oder TRBC2 exprimiert, umfasst.

14. Zelle zur Verwendung nach Anspruch 12 oder 13, wobei das T-Zell-Lymphom bzw. die T-Zell-Leukämie ausgewählt ist aus peripherem T-Zell-Lymphom ohne weitere Spezifizierung (PTCL-NOS); angioimmunoblastischem T-Zell-Lymphom (AITL), anaplastischgroßzelligem Lymphom (ALCL), mit Enteropathie assoziiertem T-Zell-Lymphom (EATL), hepatosplenischem T-Zell-Lymphom (HSTL), extranodalem NK/T-Zell-Lymphom, nasaler Typ, kutanem T-Zell-Lymphom, primär kutanem ALCL, T-Zell-Prolymphozytenleukämie und akuter lymphatischer T-Zell-Leukämie.

15. Verfahren zur Diagnose eines T-Zell-Lymphoms bzw. einer T-Zell-Leukämie bei einem Individuum, das den Schritt des Bestimmens des prozentualen Anteils TRBC1- oder TRBC2-positiver T-Zellen an den gesamten T-Zellen in einer vom Individuum isolierten Probe umfasst.

16. Verfahren zum Auswählen einer geeigneten Therapie zur Behandlung eines an T-Zell-Lymphom oder -Leukämie leidenden Individuums, das Folgendes umfasst:
i) Bestimmen, ob eine bösartige T-Zelle vom Individuum TRBC1 oder TRBC2 exprimiert; und
ii) Auswählen einer Zelle zur Verwendung nach Anspruch 12 bezogen auf die TRBC1- oder TRBC2-Expression der bösartigen T-Zelle.

17. Verfahren zur Auswahl eines an T-Zell-Lymphom oder -Leukämie leidenden Individuums für das Erhalten einer Therapie auf der Grundlage einer Zelle zur Verwendung nach Anspruch 12, das Folgendes umfasst:
i) Bestimmen, ob eine bösartige T-Zelle vom Individuum TRBC1 oder TRBC2 exprimiert; und
ii) Auswählen des Individuums für das Erhalten einer Therapie auf der Grundlage einer Zelle zur Verwendung nach Anspruch 12 bezogen auf die TRBC1- oder TRBC2-Expression der bösartigen T-Zelle.

## Revendications

1. Récepteur d'antigène chimère (CAR), comprenant un domaine de fixation de l'antigène qui fixe sélectivement la région constante bêta 1 du TCR (TRBC1) ou TRBC2.

2. CAR selon la revendication 1, qui fixe sélectivement TRBC1.

3. CAR selon la revendication 2, dans lequel le domaine de fixation de l'antigène possède une chaîne lourde variable (VH) et une chaîne légère variable (VL) qui comprennent les régions déterminant la complémentarité (CDR) suivantes :
VH CDR1 : SEQ ID n° 7 ;
VH CDR2 : SEQ ID n° 8 ;
VH CDR3 : SEQ ID n° 9 ;
VL CDR1 : SEQ ID n° 10 ;
VL CDR2 : SEQ ID n° 11 ; et
VL CDR3 : SEQ ID n° 12.

4. CAR selon la revendication 2, dans lequel le domaine de fixation de l'antigène comprend une chaîne lourde variable (VH) ayant la séquence illustrée comme étant SEQ ID n° 1 et une chaîne légère variable (VL) ayant la séquence illustrée comme étant SEQ ID n° 2.

5. CAR selon la revendication 2, comprenant une séquence d'acides aminés choisie parmi SEQ ID n° 4, 5 et 6.

6. CAR selon la revendication 1, qui fixe sélectivement TRBC2.

7. Séquence d'acide nucléique, codant pour un CAR selon l'une quelconque des revendications précédentes.

8. Vecteur, exprimant une séquence d'acide nucléique selon la revendication 7.

9. Cellule, comprenant un CAR selon l'une quelconque des revendications 1 à 6.

10. Lymphocyte T selon la revendication 9.

11. Méthode de préparation d'une cellule selon la revendication 9 ou 10, comprenant l'étape de transduction ou de transfection d'une cellule ex *vivo* avec une séquence d'acide nucléique selon la revendication 7 ou un vecteur selon la revendication 8.

12. Cellule selon la revendication 9 ou 10, pour une utilisation dans une méthode de traitement d'un lymphome à lymphocytes T ou d'une leucémie chez un sujet, comprenant l'étape
d'administration de la cellule comprenant le CAR sélectif de TCRB1 ou TCRB2 au sujet, afin de provoquer une déplétion sélective des lymphocytes T malins, conjointement avec des lymphocytes T normaux exprimant le même TRBC que les lymphocytes T malins, mais ne provoquant pas la déplétion de lymphocytes T normaux exprimant le TRBC non exprimé par les lymphocytes T malins.

13. Cellule pour une utilisation selon la revendication 12, la méthode comprenant également l'étape consistant à étudier la région constante bêta du TCR (TCRB) d'un lymphocyte T malin issu du sujet, afin de déterminer s'il exprime ou non TRBC1 ou TRBC2.

14. Cellule pour une utilisation selon la revendication 12 ou 13, le lymphome à lymphocytes T ou la leucémie étant choisi(e) parmi le lymphome à lymphocytes T périphérique, non spécifié par ailleurs (PTCL-NOS) ; le lymphome à lymphocytes T angio-immunoblastique (AITL) ; le lymphome anaplasique à grandes cellules (ALCL) ; le lymphome à lymphocytes T associé à une entéropathie (EATL) ; le lymphome à lymphocytes T hépatosplénique (HSTL) ; le lymphome NK/T extraganglionnaire de type nasal, le lymphome à lymphocytes T cutané, l'ALCL cutané primitif, la leucémie pro-lymphocytaire T, et la leucémie lymphoblastique aiguë T.

15. Méthode de diagnostic d'un lymphome à lymphocytes T ou d'une leucémie chez un sujet, comprenant l'étape consistant à déterminer le pourcentage de lymphocytes T totaux dans un échantillon isolé à partir du sujet qui sont TRBC1- ou TRBC2-positifs.

16. Méthode de sélection d'une thérapie convenable pour traiter un sujet souffrant d'un lymphome à lymphocytes T ou d'une leucémie, comprenant :
i) la détermination du fait qu'un lymphocyte T malin issu du sujet exprime ou non TRBC1 ou TRBC2 ; et
ii) la sélection d'une cellule pour une utilisation selon la revendication 12, sur la base de l'expression de TRBC1 ou TRBC2 par ledit lymphocyte T malin.

17. Méthode de sélection d'un sujet souffrant d'un lymphome à lymphocytes T ou d'une leucémie afin qu'il reçoive une thérapie basée sur une cellule pour une utilisation selon la revendication 12, comprenant :
i) la détermination du fait qu'un lymphocyte T malin issu du sujet exprime ou non TRBC1 ou TRBC2 ; et
ii) la sélection dudit sujet afin qu'il reçoive une thérapie basée sur une cellule pour une utilisation selon la revendication 12, sur la base de l'expression de TRBC1 ou TRBC2 par ledit lymphocyte T malin.


### SEQUENCE LISTING

<110> UCL Business PLC
<120> Method
<130> P104381PCT
<150> GB 1403905.1
   <151> 2014-03-05
<150> GB 1416908.0
   <151> 2014-09-25
<160> 272
<170> PatentIn version 3.5
<210> 1
   <211> 124
   <212> PRT
   <213> Artificial Sequence
<220>
   <223> Variable heavy chain (VH) Jovi-1 VH
<400> 1
   Glu Val Arg Leu Gln Gln Ser Gly Pro Asp Leu Ile Lys Pro Gly Ala
   1               5                   10                  15
   Ser Val Lys Met Ser Cys Lys Ala Ser Gly Tyr Thr Phe Thr Gly Tyr
               20                  25                  30
   Val Met His Trp Val Lys Gln Arg Pro Gly Gln Gly Leu Glu Trp Ile
           35                  40                  45
   Gly Phe Ile Asn Pro Tyr Asn Asp Asp Ile Gln Ser Asn Glu Arg Phe
       50                  55                  60
   Arg Gly Lys Ala Thr Leu Thr Ser Asp Lys Ser Ser Thr Thr Ala Tyr
   65                  70                  75                  80
   Met Glu Leu Ser Ser Leu Thr Ser Glu Asp Ser Ala Val Tyr Tyr Cys
                   85                  90                  95
   Ala Arg Gly Ala Gly Tyr Asn Phe Asp Gly Ala Tyr Arg Phe Phe Asp
               100                 105                 110
   Phe Trp Gly Gln Gly Thr Thr Leu Thr Val Ser Ser
           115                 120
<210> 2
   <211> 113
   <212> PRT
   <213> Artificial Sequence
<220>
   <223> Variable light chain (VL) Jovi-1 VL
<400> 2
   Asp Val Val Met Thr Gln Ser Pro Leu Ser Leu Pro Val Ser Leu Gly
   1               5                   10                  15
   Asp Gln Ala Ser Ile Ser Cys Arg Ser Ser Gln Arg Leu Val His Ser
               20                  25                  30
   Asn Gly Asn Thr Tyr Leu His Trp Tyr Leu Gln Lys Pro Gly Gln Ser
           35                  40                  45
   Pro Lys Leu Leu Ile Tyr Arg Val Ser Asn Arg Phe Pro Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Ile Tyr Phe Cys Ser Gln Ser
                   85                  90                  95
   Thr His Val Pro Tyr Thr Phe Gly Gly Gly Thr Lys Leu Glu Ile Lys
               100                 105                 110
   Arg
<210> 3
   <211> 252
   <212> PRT
   <213> Artificial Sequence
<220>
   <223> Jovi-1 scFv
<400> 3
   Glu Val Arg Leu Gln Gln Ser Gly Pro Asp Leu Ile Lys Pro Gly Ala
   1               5                   10                  15
   Ser Val Lys Met Ser Cys Lys Ala Ser Gly Tyr Thr Phe Thr Gly Tyr
               20                  25                  30
   Val Met His Trp Val Lys Gln Arg Pro Gly Gln Gly Leu Glu Trp Ile
           35                  40                  45
   Gly Phe Ile Asn Pro Tyr Asn Asp Asp Ile Gln Ser Asn Glu Arg Phe
       50                  55                  60
   Arg Gly Lys Ala Thr Leu Thr Ser Asp Lys Ser Ser Thr Thr Ala Tyr
   65                  70                  75                  80
   Met Glu Leu Ser Ser Leu Thr Ser Glu Asp Ser Ala Val Tyr Tyr Cys
                   85                  90                  95
   Ala Arg Gly Ala Gly Tyr Asn Phe Asp Gly Ala Tyr Arg Phe Phe Asp
               100                 105                 110
   Phe Trp Gly Gln Gly Thr Thr Leu Thr Val Ser Ser Gly Gly Gly Gly
           115                 120                 125
   Ser Gly Gly Gly Gly Ser Gly Gly Gly Gly Ser Asp Val Val Met Thr
       130                 135                 140
   Gln Ser Pro Leu Ser Leu Pro Val Ser Leu Gly Asp Gln Ala Ser Ile
   145                 150                 155                 160
   Ser Cys Arg Ser Ser Gln Arg Leu Val His Ser Asn Gly Asn Thr Tyr
                   165                 170                 175
   Leu His Trp Tyr Leu Gln Lys Pro Gly Gln Ser Pro Lys Leu Leu Ile
               180                 185                 190
   Tyr Arg Val Ser Asn Arg Phe Pro Gly Val Pro Asp Arg Phe Ser Gly
           195                 200                 205
   Ser Gly Ser Gly Thr Asp Phe Thr Leu Lys Ile Ser Arg Val Glu Ala
       210                 215                 220
   Glu Asp Leu Gly Ile Tyr Phe Cys Ser Gln Ser Thr His Val Pro Tyr
   225                 230                 235                 240
   Thr Phe Gly Gly Gly Thr Lys Leu Glu Ile Lys Arg
                   245                 250
<210> 4
<400> 4
   000
<210> 5
<400> 5
   000
<210> 6
<400> 6
   000
<210> 7
   <211> 7
   <212> PRT
   <213> Artificial Sequence
<220>
   <223> Complementarity determining region (CDR) variable heavy chain (VH) CDR1
<400> 7
   Gly Tyr Thr Phe Thr Gly Tyr
   1               5
<210> 8
   <211> 6
   <212> PRT
   <213> Artificial Sequence
<220>
   <223> VH CDR2
<400> 8
   Asn Pro Tyr Asn Asp Asp
   1               5
<210> 9
   <211> 15
   <212> PRT
   <213> Artificial Sequence
<220>
   <223> VH CDR3
<400> 9
   Gly Ala Gly Tyr Asn Phe Asp Gly Ala Tyr Arg Phe Phe Asp Phe
   1               5                   10                  15
<210> 10
   <211> 16
   <212> PRT
   <213> Artificial Sequence
<220>
   <223> Variable light chain (VL) CDR1
<400> 10
   Arg Ser Ser Gln Arg Leu Val His Ser Asn Gly Asn Thr Tyr Leu His
   1               5                   10                  15
<210> 11
   <211> 7
   <212> PRT
   <213> Artificial Sequence
<220>
   <223> VL CDR2
<400> 11
   Arg Val Ser Asn Arg Phe Pro
   1 5
<210> 12
   <211> 9
   <212> PRT
   <213> Artificial Sequence
<220>
   <223> VL CDR3
<400> 12
   Ser Gln Ser Thr His Val Pro Tyr Thr
   1 5
<210> 13
   <211> 247
   <212> PRT
   <213> Artificial Sequence
<220>
   <223> Single chain fragment variable (scFv) CP_01_E09
<400> 13
   Gln Val Gln Leu Val Glu Ser Gly Gly Gly Val Val Gln Pro Gly Arg
   1 5 10 15
   Ser Leu Arg Leu Ser Cys Ala Ala Ser Gly Phe Thr Phe Ser Ser Tyr
   20 25 30
   Ala Met His Trp Val Arg Gln Ala Pro Gly Lys Gly Leu Glu Trp Val
   35 40 45
   Ala Val Ile Ser Tyr Asp Gly Ser Asn Lys Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Ala His Asn Ser Ser Ser Trp Ser Phe Asp Tyr Trp Gly Gln
   100 105 110
   Gly Thr Leu Val Thr Val Ser Ser Gly Gly Gly Gly Ser Gly Gly Gly
               115                 120                 125
   Gly Ser Gly Gly Gly Ala Ser Asp Ile Gln Met Thr Gln Ser Pro Ser
       130                 135                 140
   Ser Leu Ser Ala Ser Val Gly Asp Arg Val Thr Ile Thr Cys Arg Ala
   145                 150                 155                 160
   Ser Gln Ser Ile Ser Ser Tyr Leu Asn Trp Tyr Gln Gln Lys Pro Gly
                   165                 170                 175
   Lys Ala Pro Lys Leu Leu Ile Tyr Asp Ala Ser Asn Leu Glu Thr Gly
               180                 185                 190
   Val Pro Ser Arg Phe Ser Gly Ser Gly Ser Gly Thr Asp Phe Thr Phe
           195                 200                 205
   Thr Ile Ser Ser Leu Gln Pro Glu Asp Ile Ala Thr Tyr Tyr Cys Gln
       210                 215                 220
   Gln Tyr Asp Asn Leu Pro Leu Thr Phe Gly Gly Gly Thr Lys Val Asp
   225                 230                 235                 240
   Ile Lys Arg Thr Ala Ala Ala
                   245
<210> 14
   <211> 246
   <212> PRT
   <213> Artificial Sequence
<220>
   <223> scFv CP_01_D12
<400> 14
   Glu Val Gln Leu Leu Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Ala Met His Trp Val Arg Gln Ala Pro Gly Lys Gly Leu Glu Trp Val
           35                  40                  45
   Ala Val Ile Ser Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Gly Asp Thr Tyr Gly Phe Leu Asp Asn Trp Gly Gln Gly
               100                 105                 110
   Thr Met Val Thr Val Ser Ser Gly Gly Gly Gly Ser Gly Gly Gly Gly
           115                 120                 125
   Ser Gly Gly Gly Ala Ser Asp Ile Gln Met Thr Gln Ser Pro Ser Thr
       130                 135                 140
   Leu Ser Ala Ser Val Gly Asp Arg Val Thr Ile Thr Cys Arg Ala Ser
   145                 150                 155                 160
   Gln Ser Ile Ser Ser Trp Leu Ala Trp Tyr Gln Gln Lys Pro Gly Lys
                   165                 170                 175
   Ala Pro Lys Leu Leu Ile Tyr Lys Ala Ser Ser Leu Glu Ser Gly Val
               180                 185                 190
   Pro Ser Arg Phe Ser Gly Ser Gly Ser Gly Thr Asp Phe Thr Leu Thr
           195                 200                 205
   Ile Ser Ser Leu Gln Pro Glu Asp Phe Ala Thr Tyr Tyr Cys Gln Gln
       210                 215                 220
   Phe Asn Ala Tyr Pro Leu Thr Phe Gly Gly Gly Thr Lys Val Glu Ile
   225                 230                 235                 240
   Lys Arg Thr Ala Ala Ala
                   245
<210> 15
   <211> 246
   <212> PRT
   <213> Artificial Sequence
<220>
   <223> scFv CP_01_D10
<400> 15
   Gln Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Ser Ser Tyr
               20                  25                  30
   Ala Met His Trp Val Arg Gln Ala Pro Gly Lys Gly Leu Glu Trp Val
           35                  40                  45
   Ala Val Ile Ser Tyr Asp Gly Ser Ser Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Gly Gly Ser Phe Gly Ala Phe Asp Ile Trp Gly Gln Gly
               100                 105                 110
   Thr Leu Val Thr Val Ser Ser Gly Gly Gly Gly Ser Gly Gly Gly Gly
           115                 120                 125
   Ser Gly Gly Gly Ala Ser Asp Ile Gln Met Thr Gln Ser Pro Ser Ser
       130                 135                 140
   Leu Ser Ala Ser Val Gly Asp Arg Val Thr Ile Thr Cys Arg Ala Ser
   145                 150                 155                 160
   Gln Ser Ile Ser Arg Tyr Leu Asn Trp Tyr Gln Gln Lys Pro Gly Lys
                   165                 170                 175
   Ala Pro Asn Leu Leu Ile Tyr Ala Ala Ser Ser Leu Gln Ser Gly Val
               180                 185                 190
   Pro Ser Arg Phe Ser Gly Ser Gly Ser Gly Thr Asp Phe Thr Leu Thr
           195                 200                 205
   Ile Ser Ser Leu Gln Pro Glu Asp Phe Ala Thr Tyr Tyr Cys Gln Gln
       210                 215                 220
   Tyr Asn Ser Tyr Pro Leu Thr Phe Gly Gly Gly Thr Lys Leu Glu Ile
   225                 230                 235                 240
   Lys Arg Thr Ala Ala Ala
                   245
<210> 16
   <211> 246
   <212> PRT
   <213> Artificial Sequence
<220>
   <223> scFv CP_01_C08
<400> 16
   Glu Val Gln Leu Leu Glu Ser Gly Gly Gly Ala Val Gln Pro Gly Arg
   1               5                   10                  15
   Ser Leu Arg Leu Ser Cys Ala Ala Ser Gly Phe Thr Phe Ser Ser Tyr
               20                  25                  30
   Ala Met His Trp Val Arg Gln Ala Pro Gly Lys Gly Leu Glu Trp Val
           35                  40                  45
   Ala Val Ile Ser Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Ser Gly Tyr Ser Ser Ser Trp Tyr Leu Asp Tyr Trp Gly Gln Gly
               100                 105                 110
   Thr Leu Val Thr Val Ser Ser Gly Gly Gly Gly Ser Gly Gly Gly Gly
           115                 120                 125
   Ser Gly Gly Gly Ala Ser Asp Ile Gln Met Thr Gln Ser Pro Ser Ser
       130                 135                 140
   Val Ser Ala Ser Val Gly Asp Arg Val Thr Ile Thr Cys Gln Ala Ser
   145                 150                 155                 160
   Gln Asp Ile Ser Asn Tyr Leu Asn Trp Tyr Gln Gln Lys Pro Gly Lys
                   165                 170                 175
   Ala Pro Lys Leu Leu Ile Tyr Asp Ala Ser Asn Leu Glu Thr Gly Val
               180                 185                 190
   Pro Ser Arg Phe Ser Gly Ser Gly Ser Gly Thr Asp Phe Thr Phe Thr
           195                 200                 205
   Ile Ser Ser Leu Gln Pro Glu Asp Ile Ala Thr Tyr Tyr Cys Gln Gln
       210                 215                 220
   Tyr Asp Asn Leu Pro Leu Thr Phe Gly Gly Gly Thr Lys Val Glu Ile
   225                 230                 235                 240
   Lys Arg Thr Ala Ala Ala
                   245
<210> 17
   <211> 245
   <212> PRT
   <213> Artificial Sequence
<220>
   <223> scFv CP_01_C11
<400> 17
   Gln Val Gln Leu Val Glu Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Met His Trp Val Arg Gln Ala Pro Gly Gln Gly Leu Glu Trp Met
           35                  40                  45
   Gly Arg Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Ser Gly Gly Ala Gly Trp Asn Trp Gly Gln Gly Thr Met Val Thr
               100                 105                 110
   Val Ser Ser Gly Gly Gly Gly Ser Gly Gly Gly Gly Ser Gly Gly Gly
           115                 120                 125
   Ala Ser Asn Phe Met Leu Thr Gln Pro His Ser Val Ser Glu Ser Pro
       130                 135                 140
   Gly Lys Thr Ala Thr Ile Ser Cys Thr Arg Ser Ser Gly Ser Ile Ala
   145                 150                 155                 160
   Ser Asn Tyr Val Gln Trp Tyr Gln Gln Arg Pro Gly Ser Ala Pro Thr
                   165                 170                 175
   Thr Val Ile Tyr Glu Asp Asn Gln Arg Pro Phe Gly Val Pro Asp Arg
               180                 185                 190
   Phe Ser Gly Ser Ile Asp Ser Ser Ser Asn Ser Ala Ser Leu Thr Ile
           195                 200                 205
   Ser Gly Leu Lys Thr Glu Asp Glu Ala Asp Tyr Tyr Cys Gln Ser His
       210                 215                 220
   Asp Ser Ser Asn Val Val Phe Gly Gly Gly Thr Gln Leu Thr Val Leu
   225                 230                 235                 240
   Gly Gln Pro Ala Ala
                   245
<210> 18
   <211> 247
   <212> PRT
   <213> Artificial Sequence
<220>
   <223> scFv CP_01_F03
<220>
   <221> misc_feature
   <222> (101)..(101)
   <223> Xaa can be any naturally occurring amino acid
<400> 18
   Glu Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Ala Met His Trp Val Arg Gln Ala Pro Gly Lys Gly Leu Glu Trp Val
           35                  40                  45
   Ala Val Ile Ser Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Tyr Xaa Ala Ser Ser Trp Ser Gln Gly Leu Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Ala Ser Asp Ile Gln Met Thr Gln Ser Pro Ser
   130 135 140
   Ser Leu Ser Ala Ser Val Arg Asp Arg Val Thr Ile Thr Cys Gln Ala
   145 150 155 160
   Ser Gln Asp Ile Ser Asn Tyr Leu Asn Trp Tyr Gln Gln Lys Pro Gly
   165 170 175
   Lys Ala Pro Lys Leu Leu Ile Tyr Asp Ala Ser Asn Leu Glu Thr Gly
   180 185 190
   Val Pro Ser Arg Phe Ser Gly Ser Gly Ser Gly Thr Asp Phe Thr Phe
   195 200 205
   Thr Ile Ser Ser Leu Gln Pro Glu Asp Ile Ala Thr Tyr Tyr Cys Gln
   210 215 220
   Gln Tyr Asp Asn Leu Pro Pro Thr Phe Gly Gly Gly Thr Lys Val Glu
   225 230 235 240
   Ile Lys Arg Thr Ala Ala Ala
   245
<210> 19
   <211> 251
   <212> PRT
   <213> Artificial Sequence
<220>
   <223> scFv CP_01_E07
<400> 19
   Gln Val Gln Leu Val Glu Ser Gly Gly Gly Val Val Gln Pro Gly Arg
   1 5 10 15
   Ser Leu Arg Leu Ser Cys Ala Ala Ser Gly Phe Thr Phe Ser Ser Tyr
   20 25 30
   Gly Met His Trp Val Arg Gln Ala Pro Gly Lys Gly Leu Glu Trp Val
   35 40 45
   Ala Val Ile Ser Tyr Asp Gly Ser Asn Lys Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Asp Leu Gly Gly Ser Gly Gly Ala Phe Asp Ile Trp Gly Gln
   100 105 110
   Gly Thr Leu Val Thr Val Ser Ser Gly Gly Gly Gly Ser Gly Gly Gly
   115 120 125
   Gly Ser Gly Gly Gly Ala Ser Asp Ile Val Met Thr Gln Thr Pro His
   130 135 140
   Ser Leu Ser Val Thr Pro Gly Gln Pro Ala Ser Ile Ser Cys Lys Ser
   145 150 155 160
   Ser Gln Ser Leu Leu Tyr Ser Asp Gly Lys Thr Tyr Leu Tyr Trp Tyr
   165 170 175
   Leu Gln Lys Pro Gly Gln Pro Pro Gln Leu Leu Ile Tyr Glu Val Ser
   180 185 190
   Asn Arg Phe Ser Gly Val Pro Asp Arg Phe Ser Gly Ser Gly Ser Gly
   195 200 205
   Thr Asp Phe Thr Leu Lys Ile Ser Arg Val Glu Ala Glu Asp Val Gly
   210 215 220
   Val Tyr Tyr Cys Met Gln Ser Ile Gln Leu Tyr Thr Phe Gly Gln Gly
   225 230 235 240
   Thr Lys Val Asp Ile Lys Arg Thr Ala Ala Ala
   245 250
<210> 20
   <211> 244
   <212> PRT
   <213> Artificial Sequence
<220>
   <223> scFv CP_01_D03
<400> 20
   Gln Val Gln Leu Val Glu Ser Gly Gly Gly Val Val Gln Pro Gly Arg
   1               5                   10                  15
   Ser Leu Arg Leu Ser Cys Ala Ala Pro Gly Phe Thr Phe Ser Ser Tyr
               20                  25                  30
   Gly Met His Trp Val Arg Gln Ala Pro Gly Lys Gly Leu Glu Trp Val
   35 40 45
   Ala Val Ile Ser Tyr Asp Gly Ser Asn Lys Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Asn Lys Gln Tyr Gly Met Asp Val Trp Gly Gln Gly Thr Leu
   100 105 110
   Val Thr Val Ser Ser Gly Gly Gly Gly Ser Gly Gly Gly Gly Ser Gly
   115 120 125
   Gly Gly Ala Ser Asp Ile Val Met Thr Gln Ser Pro Ala Thr Leu Ser
   130 135 140
   Leu Ala Pro Gly Glu Arg Ala Thr Leu Ser Cys Arg Ala Ser Gln Ser
   145 150 155 160
   Val Gly Ser Asn Leu Ala Trp Tyr Gln Gln Lys Pro Gly Gln Ala Pro
   165 170 175
   Ser Leu Leu Ile Tyr Asp Ala Ser Thr Arg Ala Thr Gly Ile Pro Ala
   180 185 190
   Arg Phe Ser Gly Ser Gly Ser Gly Thr Asp Phe Thr Leu Thr Ile Ser
   195 200 205
   Ser Leu Gln Ser Glu Asp Ile Ala Val Tyr Tyr Cys Gln Gln Tyr His
   210 215 220
   Arg Trp Pro Leu Thr Phe Gly Gly Gly Thr Lys Val Glu Ile Lys Arg
   225 230 235 240
   Thr Ala Ala Ala
<210> 21
   <211> 249
   <212> PRT
   <213> Artificial Sequence
<220>
   <223> scFv CP_01_F06
<400> 21
   Glu Val Gln Leu Leu Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Val Ile Ser Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Asp Asp Gly Ala Met Arg Tyr Trp Gly Gln Gly Thr Met Val
               100                 105                 110
   Thr Val Ser Ser Gly Gly Gly Gly Ser Gly Gly Gly Gly Ser Gly Gly
           115                 120                 125
   Gly Ala Ser Asp Ile Gln Met Thr Gln Ser Pro Asp Ser Leu Ala Val
       130                 135                 140
   Ser Leu Gly Glu Arg Ala Thr Ile Asn Cys Lys Ser Ser Gln Ser Val
   145                 150                 155                 160
   Leu Tyr Ser Ser Asn Asn Lys Asn Tyr Leu Ala Trp Tyr Gln Gln Lys
                   165                 170                 175
   Pro Gly Gln Pro Pro Lys Leu Leu Ile Tyr Trp Ala Ser Thr Arg Glu
               180                 185                 190
   Ser Gly Val Pro Asp Arg Phe Ser Gly Ser Gly Ser Gly Thr Asp Phe
           195                 200                 205
   Thr Leu Thr Ile Ser Ser Leu Gln Ala Glu Asp Val Ala Val Tyr Tyr
       210                 215                 220
   Cys Gln Gln Tyr Tyr Asp Ser Pro Tyr Thr Phe Gly Gln Gly Thr Lys
   225                 230                 235                 240
   Val Asp Ile Lys Arg Thr Ala Ala Ala
   245
<210> 22
   <211> 244
   <212> PRT
   <213> Artificial Sequence
<220>
   <223> scFv CP_01_F02
<400> 22
   Gln Val Gln Leu Val Glu Ser Gly Gly Gly Val Val Gln Pro Gly Arg
   1               5                   10                  15
   Pro Leu Arg Leu Ser Cys Ala Ala Ser Gly Phe Thr Phe Ser Ser Tyr
               20                  25                  30
   Ala Met His Trp Val Arg Gln Ala Pro Gly Lys Gly Leu Glu Trp Val
           35                  40                  45
   Ala Val Ile Ser Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Ala Gly Tyr Ser Tyr Ala Asp Tyr Trp Gly Gln Gly Thr Met
               100                 105                 110
   Val Thr Val Ser Ser Gly Gly Gly Gly Ser Gly Gly Gly Gly Ser Gly
           115                 120                 125
   Gly Gly Ala Ser Asp Ile Gln Met Thr Gln Ser Pro Ser Ser Leu Ser
       130                 135                 140
   Ala Ser Val Gly Asp Arg Val Thr Ile Thr Cys Arg Ala Ser Gln Gly
   145                 150                 155                 160
   Ile Arg Asn Asp Leu Gly Trp Tyr Gln Gln Lys Pro Gly Lys Ala Pro
                   165                 170                 175
   Lys Arg Leu Ile Tyr Ala Ala Ser Ser Leu Gln Ser Gly Val Pro Ser
               180                 185                 190
   Arg Phe Ser Gly Ser Gly Ser Gly Thr Glu Phe Thr Leu Thr Ile Ser
           195                 200                 205
   Ser Leu Gln Pro Glu Asp Phe Ala Thr Tyr Tyr Cys Leu Gln His Asn
       210                 215                 220
   Ser Tyr Pro Leu Thr Phe Gly Gly Gly Thr Lys Val Asp Ile Lys Arg
   225                 230                 235                 240
   Thr Ala Ala Ala
<210> 23
   <211> 249
   <212> PRT
   <213> Artificial Sequence
<220>
   <223> scFv CP_03_E05
<400> 23
   Glu Val Gln Leu Val Glu Ser Gly Gly Gly Val Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Ser Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Thr Arg Ser Ser Gly Ala Phe Asp Ile Trp Gly Gln Gly Thr
               100                 105                 110
   Leu Val Thr Val Ser Ser Gly Gly Gly Gly Ser Gly Gly Gly Gly Ser
           115                 120                 125
   Gly Gly Gly Ala Ser Asn Phe Met Leu Thr Gln Pro His Ser Val Ser
       130                 135                 140
   Glu Ser Pro Gly Lys Thr Val Thr Ile Ser Cys Thr Arg Ser Ser Gly
   145                 150                 155                 160
   Ser Ile Ala Ser Lys Tyr Val Gln Trp Tyr Gln Gln Arg Pro Gly Ser
   165 170 175
   Ser Pro Thr Thr Val Ile Tyr Glu Asp Asn Gln Arg Pro Ser Gly Val
   180 185 190
   Pro Asp Arg Phe Ser Gly Ser Ile Asp Thr Ser Ser Asn Ser Ala Ser
   195 200 205
   Leu Thr Ile Ser Gly Leu Arg Thr Glu Asp Glu Ala Asp Tyr Tyr Cys
   210 215 220
   His Ser Tyr Asp Ser Asn Asn His Ser Val Phe Gly Gly Gly Thr Lys
   225 230 235 240
   Val Thr Val Leu Gly Gln Pro Ala Ala
   245
<210> 24
   <211> 246
   <212> PRT
   <213> Artificial Sequence
<220>
   <223> scFv CP_03_D05
<400> 24
   Gln Val Gln Leu Val Glu Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
   35 40 45
   Gly Arg Ile Asn Pro Asn Ser Gly Gly Thr Asn Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Ser Arg Leu Arg Ser Asp Asp Thr Ala Val Tyr Tyr Cys
   85 90 95
   Ala Ser Pro Arg Gly Arg Gly Ser Ala Phe Asp Ile Trp Gly Gln Gly
   100 105 110
   Thr Leu Val Thr Val Ser Ser Gly Gly Gly Gly Ser Gly Gly Gly Gly
           115                 120                 125
   Ser Gly Gly Gly Ala Ser Ser Tyr Glu Leu Thr Gln Pro Pro Ser Val
       130                 135                 140
   Ser Val Ser Pro Gly Gln Thr Ala Thr Ile Ser Cys Ser Gly Asp Gln
   145                 150                 155                 160
   Leu Gly Gly Lys Tyr Gly His Trp Tyr Gln Lys Lys Pro Gly Gln Ser
                   165                 170                 175
   Pro Val Leu Val Leu Tyr Gln Asp Arg Lys Arg Pro Ala Gly Ile Pro
               180                 185                 190
   Glu Arg Phe Ser Gly Ser Ser Ser Gly Asn Thr Ile Thr Leu Thr Ile
           195                 200                 205
   Ser Gly Thr Gln Ala Val Asp Glu Ala Asp Tyr Tyr Cys Gln Ala Trp
       210                 215                 220
   Asp Thr Asn Leu Gly Gly Val Phe Gly Gly Gly Thr Lys Val Thr Val
   225                 230                 235                 240
   Leu Gly Gln Pro Ala Ala
                   245
<210> 25
   <211> 249
   <212> PRT
   <213> Artificial Sequence
<220>
   <223> scFv CP_03_H06
<400> 25
   Gln Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Asp Tyr
               20                  25                  30
   Tyr Met Ser Trp Ile Arg Gln Ala Pro Gly Lys Gly Leu Glu Trp Val
           35                  40                  45
   Ser Tyr Ile Ser Ser Ser Gly Ser Thr Ile Tyr Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Thr Glu Asp Thr Ala Val Tyr Tyr Cys
                   85                  90                  95
   Ala Arg Ala Arg Val Gly Gly Met Asp Val Trp Gly Gln Gly Thr Met
               100                 105                 110
   Val Thr Val Ser Ser Gly Gly Gly Gly Ser Gly Gly Gly Gly Ser Gly
           115                 120                 125
   Gly Gly Ala Ser Asn Phe Met Leu Thr Gln Pro His Ser Val Ser Glu
       130                 135                 140
   Ser Pro Gly Lys Thr Val Thr Ile Ser Cys Thr Arg Ser Ser Gly Ser
   145                 150                 155                 160
   Ile Ala Ser Asn Tyr Val Gln Trp Tyr Gln Gln Arg Pro Gly Ser Ser
                   165                 170                 175
   Pro Thr Thr Val Ile Tyr Glu Asp Asn Gln Arg Pro Ser Gly Val Pro
               180                 185                 190
   Asp Arg Phe Ser Gly Ser Ile Asp Ser Ser Ser Asn Ser Ala Ser Leu
           195                 200                 205
   Thr Ile Ser Gly Leu Lys Thr Glu Asp Glu Ala Asp Tyr Tyr Cys Gln
       210                 215                 220
   Ser Phe Asp Ala Asp Asn Leu His Val Val Phe Gly Gly Gly Thr Lys
   225                 230                 235                 240
   Leu Thr Val Leu Gly Gln Pro Ala Ala
                   245
<210> 26
   <211> 248
   <212> PRT
   <213> Artificial Sequence
<220>
   <223> scFv CP_03_C12
<400> 26
   Glu Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asp Thr Gly Pro Ile Asp Tyr Trp Gly Gln Gly Thr Met Val
               100                 105                 110
   Thr Val Ser Ser Gly Gly Gly Gly Ser Gly Gly Gly Gly Ser Gly Gly
           115                 120                 125
   Gly Ala Ser Asp Ile Val Met Thr Gln Thr Pro Leu Ser Leu Ser Val
       130                 135                 140
   Thr Pro Gly Gln Pro Ala Ser Ile Ser Cys Lys Ser Ser Gln Ser Leu
   145                 150                 155                 160
   Leu His Ser Asp Gly Lys Thr Tyr Leu Tyr Trp Tyr Leu Gln Lys Pro
                   165                 170                 175
   Gly Gln Pro Pro Gln Leu Leu Val Tyr Glu Val Ser Asn Arg Phe Ser
               180                 185                 190
   Gly Val Pro Asp Lys Phe Ser Gly Ser Gly Ser Gly Thr Asp Phe Thr
           195                 200                 205
   Leu Lys Ile Ser Arg Val Glu Ala Glu Asp Val Gly Val Tyr Tyr Cys
       210                 215                 220
   Met Gln Gly Ile Gln Leu Pro Pro Thr Phe Gly Gly Gly Thr Lys Val
   225                 230                 235                 240
   Asp Ile Lys Arg Thr Ala Ala Ala
                   245
<210> 27
   <211> 249
   <212> PRT
   <213> Artificial Sequence
<220>
   <223> scFv CP_03_G02
<400> 27
   Glu Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Ile Ile Asn Pro Ser Gly Gly Ser Thr Ser Tyr Ala Gln Lys Phe
       50                  55                  60
   Gln Gly Arg Val Thr Met Thr Arg Asp Thr Ser Thr Ser Ile Val Tyr
   65                  70                  75                  80
   Met Glu Leu Ser Ser Leu Arg Ser Glu Asp Thr Ala Val Tyr Tyr Cys
                   85                  90                  95
   Ala Arg Gly Val Trp Asn Ser Gly Ser Tyr Leu Gly Phe Asp Tyr Trp
               100                 105                 110
   Gly Gln Gly Thr Leu Val Thr Val Ser Ser Gly Gly Gly Gly Ser Gly
           115                 120                 125
   Gly Gly Gly Ser Gly Gly Gly Ala Ser Asp Ile Gln Met Thr Gln Ser
       130                 135                 140
   Pro Ser Ser Leu Ser Ala Ser Val Gly Asp Arg Val Thr Ile Thr Cys
   145                 150                 155                 160
   Gln Ala Ser Gln Asp Ile Ser Asn Tyr Leu Asn Trp Tyr Gln Gln Lys
                   165                 170                 175
   Pro Gly Lys Ala Pro Lys Leu Leu Ile Tyr Ala Ala Ser Ser Leu Gln
               180                 185                 190
   Ser Gly Val Pro Ser Arg Phe Ser Gly Ser Gly Ser Gly Thr Asp Phe
           195                 200                 205
   Thr Leu Thr Ile Ser Ser Leu Gln Pro Glu Asp Phe Ala Thr Tyr Tyr
       210                 215                 220
   Cys Gln Gln Ser Tyr Ser Thr Pro Leu Thr Phe Gly Gly Gly Thr Lys
   225                 230                 235                 240
   Leu Glu Ile Lys Arg Thr Ala Ala Ala
                   245
<210> 28
   <211> 251
   <212> PRT
   <213> Artificial Sequence
<220>
   <223> scFv CP_03_D04
<400> 28
   Glu Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Ala Met His Trp Val Arg Gln Ala Pro Gly Lys Gly Leu Glu Trp Val
           35                  40                  45
   Ala Val Ile Ser Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Phe Thr Val Pro Gly Gly Ala Phe Asp Ile Trp Gly
               100                 105                 110
   Gln Gly Thr Leu Val Thr Val Ser Ser Gly Gly Gly Gly Ser Gly Gly
           115                 120                 125
   Gly Gly Ser Gly Gly Gly Ala Ser Asn Phe Met Leu Thr Gln Pro His
       130                 135                 140
   Ser Val Ser Asp Ser Pro Gly Lys Thr Val Thr Ile Ser Cys Thr Arg
   145                 150                 155                 160
   Ser Ser Gly Arg Ile Gly Ser Asn Phe Val Gln Trp Tyr Gln Gln Arg
                   165                 170                 175
   Pro Gly Ser Ser Pro Thr Thr Val Ile Tyr Glu Asp Asp Gln Arg Pro
               180                 185                 190
   Ser Gly Val Pro Ala Arg Phe Ser Gly Ser Ile Asp Ser Ser Ser Asn
   195 200 205
   Ser Ala Ser Leu Thr Ile Ser Gly Leu Thr Thr Ala Asp Glu Ala Gly
   210 215 220
   Tyr Tyr Cys Gln Ser Tyr Asp Ala Ser Asn Val Ile Phe Gly Gly Gly
   225 230 235 240
   Thr Lys Leu Thr Val Leu Gly Gln Pro Ala Ala
   245 250
<210> 29
   <211> 247
   <212> PRT
   <213> Artificial Sequence
<220>
   <223> scFv CP_03_F10
<400> 29
   Glu Val Gln Leu Val Glu Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
   35 40 45
   Gly Arg Ile Asn Pro Asn Ser Gly Gly Thr Asn Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Ser Ser Leu Arg Ser Glu Asp Thr Ala Val Tyr Tyr Cys
   85 90 95
   Ala Arg Phe Gly Glu Arg Tyr Ala Phe Asp Ile Trp Gly Gln Gly Thr
   100 105 110
   Leu Val Thr Val Ser Ser Gly Gly Gly Gly Ser Gly Gly Gly Gly Ser
   115 120 125
   Gly Gly Gly Ala Ser Gln Ser Glu Leu Thr Gln Pro Pro Ser Ala Ser
   130 135 140
   Gly Ser Pro Gly Gln Ser Val Thr Ile Ser Cys Thr Gly Thr Ser Thr
   145                 150                 155                 160
   Asp Val Gly Ala Phe His Phe Val Ser Trp Tyr Gln His Thr Pro Gly
                   165                 170                 175
   Lys Ala Pro Lys Leu Leu Ile Ser Glu Val Arg Lys Arg Ala Ser Gly
               180                 185                 190
   Val Pro Asp Arg Phe Ser Gly Ser Arg Ser Gly Asn Thr Ala Ser Leu
           195                 200                 205
   Thr Val Ser Gly Leu Gln Ser Glu Asp Glu Ala Asp Tyr Phe Cys Ser
       210                 215                 220
   Ala Tyr Thr Gly Ser Asn Tyr Val Phe Gly Ser Gly Thr Lys Leu Thr
   225                 230                 235                 240
   Val Leu Gly Gln Pro Ala Ala
                   245
<210> 30
   <211> 252
   <212> PRT
   <213> Artificial Sequence
<220>
   <223> scFv CP_03_G09
<400> 30
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Ser Ala Ala Trp Asn Trp Ile Arg Gln Ser Pro Ser Arg Gly Leu Glu
           35                  40                  45
   Trp Leu Gly Arg Thr Tyr Tyr Arg Ser Lys Trp Tyr Asn Asp Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Asp Gln Trp Leu Ala Asn Tyr Tyr Tyr Tyr Gly
                   100                 105                 110
   Met Asp Val Trp Gly Gln Gly Thr Leu Val Thr Val Ser Ser Gly Gly
           115                 120                 125
   Gly Gly Ser Gly Gly Gly Gly Ser Gly Gly Gly Ala Ser Ser Tyr Glu
       130                 135                 140
   Leu Thr Gln Pro Leu Ser Val Ser Val Ala Leu Gly Gln Thr Ala Arg
   145                 150                 155                 160
   Ile Thr Cys Gly Gly Asn Asn Ile Gly Ser Lys Asn Val His Trp Tyr
                   165                 170                 175
   Gln Gln Lys Pro Gly Gln Ala Pro Val Leu Val Ile Tyr Arg Asp Asn
               180                 185                 190
   Asn Arg Pro Ser Gly Ile Pro Glu Arg Phe Ser Gly Ser Asn Ser Gly
           195                 200                 205
   Asn Thr Ala Thr Leu Thr Ile Ser Lys Ala Gln Ala Gly Asp Glu Ala
       210                 215                 220
   Asp Tyr Tyr Cys Gln Val Trp Asp Ser Asn Ser Trp Val Phe Gly Gly
   225                 230                 235                 240
   Gly Thr Lys Leu Thr Val Leu Gly Gln Pro Ala Ala
                   245                 250
<210> 31
   <211> 252
   <212> PRT
   <213> Artificial Sequence
<220>
   <223> scFv CP_03_F09
<400> 31
   Gln Met Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Ala Ser Tyr
               20                  25                  30
   Tyr Met His Trp Val Arg Gln Ala Pro Gly Gln Gly Leu Glu Trp Met
           35                  40                  45
   Gly Ile Ile Asn Pro Ser Gly Gly Ser Thr Ser Tyr Ala Gln Lys Phe
       50                  55                  60
   Gln Gly Arg Val Thr Met Thr Arg Asp Thr Ser Thr Ser Thr Val Tyr
   65                  70                  75                  80
   Met Glu Leu Ser Arg Leu Arg Ser Asp Asp Thr Ala Val Tyr Tyr Cys
                   85                  90                  95
   Ala Ser Asn Arg Gly Gly Ser Tyr Lys Ser Val Gly Met Asp Val Trp
               100                 105                 110
   Gly Gln Gly Thr Thr Val Thr Val Ser Ser Gly Gly Gly Gly Ser Gly
           115                 120                 125
   Gly Gly Gly Ser Gly Gly Gly Ala Ser Asn Phe Met Leu Thr Gln Pro
       130                 135                 140
   Gln Ser Val Ser Glu Ser Pro Gly Lys Thr Val Thr Ile Ser Cys Thr
   145                 150                 155                 160
   Arg Ser Ser Gly Asn Phe Ala Ser Lys Tyr Val Gln Trp Tyr Gln Gln
                   165                 170                 175
   Arg Pro Gly Ser Ser Pro Thr Thr Val Ile Tyr Glu Asn Tyr Gln Arg
               180                 185                 190
   Pro Ser Gly Val Pro Asp Arg Phe Ser Gly Ser Ile Asp Ser Ser Ser
           195                 200                 205
   Asn Ser Ala Thr Leu Thr Ile Ser Gly Leu Lys Thr Glu Asp Glu Ala
       210                 215                 220
   Asp Tyr Tyr Cys Gln Ser Tyr Asp Glu Val Ser Val Val Phe Gly Gly
   225                 230                 235                 240
   Gly Thr Gln Leu Thr Val Leu Gly Gln Pro Ala Ala
                   245                 250
<210> 32
   <211> 249
   <212> PRT
   <213> Artificial Sequence
<220>
   <223> scFv CP_03_D09
<400> 32
   Glu Val Gln Leu Val Gln Ser Gly Ala Glu Val Lys Lys Pro Gly Ser
   1               5                   10                  15
   Ser Val Lys Val Ser Cys Glu Ala Ser Gly Tyr Thr Phe Thr Ser Tyr
               20                  25                  30
   Ala Ile Ser Trp Val Arg Gln Ala Pro Gly Gln Gly Leu Glu Trp Met
           35                  40                  45
   Gly Trp Met Asn Pro Asn Ser Gly Asn Thr Gly Tyr Ala Gln Lys Phe
       50                  55                  60
   Gln Gly Arg Val Thr Met Thr Arg Asn Thr Ser Ile Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Val Ser Ser Tyr Tyr Gly Met Asp Val Trp Gly Gln Gly Thr
               100                 105                 110
   Leu Val Thr Val Ser Ser Gly Gly Gly Gly Ser Gly Gly Gly Gly Ser
           115                 120                 125
   Gly Gly Gly Ala Ser Asn Phe Met Leu Thr Gln Pro Leu Ser Val Ser
       130                 135                 140
   Glu Ser Pro Gly Lys Thr Val Thr Ile Ser Cys Thr Arg Ser Ser Gly
   145                 150                 155                 160
   Ser Ile Ala Ser Asn Tyr Val Gln Trp Tyr Gln Gln Arg Pro Gly Ser
                   165                 170                 175
   Ala Pro Thr Thr Val Ile Tyr Glu Asp Asn Gln Arg Pro Ser Gly Val
               180                 185                 190
   Pro Asp Arg Phe Ser Gly Ser Ile Asp Ser Ser Ser Asn Ser Ala Ser
           195                 200                 205
   Leu Thr Ile Ser Gly Leu Lys Thr Glu Asp Glu Ala Asp Tyr Tyr Cys
       210                 215                 220
   Gln Ser Tyr Asn Ser Ser Asn His Trp Val Phe Gly Gly Gly Thr Lys
   225                 230                 235                 240
   Val Thr Val Leu Gly Gln Pro Ala Ala
                   245
<210> 33
   <211> 537
   <212> PRT
   <213> Artificial Sequence
<220>
   <223> Chimeric antigen receptor (CAR), JOVI-1 CAR with CD8 stalk spacer
<400> 33
   Met Glu Thr Asp Thr Leu Leu Leu Trp Val Leu Leu Val Trp Ile Pro
   1               5                   10                  15
   Gly Ser Thr Gly Glu Val Arg Leu Gln Gln Ser Gly Pro Asp Leu Ile
               20                  25                  30
   Lys Pro Gly Ala Ser Val Lys Met Ser Cys Lys Ala Ser Gly Tyr Thr
           35                  40                  45
   Phe Thr Gly Tyr Val Met His Trp Val Lys Gln Arg Pro Gly Gln Gly
       50                  55                  60
   Leu Glu Trp Ile Gly Phe Ile Asn Pro Tyr Asn Asp Asp Ile Gln Ser
   65                  70                  75                  80
   Asn Glu Arg Phe Arg Gly Lys Ala Thr Leu Thr Ser Asp Lys Ser Ser
                   85                  90                  95
   Thr Thr Ala Tyr Met Glu Leu Ser Ser Leu Thr Ser Glu Asp Ser Ala
               100                 105                 110
   Val Tyr Tyr Cys Ala Arg Gly Ala Gly Tyr Asn Phe Asp Gly Ala Tyr
           115                 120                 125
   Arg Phe Phe Asp Phe Trp Gly Gln Gly Thr Thr Leu Thr Val Ser Ser
       130                 135                 140
   Gly Gly Gly Gly Ser Gly Gly Gly Gly Ser Gly Gly Gly Gly Ser Asp
   145                 150                 155                 160
   Val Val Met Thr Gln Ser Pro Leu Ser Leu Pro Val Ser Leu Gly Asp
                   165                 170                 175
   Gln Ala Ser Ile Ser Cys Arg Ser Ser Gln Arg Leu Val His Ser Asn
               180                 185                 190
   Gly Asn Thr Tyr Leu His Trp Tyr Leu Gln Lys Pro Gly Gln Ser Pro
           195                 200                 205
   Lys Leu Leu Ile Tyr Arg Val Ser Asn Arg Phe Pro Gly Val Pro Asp
       210                 215                 220
   Arg Phe Ser Gly Ser Gly Ser Gly Thr Asp Phe Thr Leu Lys Ile Ser
   225                 230                 235                 240
   Arg Val Glu Ala Glu Asp Leu Gly Ile Tyr Phe Cys Ser Gln Ser Thr
                   245                 250                 255
   His Val Pro Tyr Thr Phe Gly Gly Gly Thr Lys Leu Glu Ile Lys Arg
               260                 265                 270
   Ser Asp Pro Thr Thr Thr Pro Ala Pro Arg Pro Pro Thr Pro Ala Pro
           275                 280                 285
   Thr Ile Ala Ser Gln Pro Leu Ser Leu Arg Pro Glu Ala Cys Arg Pro
       290                 295                 300
   Ala Ala Gly Gly Ala Val His Thr Arg Gly Leu Asp Phe Ala Cys Asp
   305                 310                 315                 320
   Ile Phe Trp Val Leu Val Val Val Gly Gly Val Leu Ala Cys Tyr Ser
                   325                 330                 335
   Leu Leu Val Thr Val Ala Phe Ile Ile Phe Trp Val Arg Ser Lys Arg
               340                 345                 350
   Ser Arg Leu Leu His Ser Asp Tyr Met Asn Met Thr Pro Arg Arg Pro
           355                 360                 365
   Gly Pro Thr Arg Lys His Tyr Gln Pro Tyr Ala Pro Pro Arg Asp Phe
       370                 375                 380
   Ala Ala Tyr Arg Ser Arg Asp Gln Arg Leu Pro Pro Asp Ala His Lys
   385                 390                 395                 400
   Pro Pro Gly Gly Gly Ser Phe Arg Thr Pro Ile Gln Glu Glu Gln Ala
                   405                 410                 415
   Asp Ala His Ser Thr Leu Ala Lys Ile Arg Val Lys Phe Ser Arg Ser
               420                 425                 430
   Ala Asp Ala Pro Ala Tyr Gln Gln Gly Gln Asn Gln Leu Tyr Asn Glu
           435                 440                 445
   Leu Asn Leu Gly Arg Arg Glu Glu Tyr Asp Val Leu Asp Lys Arg Arg
       450                 455                 460
   Gly Arg Asp Pro Glu Met Gly Gly Lys Pro Arg Arg Lys Asn Pro Gln
   465                 470                 475                 480
   Glu Gly Leu Tyr Asn Glu Leu Gln Lys Asp Lys Met Ala Glu Ala Tyr
   485 490 495
   Ser Glu Ile Gly Met Lys Gly Glu Arg Arg Arg Gly Lys Gly His Asp
   500 505 510
   Gly Leu Tyr Gln Gly Leu Ser Thr Ala Thr Lys Asp Thr Tyr Asp Ala
   515 520 525
   Leu His Met Gln Ala Leu Pro Pro Arg
   530 535
<210> 34
   <211> 727
   <212> PRT
   <213> Artificial Sequence
<220>
   <223> JOVI-1 CAR with H-CH2-CH3pvaa spacer
<400> 34
   Met Glu Thr Asp Thr Leu Leu Leu Trp Val Leu Leu Val Trp Ile Pro
   1 5 10 15
   Gly Ser Thr Gly Glu Val Arg Leu Gln Gln Ser Gly Pro Asp Leu Ile
   20 25 30
   Lys Pro Gly Ala Ser Val Lys Met Ser Cys Lys Ala Ser Gly Tyr Thr
   35 40 45
   Phe Thr Gly Tyr Val Met His Trp Val Lys Gln Arg Pro Gly Gln Gly
   50 55 60
   Leu Glu Trp Ile Gly Phe Ile Asn Pro Tyr Asn Asp Asp Ile Gln Ser
   65 70 75 80
   Asn Glu Arg Phe Arg Gly Lys Ala Thr Leu Thr Ser Asp Lys Ser Ser
   85 90 95
   Thr Thr Ala Tyr Met Glu Leu Ser Ser Leu Thr Ser Glu Asp Ser Ala
   100 105 110
   Val Tyr Tyr Cys Ala Arg Gly Ala Gly Tyr Asn Phe Asp Gly Ala Tyr
   115 120 125
   Arg Phe Phe Asp Phe Trp Gly Gln Gly Thr Thr Leu Thr Val Ser Ser
   130 135 140
   Gly Gly Gly Gly Ser Gly Gly Gly Gly Ser Gly Gly Gly Gly Ser Asp
   145                 150                 155                 160
   Val Val Met Thr Gln Ser Pro Leu Ser Leu Pro Val Ser Leu Gly Asp
                   165                 170                 175
   Gln Ala Ser Ile Ser Cys Arg Ser Ser Gln Arg Leu Val His Ser Asn
               180                 185                 190
   Gly Asn Thr Tyr Leu His Trp Tyr Leu Gln Lys Pro Gly Gln Ser Pro
           195                 200                 205
   Lys Leu Leu Ile Tyr Arg Val Ser Asn Arg Phe Pro Gly Val Pro Asp
       210                 215                 220
   Arg Phe Ser Gly Ser Gly Ser Gly Thr Asp Phe Thr Leu Lys Ile Ser
   225                 230                 235                 240
   Arg Val Glu Ala Glu Asp Leu Gly Ile Tyr Phe Cys Ser Gln Ser Thr
                   245                 250                 255
   His Val Pro Tyr Thr Phe Gly Gly Gly Thr Lys Leu Glu Ile Lys Arg
               260                 265                 270
   Ser Asp Pro Ala Glu Pro Lys Ser Pro Asp Lys Thr His Thr Cys Pro
           275                 280                 285
   Pro Cys Pro Ala Pro Pro Val Ala Gly Pro Ser Val Phe Leu Phe Pro
       290                 295                 300
   Pro Lys Pro Lys Asp Thr Leu Met Ile Ala Arg Thr Pro Glu Val Thr
   305                 310                 315                 320
   Cys Val Val Val Asp Val Ser His Glu Asp Pro Glu Val Lys Phe Asn
                   325                 330                 335
   Trp Tyr Val Asp Gly Val Glu Val His Asn Ala Lys Thr Lys Pro Arg
               340                 345                 350
   Glu Glu Gln Tyr Asn Ser Thr Tyr Arg Val Val Ser Val Leu Thr Val
           355                 360                 365
   Leu His Gln Asp Trp Leu Asn Gly Lys Glu Tyr Lys Cys Lys Val Ser
       370                 375                 380
   Asn Lys Ala Leu Pro Ala Pro Ile Glu Lys Thr Ile Ser Lys Ala Lys
   385                 390                 395                 400
   Gly Gln Pro Arg Glu Pro Gln Val Tyr Thr Leu Pro Pro Ser Arg Asp
   405 410 415
   Glu Leu Thr Lys Asn Gln Val Ser Leu Thr Cys Leu Val Lys Gly Phe
   420 425 430
   Tyr Pro Ser Asp Ile Ala Val Glu Trp Glu Ser Asn Gly Gln Pro Glu
   435 440 445
   Asn Asn Tyr Lys Thr Thr Pro Pro Val Leu Asp Ser Asp Gly Ser Phe
   450 455 460
   Phe Leu Tyr Ser Lys Leu Thr Val Asp Lys Ser Arg Trp Gln Gln Gly
   465 470 475 480
   Asn Val Phe Ser Cys Ser Val Met His Glu Ala Leu His Asn His Tyr
   485 490 495
   Thr Gln Lys Ser Leu Ser Leu Ser Pro Gly Lys Lys Asp Pro Lys Phe
   500 505 510
   Trp Val Leu Val Val Val Gly Gly Val Leu Ala Cys Tyr Ser Leu Leu
   515 520 525
   Val Thr Val Ala Phe Ile Ile Phe Trp Val Arg Ser Lys Arg Ser Arg
   530 535 540
   Leu Leu His Ser Asp Tyr Met Asn Met Thr Pro Arg Arg Pro Gly Pro
   545 550 555 560
   Thr Arg Lys His Tyr Gln Pro Tyr Ala Pro Pro Arg Asp Phe Ala Ala
   565 570 575
   Tyr Arg Ser Arg Asp Gln Arg Leu Pro Pro Asp Ala His Lys Pro Pro
   580 585 590
   Gly Gly Gly Ser Phe Arg Thr Pro Ile Gln Glu Glu Gln Ala Asp Ala
   595 600 605
   His Ser Thr Leu Ala Lys Ile Arg Val Lys Phe Ser Arg Ser Ala Asp
   610 615 620
   Ala Pro Ala Tyr Gln Gln Gly Gln Asn Gln Leu Tyr Asn Glu Leu Asn
   625 630 635 640
   Leu Gly Arg Arg Glu Glu Tyr Asp Val Leu Asp Lys Arg Arg Gly Arg
   645 650 655
   Asp Pro Glu Met Gly Gly Lys Pro Arg Arg Lys Asn Pro Gln Glu Gly
   660 665 670
   Leu Tyr Asn Glu Leu Gln Lys Asp Lys Met Ala Glu Ala Tyr Ser Glu
   675 680 685
   Ile Gly Met Lys Gly Glu Arg Arg Arg Gly Lys Gly His Asp Gly Leu
   690 695 700
   Tyr Gln Gly Leu Ser Thr Ala Thr Lys Asp Thr Tyr Asp Ala Leu His
   705 710 715 720
   Met Gln Ala Leu Pro Pro Arg
   725
<210> 35
   <211> 511
   <212> PRT
   <213> Artificial Sequence
<220>
   <223> JOVI-1 CAR with IgG1 hinge spacer
<400> 35
   Met Glu Thr Asp Thr Leu Leu Leu Trp Val Leu Leu Val Trp Ile Pro
   1               5                   10                  15
   Gly Ser Thr Gly Glu Val Arg Leu Gln Gln Ser Gly Pro Asp Leu Ile
               20                  25                  30
   Lys Pro Gly Ala Ser Val Lys Met Ser Cys Lys Ala Ser Gly Tyr Thr
           35                  40                  45
   Phe Thr Gly Tyr Val Met His Trp Val Lys Gln Arg Pro Gly Gln Gly
       50                  55                  60
   Leu Glu Trp Ile Gly Phe Ile Asn Pro Tyr Asn Asp Asp Ile Gln Ser
   65                  70                  75                  80
   Asn Glu Arg Phe Arg Gly Lys Ala Thr Leu Thr Ser Asp Lys Ser Ser
                   85                  90                  95
   Thr Thr Ala Tyr Met Glu Leu Ser Ser Leu Thr Ser Glu Asp Ser Ala
               100                 105                 110
   Val Tyr Tyr Cys Ala Arg Gly Ala Gly Tyr Asn Phe Asp Gly Ala Tyr
           115                 120                 125
   Arg Phe Phe Asp Phe Trp Gly Gln Gly Thr Thr Leu Thr Val Ser Ser
       130                 135                 140
   Gly Gly Gly Gly Ser Gly Gly Gly Gly Ser Gly Gly Gly Gly Ser Asp
   145                 150                 155                 160
   Val Val Met Thr Gln Ser Pro Leu Ser Leu Pro Val Ser Leu Gly Asp
                   165                 170                 175
   Gln Ala Ser Ile Ser Cys Arg Ser Ser Gln Arg Leu Val His Ser Asn
               180                 185                 190
   Gly Asn Thr Tyr Leu His Trp Tyr Leu Gln Lys Pro Gly Gln Ser Pro
           195                 200                 205
   Lys Leu Leu Ile Tyr Arg Val Ser Asn Arg Phe Pro Gly Val Pro Asp
       210                 215                 220
   Arg Phe Ser Gly Ser Gly Ser Gly Thr Asp Phe Thr Leu Lys Ile Ser
   225                 230                 235                 240
   Arg Val Glu Ala Glu Asp Leu Gly Ile Tyr Phe Cys Ser Gln Ser Thr
                   245                 250                 255
   His Val Pro Tyr Thr Phe Gly Gly Gly Thr Lys Leu Glu Ile Lys Arg
               260                 265                 270
   Ser Asp Pro Ala Glu Pro Lys Ser Pro Asp Lys Thr His Thr Cys Pro
           275                 280                 285
   Pro Cys Pro Lys Asp Pro Lys Phe Trp Val Leu Val Val Val Gly Gly
       290                 295                 300
   Val Leu Ala Cys Tyr Ser Leu Leu Val Thr Val Ala Phe Ile Ile Phe
   305                 310                 315                 320
   Trp Val Arg Ser Lys Arg Ser Arg Leu Leu His Ser Asp Tyr Met Asn
                   325                 330                 335
   Met Thr Pro Arg Arg Pro Gly Pro Thr Arg Lys His Tyr Gln Pro Tyr
               340                 345                 350
   Ala Pro Pro Arg Asp Phe Ala Ala Tyr Arg Ser Arg Asp Gln Arg Leu
           355                 360                 365
   Pro Pro Asp Ala His Lys Pro Pro Gly Gly Gly Ser Phe Arg Thr Pro
   370 375 380
   Ile Gln Glu Glu Gln Ala Asp Ala His Ser Thr Leu Ala Lys Ile Arg
   385 390 395 400
   Val Lys Phe Ser Arg Ser Ala Asp Ala Pro Ala Tyr Gln Gln Gly Gln
   405 410 415
   Asn Gln Leu Tyr Asn Glu Leu Asn Leu Gly Arg Arg Glu Glu Tyr Asp
   420 425 430
   Val Leu Asp Lys Arg Arg Gly Arg Asp Pro Glu Met Gly Gly Lys Pro
   435 440 445
   Arg Arg Lys Asn Pro Gln Glu Gly Leu Tyr Asn Glu Leu Gln Lys Asp
   450 455 460
   Lys Met Ala Glu Ala Tyr Ser Glu Ile Gly Met Lys Gly Glu Arg Arg
   465 470 475 480
   Arg Gly Lys Gly His Asp Gly Leu Tyr Gln Gly Leu Ser Thr Ala Thr
   485 490 495
   Lys Asp Thr Tyr Asp Ala Leu His Met Gln Ala Leu Pro Pro Arg
   500 505 510
<210> 36
   <211> 15
   <212> PRT
   <213> Artificial Sequence
<220>
   <223> Synthetic peptide for TRBC2
<400> 36
   Val Leu Glu Asp Leu Lys Asn Val Phe Pro Pro Glu Val Ala Val
   1 5 10 15
<210> 37
   <211> 15
   <212> PRT
   <213> Artificial Sequence
<220>
   <223> Synthetic peptide for TRBC1
<400> 37
   Val Leu Glu Asp Leu Asn Lys Val Phe Pro Pro Glu Val Ala Val
   1 5 10 15
<210> 38
   <211> 16
   <212> PRT
   <213> Artificial Sequence
<220>
   <223> Synthetic peptide for TRBC1
<400> 38
   Val Leu Glu Asp Leu Asn Lys Val Phe Pro Pro Glu Val Ala Val Cys
   1               5                   10                  15
<210> 39
   <211> 16
   <212> PRT
   <213> Artificial Sequence
<220>
   <223> Synthetic peptide for TRBC2
<400> 39
   Val Leu Glu Asp Leu Lys Asn Val Phe Pro Pro Glu Val Ala Val Cys
   1               5                   10                  15
<210> 40
   <211> 175
   <212> PRT
   <213> Homo sapiens
<400> 40
   Asp Leu Asn Lys Val Phe Pro Pro Glu Val Ala Val Phe Glu Pro Ser
   1               5                   10                  15
   Glu Ala Glu Ile Ser His Thr Gln Lys Ala Thr Leu Val Cys Leu Ala
               20                  25                  30
   Thr Gly Phe Phe Pro Asp His Val Glu Leu Ser Trp Trp Val Asn Gly
           35                  40                  45
   Lys Glu Val His Ser Gly Val Ser Thr Asp Pro Gln Pro Leu Lys Glu
       50                  55                  60
   Gln Pro Ala Leu Asn Asp Ser Arg Tyr Cys Leu Ser Ser Arg Leu Arg
   65                  70                  75                  80
   Val Ser Ala Thr Phe Trp Gln Asn Pro Arg Asn His Phe Arg Cys Gln
                   85                  90                  95
   Val Gln Phe Tyr Gly Leu Ser Glu Asn Asp Glu Trp Thr Gln Asp Arg
               100                 105                 110
   Ala Lys Pro Val Thr Gln Ile Val Ser Ala Glu Ala Trp Gly Arg Ala
   115 120 125
   Asp Cys Gly Phe Thr Ser Val Ser Tyr Gln Gln Gly Val Leu Ser Ala
   130 135 140
   Thr Ile Leu Tyr Glu Ile Leu Leu Gly Lys Ala Thr Leu Tyr Ala Val
   145 150 155 160
   Leu Val Ser Ala Leu Val Leu Met Ala Met Val Lys Arg Lys Asp
   165 170 175
<210> 41
   <211> 175
   <212> PRT
   <213> Homo sapiens
<400> 41
   Asp Leu Lys Asn Val Phe Pro Pro Glu Val Ala Val Phe Glu Pro Ser
   1 5 10 15
   Glu Ala Glu Ile Ser His Thr Gln Lys Ala Thr Leu Val Cys Leu Ala
   20 25 30
   Thr Gly Phe Tyr Pro Asp His Val Glu Leu Ser Trp Trp Val Asn Gly
   35 40 45
   Lys Glu Val His Ser Gly Val Ser Thr Asp Pro Gln Pro Leu Lys Glu
   50 55 60
   Gln Pro Ala Leu Asn Asp Ser Arg Tyr Cys Leu Ser Ser Arg Leu Arg
   65 70 75 80
   Val Ser Ala Thr Phe Trp Gln Asn Pro Arg Asn His Phe Arg Cys Gln
   85 90 95
   Val Gln Phe Tyr Gly Leu Ser Glu Asn Asp Glu Trp Thr Gln Asp Arg
   100 105 110
   Ala Lys Pro Val Thr Gln Ile Val Ser Ala Glu Ala Trp Gly Arg Ala
   115 120 125
   Asp Cys Gly Phe Thr Ser Glu Ser Tyr Gln Gln Gly Val Leu Ser Ala
   130 135 140
   Thr Ile Leu Tyr Glu Ile Leu Leu Gly Lys Ala Thr Leu Tyr Ala Val
   145 150 155 160
   Leu Val Ser Ala Leu Val Leu Met Ala Met Val Lys Arg Lys Asp
   165 170 175
<210> 42
   <211> 9
   <212> PRT
   <213> Artificial Sequence
<220>
   <223> Heavy CDR3 Clone CP_01_E09
<400> 42
   Ala His Asn Ser Ser Ser Trp Ser Phe
   1 5
<210> 43
   <211> 8
   <212> PRT
   <213> Artificial Sequence
<220>
   <223> Heavy CDR3 Clone CP_01_D12
<400> 43
   Gly Gly Asp Thr Tyr Gly Phe Leu
   1 5
<210> 44
   <211> 8
   <212> PRT
   <213> Artificial Sequence
<220>
   <223> Heavy CDR3 Clone CP_01_D10
<400> 44
   Gly Gly Gly Ser Phe Gly Ala Phe
   1 5
<210> 45
   <211> 8
   <212> PRT
   <213> Artificial Sequence
<220>
   <223> Heavy CDR3 Clone CP_01_C08
<400> 45
   Gly Tyr Ser Ser Ser Trp Tyr Leu
   1 5
<210> 46
   <211> 4
   <212> PRT
   <213> Artificial Sequence
<220>
   <223> Heavy CDR3 Clone CP_01_C11
<400> 46
   Gly Gly Ala Gly
   1
<210> 47
   <211> 9
   <212> PRT
   <213> Artificial Sequence
<220>
   <223> Heavy CDR3 Clone CP_01_F03
<220>
   <221> misc_feature
   <222> (3)..(3)
   <223> Xaa can be any naturally occurring amino acid
<400> 47
   Gly Tyr Xaa Ala Ser Ser Trp Ser Gln
   1 5
<210> 48
   <211> 9
   <212> PRT
   <213> Artificial Sequence
<220>
   <223> Heavy CDR3 Clone CP_01_E07
<400> 48
   Asp Leu Gly Gly Ser Gly Gly Ala Phe
   1 5
<210> 49
   <211> 6
   <212> PRT
   <213> Artificial Sequence
<220>
   <223> Heavy CDR3 Clone CP_01_D03
<400> 49
   Asn Lys Gln Tyr Gly Met
   1 5
<210> 50
   <211> 5
   <212> PRT
   <213> Artificial Sequence
<220>
   <223> Heavy CDR3 Clone CP_01_F06
<400> 50
   Asp Asp Gly Ala Met
   1 5
<210> 51
   <211> 6
   <212> PRT
   <213> Artificial Sequence
<220>
   <223> Heavy CDR3 Clone CP_01_F02
<400> 51
   Ala Gly Tyr Ser Tyr Ala
   1 5
<210> 52
   <211> 9
   <212> PRT
   <213> Artificial Sequence
<220>
   <223> Heavy CDR3 Clone CP_02_C03
<400> 52
   Gly Gly Arg Tyr Ser Ser Asn Tyr Phe
   1 5
<210> 53
   <211> 7
   <212> PRT
   <213> Artificial Sequence
<220>
   <223> Heavy CDR3 Clone CP_02_D10
<400> 53
   Val Gly Glu Gly Ser Ala Met
   1 5
<210> 54
   <211> 15
   <212> PRT
   <213> Artificial Sequence
<220>
   <223> Heavy CDR3 Clone CP_02_B01
<400> 54
   Val Ser Ser His Tyr Asp Ser Ser Gly Tyr Tyr Ala Gly Gly Phe
   1 5 10 15
<210> 55
   <211> 9
   <212> PRT
   <213> Artificial Sequence
<220>
   <223> Heavy CDR3 Clone CP_02_D02
<400> 55
   Gly Arg Asp Ser Ser Ser Trp Ser Pro
   1 5
<210> 56
   <211> 9
   <212> PRT
   <213> Artificial Sequence
<220>
   <223> Heavy CDR3 Clone CP_02_A02
<400> 56
   Val Thr Thr Tyr Ser Gly Leu Asp Phe
   1 5
<210> 57
   <211> 10
   <212> PRT
   <213> Artificial Sequence
<220>
   <223> Heavy CDR3 Clone CP_02_D04
<400> 57
   Lys Gly Ala Val Val Val Pro Gly Ala Leu
   1 5 10
<210> 58
   <211> 9
   <212> PRT
   <213> Artificial Sequence
<220>
   <223> Heavy CDR3 Clone CP_01_E10
<400> 58
   Asn Ser Leu Tyr Gly Gly Asn Ser Ala
   1 5
<210> 59
   <211> 7
   <212> PRT
   <213> Artificial Sequence
<220>
   <223> Heavy CDR3 Clone CP_01_H08
<400> 59
   Asp Gly Gly Gly Gly Arg Phe
   1               5
<210> 60
   <211> 9
   <212> PRT
   <213> Artificial Sequence
<220>
   <223> Heavy CDR3 Clone CP_01_F11
<400> 60
   Gly Gly Gly Ala Leu Gly Arg Gly Met
   1               5
<210> 61
   <211> 11
   <212> PRT
   <213> Artificial Sequence
<220>
   <223> Heavy CDR3 Clone CP_01_F09
<400> 61
   Leu Leu Arg Ser Gly Gly Gln Ser Tyr Ala Phe
   1               5                   10
<210> 62
   <211> 8
   <212> PRT
   <213> Artificial Sequence
<220>
   <223> Heavy CDR3 Clone CP_02_D05
<400> 62
   Gly Tyr Ser Ser Ser Trp Ser Phe
   1               5
<210> 63
   <211> 8
   <212> PRT
   <213> Artificial Sequence
<220>
   <223> Heavy CDR3 Clone CP_02_A09
<400> 63
   Ala Gly Ser Ser Gly Trp Thr Leu
   1 5
<210> 64
   <211> 6
   <212> PRT
   <213> Artificial Sequence
<220>
   <223> Heavy CDR3 Clone CP_02_D03
<400> 64
   Asp Lys Gly Trp Gly Phe
   1 5
<210> 65
   <211> 11
   <212> PRT
   <213> Artificial Sequence
<220>
   <223> Heavy CDR3 Clone CP_02_C11
<400> 65
   Leu Gly Val Val Arg Gly Val Met Lys Gly Phe
   1 5 10
<210> 66
   <211> 9
   <212> PRT
   <213> Artificial Sequence
<220>
   <223> Heavy CDR3 Clone CP_01_H10
<400> 66
   Ser Ser Tyr Ser Ser Ser Trp Gly Met
   1 5
<210> 67
   <211> 9
   <212> PRT
   <213> Artificial Sequence
<220>
   <223> Heavy CDR3 Clone CP_02_C04
<400> 67
   Ala Asn Ser Trp Ser Ala Gly Gly Met
   1 5
<210> 68
   <211> 9
   <212> PRT
   <213> Artificial Sequence
<220>
   <223> Heavy CDR3 Clone CP_01_G03
<400> 68
   Glu Arg Gly Arg Gly Tyr Ser Tyr Met
   1 5
<210> 69
   <211> 9
   <212> PRT
   <213> Artificial Sequence
<220>
   <223> Heavy CDR3 Clone CP_01_G06
<400> 69
   Val Ala Arg Gly Ile His Asp Ala Phe
   1 5
<210> 70
   <211> 4
   <212> PRT
   <213> Artificial Sequence
<220>
   <223> Heavy CDR3 Clone CP_01_D06
<400> 70
   Arg His Gly Met
   1
<210> 71
   <211> 8
   <212> PRT
   <213> Artificial Sequence
<220>
   <223> Heavy CDR3 Clone CP_02_B03
<400> 71
   Phe Asp Ser Ser Gly Tyr Tyr Tyr
   1 5
<210> 72
   <211> 8
   <212> PRT
   <213> Artificial Sequence
<220>
   <223> Heavy CDR3 Clone CP_02_A12
<400> 72
   Asp Leu Val Thr Thr Gly Ala Phe
   1 5
<210> 73
   <211> 12
   <212> PRT
   <213> Artificial Sequence
<220>
   <223> Heavy CDR3 Clone CP_01_H03
<400> 73
   Ala Ile Arg Val Ser Gly Thr Pro Glu Asn Gly Phe
   1 5 10
<210> 74
   <211> 7
   <212> PRT
   <213> Artificial Sequence
<220>
   <223> Heavy CDR3 Clone CP_01_G08
<400> 74
   Val Arg Ile Thr His Gly Met
   1 5
<210> 75
   <211> 5
   <212> PRT
   <213> Artificial Sequence
<220>
   <223> Heavy CDR3 Clone CP_01_A06
<400> 75
   Gly Lys Leu Ala Phe
   1 5
<210> 76
   <211> 15
   <212> PRT
   <213> Artificial Sequence
<220>
   <223> Heavy CDR3 Clone CP_02_A04
<400> 76
   Asn Gly Asp Ser Ser Gly Tyr His Thr Ser Pro Asn Trp Tyr Phe
   1 5 10 15
<210> 77
   <211> 8
   <212> PRT
   <213> Artificial Sequence
<220>
   <223> Heavy CDR3 Clone CP_01_E08
<400> 77
   Val Ser Thr Asp Ser Ser Ser Met
   1 5
<210> 78
   <211> 8
   <212> PRT
   <213> Artificial Sequence
<220>
   <223> Heavy CDR3 Clone CP_01_A08
<400> 78
   Thr Ser Gln Asp Pro Gly Ala Phe
   1 5
<210> 79
   <211> 11
   <212> PRT
   <213> Artificial Sequence
<220>
   <223> Heavy CDR3 Clone CP_01_D01
<400> 79
   Ala Glu Ser Gly Val Tyr Ser Ser Asn Gly Met
   1 5 10
<210> 80
   <211> 8
   <212> PRT
   <213> Artificial Sequence
<220>
   <223> Heavy CDR3 Clone CP_02_A07
<400> 80
   Val Asp Arg Val Arg Ser Gly Met
   1 5
<210> 81
   <211> 12
   <212> PRT
   <213> Artificial Sequence
<220>
   <223> Heavy CDR3 Clone CP_02_B08
<400> 81
   Ile Gly Gln Tyr Cys Ser Ser Thr Ser Cys Tyr Met
   1 5 10
<210> 82
   <211> 9
   <212> PRT
   <213> Artificial Sequence
<220>
   <223> Heavy CDR3 Clone CP_01_D09
<400> 82
   Asp Leu Gly Gly Ser Gly Gly Ala Phe
   1 5
<210> 83
   <211> 7
   <212> PRT
   <213> Artificial Sequence
<220>
   <223> Heavy CDR3 Clone CP_01_G07
<400> 83
   Asp Ser Asp Ala Gly Tyr Phe
   1 5
<210> 84
   <211> 9
   <212> PRT
   <213> Artificial Sequence
<220>
   <223> Heavy CDR3 Clone CP_01_A05
<400> 84
   Ala Ser Ile Val Ala Ser Gly Ala Phe
   1 5
<210> 85
   <211> 7
   <212> PRT
   <213> Artificial Sequence
<220>
   <223> Heavy CDR3 Clone CP_02_A08
<400> 85
   Ala Gly Gly Ser Asn Ala Phe
   1 5
<210> 86
   <211> 12
   <212> PRT
   <213> Artificial Sequence
<220>
   <223> Heavy CDR3 Clone CP_02_D07
<400> 86
   Val Ser Thr Asp Ser Tyr Gly Arg Gln Asn Trp Phe
   1 5 10
<210> 87
   <211> 14
   <212> PRT
   <213> Artificial Sequence
<220>
   <223> Heavy CDR3 Clone CP_01_C04
<400> 87
   Gln Tyr Thr Ser Gly Arg Leu Ala Tyr Tyr Tyr His Tyr Met
   1 5 10
<210> 88
   <211> 6
   <212> PRT
   <213> Artificial Sequence
<220>
   <223> Heavy CDR3 Clone CP_01_A07
<400> 88
   Gly Ile Arg Gly Ala Phe
   1 5
<210> 89
   <211> 8
   <212> PRT
   <213> Artificial Sequence
<220>
   <223> Heavy CDR3 Clone CP_01_H02
<400> 89
   Val Gly Tyr Ser Thr Thr Gln Leu
   1 5
<210> 90
   <211> 8
   <212> PRT
   <213> Artificial Sequence
<220>
   <223> Heavy CDR3 Clone CP_01_F10
<400> 90
   Met Ala Gly Ser Tyr Tyr Ala Phe
   1 5
<210> 91
   <211> 15
   <212> PRT
   <213> Artificial Sequence
<220>
   <223> Heavy CDR3 Clone CP_02_C10
<400> 91
   Val Gly Asp Tyr Tyr Asp Ser Ser Gly Tyr Leu Asp Trp Tyr Phe
   1 5 10 15
<210> 92
   <211> 9
   <212> PRT
   <213> Artificial Sequence
<220>
   <223> Heavy CDR3 Clone CP_02_B05
<400> 92
   Gly Ser Asp Thr Thr Ser Phe Val Ser
   1 5
<210> 93
   <211> 8
   <212> PRT
   <213> Artificial Sequence
<220>
   <223> Heavy CDR3 Clone CP_01_G04
<400> 93
   Ala Gly His Tyr Tyr Tyr Tyr Met
   1 5
<210> 94
   <211> 13
   <212> PRT
   <213> Artificial Sequence
<220>
   <223> Heavy CDR3 Clone CP_01_F08
<400> 94
   Val Thr Gly Tyr Pro Asp Tyr Tyr Asp Ser Ser Gly Phe
   1 5 10
<210> 95
   <211> 9
   <212> PRT
   <213> Artificial Sequence
<220>
   <223> Heavy CDR3 Clone CP_01_G05
<400> 95
   Val Glu Gly Gly Pro Pro Tyr Tyr Phe
   1 5
<210> 96
   <211> 8
   <212> PRT
   <213> Artificial Sequence
<220>
   <223> Heavy CDR3 Clone CP_02_A03
<400> 96
   Asn Gly Leu Asp Asn Tyr Gly Met
   1 5
<210> 97
   <211> 8
   <212> PRT
   <213> Artificial Sequence
<220>
   <223> Heavy CDR3 Clone CP_01_B09
<400> 97
   Leu Gly Thr Thr Lys Arg Ala Phe
   1 5
<210> 98
   <211> 8
   <212> PRT
   <213> Artificial Sequence
<220>
   <223> Heavy CDR3 Clone CP_01_A10
<400> 98
   Val Tyr Val Asp His Glu Gly Met
   1 5
<210> 99
   <211> 6
   <212> PRT
   <213> Artificial Sequence
<220>
   <223> Heavy CDR3 Clone CP_01_H04
<400> 99
   Trp Ser Gly Ser Gly Phe
   1 5
<210> 100
   <211> 8
   <212> PRT
   <213> Artificial Sequence
<220>
   <223> Heavy CDR3 Clone CP_02_B04
<400> 100
   Asp Phe Gly Trp Gly Gly Ala Phe
   1 5
<210> 101
   <211> 7
   <212> PRT
   <213> Artificial Sequence
<220>
   <223> Heavy CDR3 Clone CP_02_A05
<400> 101
   Val Val Gly Gly Thr Gln His
   1 5
<210> 102
   <211> 14
   <212> PRT
   <213> Artificial Sequence
<220>
   <223> Heavy CDR3 Clone CP_01_F07
<400> 102
   Asn Trp Leu Leu Tyr Tyr Gly Asp Pro Gln Gln Asn Ala Phe
   1 5 10
<210> 103
   <211> 13
   <212> PRT
   <213> Artificial Sequence
<220>
   <223> Heavy CDR3 Clone CP_01_H01
<400> 103
   Leu Tyr Phe Asp Trp Phe Ala Asp Ser Gln Asn Ala Phe
   1 5 10
<210> 104
   <211> 13
   <212> PRT
   <213> Artificial Sequence
<220>
   <223> Heavy CDR3 Clone CP_01_G10
<400> 104
   Val Gly Tyr Gln Pro Leu Leu Tyr Ala Asp Tyr Tyr Phe
   1 5 10
<210> 105
   <211> 5
   <212> PRT
   <213> Artificial Sequence
<220>
   <223> Heavy CDR3 Clone CP_01_G11
<400> 105
   Gly Ala Met Gly Leu
   1 5
<210> 106
   <211> 10
   <212> PRT
   <213> Artificial Sequence
<220>
   <223> Heavy CDR3 Clone CP_01_G01
<400> 106
   Val Tyr Tyr Leu Ser Gly Val His Ala Phe
   1 5 10
<210> 107
   <211> 7
   <212> PRT
   <213> Artificial Sequence
<220>
   <223> Heavy CDR3 Clone CP_01_A12
<400> 107
   Thr Glu Arg Trp Leu Gln Phe
   1 5
<210> 108
   <211> 6
   <212> PRT
   <213> Artificial Sequence
<220>
   <223> Heavy CDR3 Clone CP_01_H05
<400> 108
   Asn Gly Asp Tyr Ala Phe
   1 5
<210> 109
   <211> 10
   <212> PRT
   <213> Artificial Sequence
<220>
   <223> Heavy CDR3 Clone CP_02_B07
<400> 109
   Ala Ser Arg Tyr Ser Gly Ser Tyr His Phe
   1 5 10
<210> 110
   <211> 7
   <212> PRT
   <213> Artificial Sequence
<220>
   <223> Heavy CDR3 Clone CP_01_G09
<400> 110
   His Gly Ser Gln Gly Gly Phe
   1 5
<210> 111
   <211> 7
   <212> PRT
   <213> Artificial Sequence
<220>
   <223> Heavy CDR3 Clone CP_02_C02
<400> 111
   Val Gly Tyr Met Gly Gly Met
   1 5
<210> 112
   <211> 10
   <212> PRT
   <213> Artificial Sequence
<220>
   <223> Heavy CDR3 Clone CP_01_D05
<400> 112
   Asn Thr Pro Gly Ile Ala Ala Ala Gly Pro
   1 5 10
<210> 113
   <211> 9
   <212> PRT
   <213> Artificial Sequence
<220>
   <223> Heavy CDR3 Clone CP_01_D08
<400> 113
   Val Gly Thr Thr Thr Val Thr Ser Phe
   1 5
<210> 114
   <211> 9
   <212> PRT
   <213> Artificial Sequence
<220>
   <223> Heavy CDR3 Clone CP_02_A11
<400> 114
   Val Gly Gly Pro Leu Asn Asp Ala Phe
   1 5
<210> 115
   <211> 7
   <212> PRT
   <213> Artificial Sequence
<220>
   <223> Heavy CDR3 Clone CP_02_D08
<400> 115
   His Ser Ser Gly Gly Ala Phe
   1 5
<210> 116
   <211> 7
   <212> PRT
   <213> Artificial Sequence
<220>
   <223> Light CDR3 Clone CP_01_E09
<400> 116
   Gln Gln Tyr Asp Asn Leu Pro
   1               5
<210> 117
   <211> 7
   <212> PRT
   <213> Artificial Sequence
<220>
   <223> Light CDR3 Clone CP_01_D12
<400> 117
   Gln Gln Phe Asn Ala Tyr Pro
   1               5
<210> 118
   <211> 7
   <212> PRT
   <213> Artificial Sequence
<220>
   <223> Light CDR3 Clone CP_01_D10
<400> 118
   Gln Gln Tyr Asn Ser Tyr Pro
   1               5
<210> 119
   <211> 7
   <212> PRT
   <213> Artificial Sequence
<220>
   <223> Light CDR3 Clone CP_01_C08
<400> 119
   Gln Gln Tyr Asp Asn Leu Pro
   1               5
<210> 120
   <211> 7
   <212> PRT
   <213> Artificial Sequence
<220>
   <223> Light CDR3 Clone CP_01_C11
<400> 120
   Gln Ser His Asp Ser Ser Asn
   1               5
<210> 121
   <211> 7
   <212> PRT
   <213> Artificial Sequence
<220>
   <223> Light CDR3 Clone CP_01_F03
<400> 121
   Gln Gln Tyr Asp Asn Leu Pro
   1               5
<210> 122
   <211> 6
   <212> PRT
   <213> Artificial Sequence
<220>
   <223> Light CDR3 Clone CP_01_E07
<400> 122
   Met Gln Ser Ile Gln Leu
   1               5
<210> 123
   <211> 7
   <212> PRT
   <213> Artificial Sequence
<220>
   <223> Light CDR3 Clone CP_01_D03
<400> 123
   Gln Gln Tyr His Arg Trp Pro
   1               5
<210> 124
   <211> 7
   <212> PRT
   <213> Artificial Sequence
<220>
   <223> Light CDR3 Clone CP_01_F06
<400> 124
   Gln Gln Tyr Tyr Asp Ser Pro
   1 5
<210> 125
   <211> 7
   <212> PRT
   <213> Artificial Sequence
<220>
   <223> Light CDR3 Clone CP_01_F02
<400> 125
   Leu Gln His Asn Ser Tyr Pro
   1 5
<210> 126
   <211> 6
   <212> PRT
   <213> Artificial Sequence
<220>
   <223> Light CDR3 Clone CP_02_C03
<400> 126
   Gln Gln Tyr Phe Gly Thr
   1 5
<210> 127
   <211> 7
   <212> PRT
   <213> Artificial Sequence
<220>
   <223> Light CDR3 Clone CP_02_D10
<400> 127
   Gln Gln Tyr Asn Asp Trp Pro
   1 5
<210> 128
   <211> 8
   <212> PRT
   <213> Artificial Sequence
<220>
   <223> Light CDR3 Clone CP_02_B01
<400> 128
   Gln Ser Phe Asp Thr Asn Ser Leu
   1 5
<210> 129
   <211> 7
   <212> PRT
   <213> Artificial Sequence
<220>
   <223> Light CDR3 Clone CP_02_D02
<400> 129
   Gln Gln Tyr Asp Asn Leu Pro
   1 5
<210> 130
   <211> 8
   <212> PRT
   <213> Artificial Sequence
<220>
   <223> Light CDR3 Clone CP_02_A02
<400> 130
   Ser Ser Tyr Thr Ser Ser Ser Thr
   1 5
<210> 131
   <211> 7
   <212> PRT
   <213> Artificial Sequence
<220>
   <223> Light CDR3 Clone CP_02_D04
<400> 131
   Gln Gln Tyr Asn Ser Tyr Pro
   1 5
<210> 132
   <211> 7
   <212> PRT
   <213> Artificial Sequence
<220>
   <223> Light CDR3 Clone CP_01_E10
<400> 132
   Gln Gln Thr Phe Thr Thr Pro
   1 5
<210> 133
   <211> 7
   <212> PRT
   <213> Artificial Sequence
<220>
   <223> Light CDR3 Clone CP_01_H08
<400> 133
   Gln Gln Tyr Asp Asn Leu Pro
   1 5
<210> 134
   <211> 7
   <212> PRT
   <213> Artificial Sequence
<220>
   <223> Light CDR3 Clone CP_01_F11
<400> 134
   Gln Ser Tyr Asp Thr Asn Asn
   1 5
<210> 135
   <211> 7
   <212> PRT
   <213> Artificial Sequence
<220>
   <223> Light CDR3 Clone CP_01_F09
<400> 135
   Gln Gln Ser Tyr Ser Thr Pro
   1 5
<210> 136
   <211> 7
   <212> PRT
   <213> Artificial Sequence
<220>
   <223> Light CDR3 Clone CP_02_D05
<400> 136
   Gln Gln Tyr Asp Asn Leu Pro
   1 5
<210> 137
   <211> 7
   <212> PRT
   <213> Artificial Sequence
<220>
   <223> Light CDR3 Clone CP_02_A09
<400> 137
   Gln Gln Tyr Asp Asn Leu Pro
   1 5
<210> 138
   <211> 7
   <212> PRT
   <213> Artificial Sequence
<220>
   <223> Light CDR3 Clone CP_02_D03
<400> 138
   Gln Gln Tyr Asp Asn Leu Pro
   1 5
<210> 139
   <211> 7
   <212> PRT
   <213> Artificial Sequence
<220>
   <223> Light CDR3 Clone CP_02_C11
<400> 139
   Gln Ser Tyr Asp Ser Ser Asn
   1 5
<210> 140
   <211> 7
   <212> PRT
   <213> Artificial Sequence
<220>
   <223> Light CDR3 Clone CP_01_H10
<400> 140
   Gln Gln Tyr Asp Asn Leu Pro
   1 5
<210> 141
   <211> 7
   <212> PRT
   <213> Artificial Sequence
<220>
   <223> Light CDR3 Clone CP_02_C04
<400> 141
   Gln Gln Tyr Asp Asp Leu Pro
   1 5
<210> 142
   <211> 7
   <212> PRT
   <213> Artificial Sequence
<220>
   <223> Light CDR3 Clone CP_01_G03
<400> 142
   Met Gln Arg Ile Glu Phe Pro
   1 5
<210> 143
   <211> 8
   <212> PRT
   <213> Artificial Sequence
<220>
   <223> Light CDR3 Clone CP_01_G06
<400> 143
   Gln Ser Tyr Asp Asn Thr Arg His
   1 5
<210> 144
   <211> 7
   <212> PRT
   <213> Artificial Sequence
<220>
   <223> Light CDR3 Clone CP_01_D06
<400> 144
   Gln Ser Tyr Asp Ser Ser Asn
   1 5
<210> 145
   <211> 7
   <212> PRT
   <213> Artificial Sequence
<220>
   <223> Light CDR3 Clone CP_02_B03
<400> 145
   Gln Ser Tyr Asp Ser Ser Asn
   1 5
<210> 146
   <211> 7
   <212> PRT
   <213> Artificial Sequence
<220>
   <223> Light CDR3 Clone CP_02_A12
<400> 146
   Gln Gln His Asn Asp Trp Pro
   1               5
<210> 147
   <211> 8
   <212> PRT
   <213> Artificial Sequence
<220>
   <223> Light CDR3 Clone CP_01_H03
<400> 147
   Gln Ser Tyr His Ser Ser Asn Leu
   1               5
<210> 148
   <211> 7
   <212> PRT
   <213> Artificial Sequence
<220>
   <223> Light CDR3 Clone CP_01_G08
<400> 148
   Met Gln Ala Thr His Phe Pro
   1               5
<210> 149
   <211> 7
   <212> PRT
   <213> Artificial Sequence
<220>
   <223> Light CDR3 Clone CP_01_A06
<400> 149
   Gln Gln Tyr Tyr Ser Thr Pro
   1               5
<210> 150
   <211> 8
   <212> PRT
   <213> Artificial Sequence
<220>
   <223> Light CDR3 Clone CP_02_A04
<400> 150
   Gln Ser Tyr Asp Asp Ser Asn Tyr
   1               5
<210> 151
   <211> 9
   <212> PRT
   <213> Artificial Sequence
<220>
   <223> Light CDR3 Clone CP_01_E08
<400> 151
   Ser Ser Tyr Ala Gly Ser Asn Thr Leu
   1 5
<210> 152
   <211> 7
   <212> PRT
   <213> Artificial Sequence
<220>
   <223> Light CDR3 Clone CP_01_A08
<400> 152
   Gln Gln Ala Asn Ser Phe Pro
   1 5
<210> 153
   <211> 7
   <212> PRT
   <213> Artificial Sequence
<220>
   <223> Light CDR3 Clone CP_01_D01
<400> 153
   Gln Ser Tyr Asp Ser Ser Ile
   1 5
<210> 154
   <211> 7
   <212> PRT
   <213> Artificial Sequence
<220>
   <223> Light CDR3 Clone CP_02_A07
<400> 154
   Gln Ser Tyr Asp Ser Ile His
   1 5
<210> 155
   <211> 8
   <212> PRT
   <213> Artificial Sequence
<220>
   <223> Light CDR3 Clone CP_02_B08
<400> 155
   Gln Ser Tyr Asp Ser Ser Thr His
   1 5
<210> 156
   <211> 7
   <212> PRT
   <213> Artificial Sequence
<220>
   <223> Light CDR3 Clone CP_01_D09
<400> 156
   Gln Gln Tyr Asp Asn Leu Pro
   1 5
<210> 157
   <211> 8
   <212> PRT
   <213> Artificial Sequence
<220>
   <223> Light CDR3 Clone CP_01_G07
<400> 157
   Gln Ser Phe Thr Ser Ser Thr Leu
   1 5
<210> 158
   <211> 7
   <212> PRT
   <213> Artificial Sequence
<220>
   <223> Light CDR3 Clone CP_01_A05
<400> 158
   Gln Gln Tyr Asn Lys Trp Pro
   1 5
<210> 159
   <211> 8
   <212> PRT
   <213> Artificial Sequence
<220>
   <223> Light CDR3 Clone CP_02_A08
<400> 159
   Gln Ser Tyr Asp Asp Ser Asn Tyr
   1 5
<210> 160
   <211> 8
   <212> PRT
   <213> Artificial Sequence
<220>
   <223> Light CDR3 Clone CP_02_D07
<400> 160
   Gln Ser Tyr Asp Ser Ser Asn His
   1 5
<210> 161
   <211> 7
   <212> PRT
   <213> Artificial Sequence
<220>
   <223> Light CDR3 Clone CP_01_C04
<400> 161
   Gln Gln Ser Tyr Ser Thr Pro
   1 5
<210> 162
   <211> 10
   <212> PRT
   <213> Artificial Sequence
<220>
   <223> Light CDR3 Clone CP_01_A07
<400> 162
   Asn Ser Arg Asp Ser Ser Gly Asn Pro Asn
   1 5 10
<210> 163
   <211> 8
   <212> PRT
   <213> Artificial Sequence
<220>
   <223> Light CDR3 Clone CP_01_H02
<400> 163
   Gln Ser Tyr Asp Ser Ser Asn Leu
   1 5
<210> 164
   <211> 8
   <212> PRT
   <213> Artificial Sequence
<220>
   <223> Light CDR3 Clone CP_01_F10
<400> 164
   Gln Ser Tyr Asp Ser Ser Asn His
   1 5
<210> 165
   <211> 9
   <212> PRT
   <213> Artificial Sequence
<220>
   <223> Light CDR3 Clone CP_02_C10
<400> 165
   Ala Val Trp Asp Asp Arg Leu Asn Gly
   1 5
<210> 166
   <211> 7
   <212> PRT
   <213> Artificial Sequence
<220>
   <223> Light CDR3 Clone CP_02_B05
<400> 166
   Gln Gln Tyr Asp Ser Tyr Ser
   1 5
<210> 167
   <211> 9
   <212> PRT
   <213> Artificial Sequence
<220>
   <223> Light CDR3 Clone CP_01_G04
<400> 167
   Gln Ser Ala Asp Ser Ser Gly Thr Asn
   1 5
<210> 168
   <211> 8
   <212> PRT
   <213> Artificial Sequence
<220>
   <223> Light CDR3 Clone CP_01_F08
<400> 168
   Gln Ser Tyr Asp Ser Ser Asn His
   1 5
<210> 169
   <211> 8
   <212> PRT
   <213> Artificial Sequence
<220>
   <223> Light CDR3 Clone CP_01_G05
<400> 169
   Gln Ser Tyr Asp Thr Arg Asn Gln
   1 5
<210> 170
   <211> 7
   <212> PRT
   <213> Artificial Sequence
<220>
   <223> Light CDR3 Clone CP_02_A03
<400> 170
   Gln Gln Tyr Tyr Ser Thr Pro
   1 5
<210> 171
   <211> 6
   <212> PRT
   <213> Artificial Sequence
<220>
   <223> Light CDR3 Clone CP_01_B09
<400> 171
   Gln Gln Ser Tyr Ser Thr
   1 5
<210> 172
   <211> 8
   <212> PRT
   <213> Artificial Sequence
<220>
   <223> Light CDR3 Clone CP_01_A10
<400> 172
   Ala Ala Trp Asp Asp Ser Leu Phe
   1 5
<210> 173
   <211> 7
   <212> PRT
   <213> Artificial Sequence
<220>
   <223> Light CDR3 Clone CP_01_H04
<400> 173
   Met Gln Arg Ile Glu Phe Pro
   1 5
<210> 174
   <211> 7
   <212> PRT
   <213> Artificial Sequence
<220>
   <223> Light CDR3 Clone CP_02_B04
<400> 174
   Gln Gln Tyr Tyr Asn Thr Pro
   1 5
<210> 175
   <211> 7
   <212> PRT
   <213> Artificial Sequence
<220>
   <223> Light CDR3 Clone CP_02_A05
<400> 175
   Gln Ser Tyr Asp Ser Ser Ile
   1 5
<210> 176
   <211> 8
   <212> PRT
   <213> Artificial Sequence
<220>
   <223> Light CDR3 Clone CP_01_F07
<400> 176
   Gln Ser Tyr Asp Ser Thr Asn Leu
   1 5
<210> 177
   <211> 7
   <212> PRT
   <213> Artificial Sequence
<220>
   <223> Light CDR3 Clone CP_01_H01
<400> 177
   Gln Gln Ser Tyr Ser Thr Pro
   1 5
<210> 178
   <211> 9
   <212> PRT
   <213> Artificial Sequence
<220>
   <223> Light CDR3 Clone CP_01_G10
<400> 178
   Asn Ser Arg Asp Ser Ser Gly Asn His
   1 5
<210> 179
   <211> 7
   <212> PRT
   <213> Artificial Sequence
<220>
   <223> Light CDR3 Clone CP_01_G11
<400> 179
   Gln Gln Ser Tyr Ser Thr Pro
   1 5
<210> 180
   <211> 9
   <212> PRT
   <213> Artificial Sequence
<220>
   <223> Light CDR3 Clone CP_01_G01
<220>
   <221> misc_feature
   <222> (9)..(9)
   <223> Xaa can be any naturally occurring amino acid
<400> 180
   Asp Ser Arg Asp Thr Arg Val Asn Xaa
   1 5
<210> 181
   <211> 6
   <212> PRT
   <213> Artificial Sequence
<220>
   <223> Light CDR3 Clone CP_01_A12
<400> 181
   Gln Gln Tyr Asp Asn Leu
   1 5
<210> 182
   <211> 7
   <212> PRT
   <213> Artificial Sequence
<220>
   <223> Light CDR3 Clone CP_01_H05
<400> 182
   Met Gln Ala Leu Gln Thr Pro
   1 5
<210> 183
   <211> 7
   <212> PRT
   <213> Artificial Sequence
<220>
   <223> Light CDR3 Clone CP_02_B07
<400> 183
   Gln Ser Tyr Asp Ser Ser Asn
   1 5
<210> 184
   <211> 8
   <212> PRT
   <213> Artificial Sequence
<220>
   <223> Light CDR3 Clone CP_01_G09
<400> 184
   Ser Ser Tyr Thr Ser Ser Ser Thr
   1 5
<210> 185
   <211> 8
   <212> PRT
   <213> Artificial Sequence
<220>
   <223> Light CDR3 Clone CP_02_C02
<400> 185
   Met Gln Ala Leu Gln Thr Pro Pro
   1 5
<210> 186
   <211> 8
   <212> PRT
   <213> Artificial Sequence
<220>
   <223> Light CDR3 Clone CP_01_D05
<400> 186
   Gln Ser Tyr Asp Ser Thr Asn His
   1 5
<210> 187
   <211> 8
   <212> PRT
   <213> Artificial Sequence
<220>
   <223> Light CDR3 Clone CP_01_D08
<400> 187
   Gln Ser Tyr Asp Ser Ala Asn Leu
   1 5
<210> 188
   <211> 8
   <212> PRT
   <213> Artificial Sequence
<220>
   <223> Light CDR3 Clone CP_02_A11
<400> 188
   Gln Ser Phe Asp Glu Asn Ile Ser
   1 5
<210> 189
   <211> 7
   <212> PRT
   <213> Artificial Sequence
<220>
   <223> Light CDR3 Clone CP_02_D08
<400> 189
   His Gln Ser Ala Thr Ser Pro
   1 5
<210> 190
   <211> 7
   <212> PRT
   <213> Artificial Sequence
<220>
   <223> Heavy CDR3 Clone CP_03_E05
<400> 190
   Thr Arg Ser Ser Gly Ala Phe
   1 5
<210> 191
   <211> 8
   <212> PRT
   <213> Artificial Sequence
<220>
   <223> Heavy CDR3 Clone CP_03_D05
<400> 191
   Pro Arg Gly Arg Gly Ser Ala Phe
   1 5
<210> 192
   <211> 6
   <212> PRT
   <213> Artificial Sequence
<220>
   <223> Heavy CDR3 Clone CP_03_H06
<400> 192
   Ala Arg Val Gly Gly Met
   1 5
<210> 193
   <211> 5
   <212> PRT
   <213> Artificial Sequence
<220>
   <223> Heavy CDR3 Clone CP_03_C12
<400> 193
   Asp Thr Gly Pro Ile
   1 5
<210> 194
   <211> 11
   <212> PRT
   <213> Artificial Sequence
<220>
   <223> Heavy CDR3 Clone CP_03_G02
<400> 194
   Gly Val Trp Asn Ser Gly Ser Tyr Leu Gly Phe
   1 5 10
<210> 195
   <211> 10
   <212> PRT
   <213> Artificial Sequence
<220>
   <223> Heavy CDR3 Clone CP_03_D04
<400> 195
   Gly Gly Phe Thr Val Pro Gly Gly Ala Phe
   1 5 10
<210> 196
   <211> 7
   <212> PRT
   <213> Artificial Sequence
<220>
   <223> Heavy CDR3 Clone CP_03_F10
<400> 196
   Phe Gly Glu Arg Tyr Ala Phe
   1 5
<210> 197
   <211> 12
   <212> PRT
   <213> Artificial Sequence
<220>
   <223> Heavy CDR3 Clone CP_03_G09
<400> 197
   Asp Gln Trp Leu Ala Asn Tyr Tyr Tyr Tyr Gly Met
   1 5 10
<210> 198
   <211> 11
   <212> PRT
   <213> Artificial Sequence
<220>
   <223> Heavy CDR3 Clone CP_03_F09
<400> 198
   Asn Arg Gly Gly Ser Tyr Lys Ser Val Gly Met
   1 5 10
<210> 199
   <211> 7
   <212> PRT
   <213> Artificial Sequence
<220>
   <223> Heavy CDR3 Clone CP_03_D09
<400> 199
   Val Ser Ser Tyr Tyr Gly Met
   1               5
<210> 200
   <211> 7
   <212> PRT
   <213> Artificial Sequence
<220>
   <223> Heavy CDR3 Clone CP_03_F02
<400> 200
   Ala Pro Ala Ser Ser Ala His
   1               5
<210> 201
   <211> 8
   <212> PRT
   <213> Artificial Sequence
<220>
   <223> Heavy CDR3 Clone CP_02_E03
<400> 201
   Gln Arg Gly Tyr Tyr Tyr Gly Met
   1               5
<210> 202
   <211> 8
   <212> PRT
   <213> Artificial Sequence
<220>
   <223> Heavy CDR3 Clone CP_03_H07
<400> 202
   Ser Ser Val Ala Ala Gly Ala Phe
   1               5
<210> 203
   <211> 8
   <212> PRT
   <213> Artificial Sequence
<220>
   <223> Heavy CDR3 Clone CP_03_C02
<400> 203
   Leu Ser Gly Arg Gly Leu Gly Phe
   1 5
<210> 204
   <211> 4
   <212> PRT
   <213> Artificial Sequence
<220>
   <223> Heavy CDR3 Clone CP_03_E09
<400> 204
   Asp His Tyr Phe
   1
<210> 205
   <211> 8
   <212> PRT
   <213> Artificial Sequence
<220>
   <223> Heavy CDR3 Clone CP_03_D08
<400> 205
   Ser Gly Arg Arg Val Thr Ala Ile
   1 5
<210> 206
   <211> 8
   <212> PRT
   <213> Artificial Sequence
<220>
   <223> Heavy CDR3 Clone CP_03_E11
<400> 206
   Met Gly Arg Tyr Ser Ser Ser Trp
   1 5
<210> 207
   <211> 8
   <212> PRT
   <213> Artificial Sequence
<220>
   <223> Heavy CDR3 Clone CP_03_B05
<400> 207
   His Ser Arg Phe Gly Pro Ala Phe
   1 5
<210> 208
   <211> 5
   <212> PRT
   <213> Artificial Sequence
<220>
   <223> Heavy CDR3 Clone CP_03_H02
<400> 208
   Asp Arg Glu Ala Phe
   1               5
<210> 209
   <211> 13
   <212> PRT
   <213> Artificial Sequence
<220>
   <223> Heavy CDR3 Clone CP_03_D02
<400> 209
   Leu Arg Gly Arg Tyr Ser Tyr Gly Tyr Ser Asp Ala Phe
   1               5                   10
<210> 210
   <211> 9
   <212> PRT
   <213> Artificial Sequence
<220>
   <223> Heavy CDR3 Clone CP_03_E01
<400> 210
   Leu Leu Asn Ala Val Thr Tyr Ala Phe
   1               5
<210> 211
   <211> 7
   <212> PRT
   <213> Artificial Sequence
<220>
   <223> Heavy CDR3 Clone CP_03_C11
<400> 211
   Ile Gly Val Ile Gly Gly Phe
   1               5
<210> 212
   <211> 10
   <212> PRT
   <213> Artificial Sequence
<220>
   <223> Heavy CDR3 Clone CP_03_B12
<400> 212
   Ile Glu Tyr Ser Ser Ser Ser Pro Tyr Phe
   1 5 10
<210> 213
   <211> 13
   <212> PRT
   <213> Artificial Sequence
<220>
   <223> Heavy CDR3 Clone CP_03_E03
<400> 213
   Asp Leu Leu Pro Thr Thr Val Thr Thr Thr Gly Ala Phe
   1 5 10
<210> 214
   <211> 6
   <212> PRT
   <213> Artificial Sequence
<220>
   <223> Heavy CDR3 Clone CP_03_F01
<400> 214
   Asp Ser Gly Ser Tyr Ser
   1 5
<210> 215
   <211> 12
   <212> PRT
   <213> Artificial Sequence
<220>
   <223> Heavy CDR3 Clone CP_03_C01
<400> 215
   Ala Ser Tyr Pro Tyr Tyr Tyr Tyr Tyr Tyr Gly Met
   1 5 10
<210> 216
   <211> 5
   <212> PRT
   <213> Artificial Sequence
<220>
   <223> Heavy CDR3 Clone CP_03_G07
<400> 216
   Ala Leu Gly His Phe
   1 5
<210> 217
   <211> 7
   <212> PRT
   <213> Artificial Sequence
<220>
   <223> Heavy CDR3 Clone CP_03_E02
<400> 217
   Phe Thr Thr Gly Ser Ala Leu
   1 5
<210> 218
   <211> 5
   <212> PRT
   <213> Artificial Sequence
<220>
   <223> Heavy CDR3 Clone CP_03_C07
<400> 218
   Asp Ala Ser Gly Tyr
   1 5
<210> 219
   <211> 11
   <212> PRT
   <213> Artificial Sequence
<220>
   <223> Heavy CDR3 Clone CP_03_H04
<400> 219
   Asp Leu Gly Thr Tyr Tyr Tyr Gly Ser Gly Asp
   1 5 10
<210> 220
   <211> 8
   <212> PRT
   <213> Artificial Sequence
<220>
   <223> Heavy CDR3 Clone CP_03_E06
<400> 220
   Val Gly Glu Leu Leu Gly Ala Phe
   1 5
<210> 221
   <211> 8
   <212> PRT
   <213> Artificial Sequence
<220>
   <223> Light CDR3 Clone CP_03_E05
<400> 221
   His Ser Tyr Asp Ser Asn Asn His
   1 5
<210> 222
   <211> 9
   <212> PRT
   <213> Artificial Sequence
<220>
   <223> Heavy CDR3 Clone CP_03_G11
<400> 222
   His Ser Gly Val Gly Gly Leu Ala Phe
   1 5
<210> 223
   <211> 10
   <212> PRT
   <213> Artificial Sequence
<220>
   <223> Heavy CDR3 Clone CP_03_G01
<400> 223
   Gly Gly Ser Ile Ala Ala Ala Leu Ala Phe
   1 5 10
<210> 224
   <211> 8
   <212> PRT
   <213> Artificial Sequence
<220>
   <223> Heavy CDR3 Clone CP_03_H01
<400> 224
   Val Glu Tyr Ser Arg Asn Gly Met
   1 5
<210> 225
   <211> 4
   <212> PRT
   <213> Artificial Sequence
<220>
   <223> Heavy CDR3 Clone CP_03_F11
<400> 225
   Gly Arg Tyr Asn
   1
<210> 226
   <211> 7
   <212> PRT
   <213> Artificial Sequence
<220>
   <223> Heavy CDR3 Clone CP_03_C06
<400> 226
   Leu Asp Tyr Tyr Tyr Gly Met
   1               5
<210> 227
   <211> 7
   <212> PRT
   <213> Artificial Sequence
<220>
   <223> Heavy CDR3 Clone CP_03_D03
<400> 227
   Gly Gly Leu Ser Ser Ala Phe
   1               5
<210> 228
   <211> 5
   <212> PRT
   <213> Artificial Sequence
<220>
   <223> Heavy CDR3 Clone CP_03_G05
<400> 228
   Tyr Gly Gly Gly Leu
   1               5
<210> 229
   <211> 7
   <212> PRT
   <213> Artificial Sequence
<220>
   <223> Heavy CDR3 Clone CP_03_G12
<400> 229
   Pro Asp His Leu Thr Val Phe
   1               5
<210> 230
   <211> 6
   <212> PRT
   <213> Artificial Sequence
<220>
   <223> Heavy CDR3 Clone CP_03_C10
<400> 230
   Val Gly Tyr Tyr Gly Met
   1 5
<210> 231
   <211> 7
   <212> PRT
   <213> Artificial Sequence
<220>
   <223> Heavy CDR3 Clone CP_03_F04
<400> 231
   Tyr Glu Gly Tyr Ala Gly Phe
   1 5
<210> 232
   <211> 8
   <212> PRT
   <213> Artificial Sequence
<220>
   <223> Light CDR3 Clone CP_03_D05
<400> 232
   Gln Ala Trp Asp Thr Asn Leu Gly
   1 5
<210> 233
   <211> 9
   <212> PRT
   <213> Artificial Sequence
<220>
   <223> Light CDR3 Clone CP_03_H06
<400> 233
   Gln Ser Phe Asp Ala Asp Asn Leu His
   1 5
<210> 234
   <211> 7
   <212> PRT
   <213> Artificial Sequence
<220>
   <223> Light CDR3 Clone CP_03_C12
<400> 234
   Met Gln Gly Ile Gln Leu Pro
   1 5
<210> 235
   <211> 7
   <212> PRT
   <213> Artificial Sequence
<220>
   <223> Light CDR3 Clone CP_03_G02
<400> 235
   Gln Gln Ser Tyr Ser Thr Pro
   1               5
<210> 236
   <211> 7
   <212> PRT
   <213> Artificial Sequence
<220>
   <223> Light CDR3 Clone CP_03_D04
<400> 236
   Gln Ser Tyr Asp Ala Ser Asn
   1               5
<210> 237
   <211> 7
   <212> PRT
   <213> Artificial Sequence
<220>
   <223> Light CDR3 Clone CP_03_F10
<400> 237
   Ser Ala Tyr Thr Gly Ser Asn
   1               5
<210> 238
   <211> 7
   <212> PRT
   <213> Artificial Sequence
<220>
   <223> Light CDR3 Clone CP_03_G09
<400> 238
   Gln Val Trp Asp Ser Asn Ser
   1               5
<210> 239
   <211> 7
   <212> PRT
   <213> Artificial Sequence
<220>
   <223> Light CDR3 Clone CP_03_F09
<400> 239
   Gln Ser Tyr Asp Glu Val Ser
   1 5
<210> 240
   <211> 8
   <212> PRT
   <213> Artificial Sequence
<220>
   <223> Light CDR3 Clone CP_03_D09
<400> 240
   Gln Ser Tyr Asn Ser Ser Asn His
   1 5
<210> 241
   <211> 7
   <212> PRT
   <213> Artificial Sequence
<220>
   <223> Light CDR3 Clone CP_03_F02
<400> 241
   Gln Ser Tyr Asp Ser Ser His
   1 5
<210> 242
   <211> 7
   <212> PRT
   <213> Artificial Sequence
<220>
   <223> Light CDR3 Clone CP_02_E03
<400> 242
   Gln Gln Ser Arg Ser Thr Pro
   1 5
<210> 243
   <211> 8
   <212> PRT
   <213> Artificial Sequence
<220>
   <223> Light CDR3 Clone CP_03_H07
<400> 243
   Ser Ser Tyr Thr Ser Ser Ser Thr
   1 5
<210> 244
   <211> 9
   <212> PRT
   <213> Artificial Sequence
<220>
   <223> Light CDR3 Clone CP_03_C02
<400> 244
   Ser Ser Tyr Ala Gly Ser Ser Asn Leu
   1 5
<210> 245
   <211> 8
   <212> PRT
   <213> Artificial Sequence
<220>
   <223> Light CDR3 Clone CP_03_E09
<400> 245
   Asn Ser Tyr Thr Arg Ser Ser Thr
   1 5
<210> 246
   <211> 7
   <212> PRT
   <213> Artificial Sequence
<220>
   <223> Light CDR3 Clone CP_03_D08
<400> 246
   Gln Ser Tyr Asp Asp Thr Asn
   1 5
<210> 247
   <211> 8
   <212> PRT
   <213> Artificial Sequence
<220>
   <223> Light CDR3 Clone CP_03_E11
<400> 247
   Gln Ala Trp Asp Thr Asn Ile Gly
   1 5
<210> 248
   <211> 8
   <212> PRT
   <213> Artificial Sequence
<220>
   <223> Light CDR3 Clone CP_03_B05
<400> 248
   Ser Ser Tyr Ala Gly Ser Asn Asn
   1               5
<210> 249
   <211> 8
   <212> PRT
   <213> Artificial Sequence
<220>
   <223> Light CDR3 Clone CP_03_H02
<400> 249
   Gln Ala Trp Asp Thr Asn Ile Gly
   1               5
<210> 250
   <211> 8
   <212> PRT
   <213> Artificial Sequence
<220>
   <223> Light CDR3 Clone CP_03_D02
<400> 250
   Ser Ser Tyr Ala Gly Ser Ser Thr
   1               5
<210> 251
   <211> 8
   <212> PRT
   <213> Artificial Sequence
<220>
   <223> Light CDR3 Clone CP_03_E01
<400> 251
   Asn Ser Arg Asp Ser Ser Gly Phe
   1               5
<210> 252
   <211> 7
   <212> PRT
   <213> Artificial Sequence
<220>
   <223> Light CDR3 Clone CP_03_C11
<400> 252
   Ser Ser Tyr Thr Ser Ser Ser
   1               5
<210> 253
   <211> 8
   <212> PRT
   <213> Artificial Sequence
<220>
   <223> Light CDR3 Clone CP_03_B12
<400> 253
   Gln Ser Tyr Asp Ser Asn Asn Arg
   1 5
<210> 254
   <211> 9
   <212> PRT
   <213> Artificial Sequence
<220>
   <223> Light CDR3 Clone CP_03_E03
<400> 254
   Ser Ser Arg Asp Ser Ser Gly Asn His
   1 5
<210> 255
   <211> 8
   <212> PRT
   <213> Artificial Sequence
<220>
   <223> Light CDR3 Clone CP_03_F01
<400> 255
   Gln Ala Trp Asp Thr Asn Ile Gly
   1 5
<210> 256
   <211> 9
   <212> PRT
   <213> Artificial Sequence
<220>
   <223> Light CDR3 Clone CP_03_C01
<400> 256
   Gln Val Trp Asp Ser Ser Thr Ala Asn
   1 5
<210> 257
   <211> 9
   <212> PRT
   <213> Artificial Sequence
<220>
   <223> Light CDR3 Clone CP_03_G07
<400> 257
   Gln Ser Tyr Asp Ser Ser Asn His His
   1 5
<210> 258
   <211> 8
   <212> PRT
   <213> Artificial Sequence
<220>
   <223> Light CDR3 Clone CP_03_E02
<400> 258
   Ser Ser Tyr Ala Gly Asn Ser Asn
   1 5
<210> 259
   <211> 8
   <212> PRT
   <213> Artificial Sequence
<220>
   <223> Light CDR3 Clone CP_03_C07
<400> 259
   Gln Ala Trp Asp Thr Asn Ile Gly
   1 5
<210> 260
   <211> 10
   <212> PRT
   <213> Artificial Sequence
<220>
   <223> Light CDR3 Clone CP_03_H04
<400> 260
   Gly Thr Trp Asp Ser Ser Leu Ser Ala Gly
   1 5 10
<210> 261
   <211> 9
   <212> PRT
   <213> Artificial Sequence
<220>
   <223> Light CDR3 Clone CP_03_E06
<400> 261
   Ser Ser Leu Asp Ser Asn Asp Asn His
   1 5
<210> 262
   <211> 9
   <212> PRT
   <213> Artificial Sequence
<220>
   <223> Light CDR3 Clone CP_03_H03
<400> 262
   Ala Ala Trp Asp Asp Ser Leu Asn Gly
   1 5
<210> 263
   <211> 8
   <212> PRT
   <213> Artificial Sequence
<220>
   <223> Light CDR3 Clone CP_03_G11
<400> 263
   Ser Ser Tyr Ala Gly Ser Ser Thr
   1 5
<210> 264
   <211> 7
   <212> PRT
   <213> Artificial Sequence
<220>
   <223> Light CDR3 Clone CP_03_G01
<400> 264
   His Gln Tyr Asp Val Tyr Pro
   1 5
<210> 265
   <211> 9
   <212> PRT
   <213> Artificial Sequence
<220>
   <223> Light CDR3 Clone CP_03_H01
<400> 265
   Asn Ser Arg Asp Ser Ser Gly Asn His
   1 5
<210> 266
   <211> 7
   <212> PRT
   <213> Artificial Sequence
<220>
   <223> Light CDR3 Clone CP_03_F11
<400> 266
   Gln Ser Tyr Asp Ser Ser Asn
   1 5
<210> 267
   <211> 7
   <212> PRT
   <213> Artificial Sequence
<220>
   <223> Light CDR3 Clone CP_03_C06
<400> 267
   Gln Ser Tyr Asp Ser Ser Asn
   1 5
<210> 268
   <211> 7
   <212> PRT
   <213> Artificial Sequence
<220>
   <223> Light CDR3 Clone CP_03_D03
<400> 268
   Gln Ser Tyr Asp Ser Ser Asn
   1 5
<210> 269
   <211> 8
   <212> PRT
   <213> Artificial Sequence
<220>
   <223> Light CDR3 Clone CP_03_G05
<400> 269
   Ser Ser Tyr Ala Gly Ser Tyr Thr
   1 5
<210> 270
   <211> 7
   <212> PRT
   <213> Artificial Sequence
<220>
   <223> Light CDR3 Clone CP_03_G12
<400> 270
   Ser Ser Tyr Thr Pro Ser Ser
   1 5
<210> 271
   <211> 8
   <212> PRT
   <213> Artificial Sequence
<220>
   <223> Light CDR3 Clone CP_03_C10
<400> 271
   Gln Ala Trp Asp Thr Asn Ile Gly
   1 5
<210> 272
   <211> 8
   <212> PRT
   <213> Artificial Sequence
<220>
   <223> Light CDR3 Clone CP_03_F04
<400> 272
   Gln Ala Trp Asp Thr Asn Ile Gly
   1 5